# EUROPEAN PATENT SPECIFICATION

(11) **EP 1 242 403 B1**
(45) Date of publication and mention of the grant of the patent: **04.01.2006**
(21) Application number: 00971650.7
(22) Date of filing: 09.11.2000
(51) Int. Cl.: C07D 401/04, A61K 31/506, A61P 37/06, C07D 239/00, C07D 215/00

(54) **2,4-DIAMINOPYRIMIDINE COMPOUNDS USFUL AS IMMUNOSUPPRESSANTS**
2,4-DIAMINOPYRIMIDINDERIVATE ALS IMMUNOSUPPRESSIVA
COMPOSES DE 2,4-DIAMINOPYRIMIDINE UTILISES COMME IMMUNODEPRESSEURS

(30) Priority: 30.11.1999 US 168224 P
(43) Date of publication of application: 25.09.2002
(73) Proprietor: Pfizer Products Inc., Groton, Connecticut 06340 (US)
(72) Inventor: BLUMENKOPF, Todd Andrew, Groton, CT 06340 (US); MUELLER, Eileen Elliott, Groton, CT 06340 (US); ROSKAMP, Eric Jan, Groton, CT 06340 (US)
(74) Representative: Motion, Keith Robert
(86) International application number: PCT/IB2000/001628
(87) International publication number: WO 2001/040215

(56) References cited:
- EP-A- 0 837 063
- WO-A-00/53595
- WO-A-97/19065

## Description

### Field of the Invention

The present invention relates to therapeutic modulation of T-cell mediated cellular processes. T-cells ("thymus-derived" cells) are responsible for numerous cell-mediated immune functions, and indirectly, by stimulating B-cells, contribute to antibody production. The cell membrane of a T-cell contains numerous receptor and accessory protein molecules that facilitate activation of T-cells, differentiation of T-cells into various subtypes, and the interaction of T-cells with other cells or cell components.

Although T-cell mediated immune responses are normally of great benefit, there are circumstances where it is appropriate to suppress or otherwise modulate immune response mediated by activated T-cells. An important example is organ, tissue, or cell transplantation, where suppression of immune response against the transplant (whether allograft or xenograft) is essential. Additional examples include treatment of allergy, autoimmune disease, and disease states involving inflammation. Since T-cells are involved in so many immune-mediated processes, and such processes generally involve overlapping use of various T-cell proteins and signaling functions, it has been very difficult to modulate only certain T-cell functions, without adversely affecting other desirable cellular processes. The present invention is directed to a class of therapeutic compounds that selectively interfere with certain signaling events that occur during T-cell activation, thus permitting selective regulation of immune response.

### Reported Developments

T-cells differentiate and proliferate in response to recognition of antigens (generally, foreign macromolecules) in order to carry out various cell-mediated immune processes. This recognition of antigen, followed by functional and morphological changes in the T-cell, is termed activation. Among the functions carried out by differentiated T-cells are (1) killing of virus-infected self cells, (2) killing foreign cells, (3) activation of other cells (for example, macrophages) that are capable of engulfing foreign particles (such as bacteria and viruses), and in turn processing their macromolecules for presentation to, and activation of, additional T-cells, (4) suppression of immune response of B-cells and T-cetts to antigen, which, for example, may act to establish immune tolerance, (5) activation of other T-cells, and (6) once themselves activated by antigen, helping B-cells respond to foreign antigens so that antibodies can be produced. In some cases these effects are carried out by direct contact of T-cells with their targets, and in other cases T-cells secrete a variety of substances (generically termed lymphokines) in order to activate target cells at a distance, or both mechanisms may be involved.

As aforementioned, autoimmune disease, transplant rejection, allergy, and inflammation represent disease states wherein undesired activation of antigen-specific T-cells appears necessary for induction and/or progression of the unwanted clinical state. For example, necessary release of some lymphokines (such as γ-interferon ) by T-cells may cause macrophage cells to not only migrate to a site of infection or tissue damage, but to release other soluble factors that slowly trigger undesired inflammation (for example, in delayed type hypersensitivity). Accordingly, pharmaceutical compounds that interrupt activation of T-cells under specific circumstances, or specific downstream signalling events, are expected to be of great therapeutic value. See, for example, J. H. Hanke, et al., *Inflammation Research,* 44, pp. 357-371, 1995.

T-cells recognize antigen through membrane glycoprotein receptors, called TcR. which are, in part, similar in structure and sequence to the antibodies of B-cells. The genetic elements from which the two protein classes are expressed are undoubtedly of common origin. In general, T-cells only recognize antigen that is presented to them, in processed form, on the surface of other cells. Like antibody producing B-cells, each individual progenitor T-cell only recognizes a particular amino acid or carbohydrate sequence and/or other molecular structure (termed an epitope) in the processed antigen, which structure is usually unique to the antigen . Such specific recognition of antigen permits response against a wide range of foreign macromolecules, and is a necessary feature of mechanisms whereby immune responses against self-molecules are normally prevented.

Following the binding of antigen to the T-cell surface, numerous events must occur in the cell membrane and inside the T-cell to complete its activation. As reviewed in *Hanke et al*., activation of the T-cell involves association of other cell membane glycoproteins, such as among CD4, CD8, CD3 and CD28, with the TcR, and also phosphorylation of tyrosine amino acid residues in these proteins (see C.H. June et al., *Journal of Immunology,* 144, pp. 1591-1599 (1990), and D. B. Strauss et al., Cell, 70, pp. 585-593, 1992). Phosphorylation of tyrosine amino acid residues is carried out by a class of enzymes known as protein tyrosine kinases (PTKs). Inhibition of phosphorylation by tyrosine kinases has been shown to modulate T-cell activation, and numerous T-cell mediated immune processes. (see, for example, C.H. June et al., *Proceedings of the National Academy of Sciences, USA,* 87, pp. 7722-7726, 1990). Accordingly, regulation of T-cell activation (or subsequent signal transducting events) by selective inhibition of particular PTKs has been of particular interest.

However, phosphorylation of tyrosine resides in membrane bound and cytoplasmic proteins is a general mechanism. It plays an important role in numerous signaling pathways, not merely those confined to the immune system. Tyrosine phosphorylation occurs, for example, in response to binding of growth factors such as epidermal growth factor (EGF), platelet-derived growth factor(PDGF), nerve growth factor (NGF), and also insulin. Given the presence of a large number of cellular processes dependent upon tyrosine phosphorylation, it will be immediately apparent that preferred compounds for inhibition of T-cell activation, and/or subsequent immune system signalling events, should be designed to inhibit only one (or at most a very few) tyrosine kinases, to thus avoid interfering with a wide range of other cellular metabolic pathways.

Additionally, since the specificity of a particular inhibitor compound cannot be practically tested against all tyrosine kinases, and indeed numerous kinases remain to be discovered, it is most preferred to provide compounds that are highly specific for a particular tyrosine kinase. A general discussion of tyrosine kinase proteins known to be associated with T-cell activation is provided in *J.H. Hanke et al.,* 1995. Tyrosine kinases (PTKs) involved in regulation of T-cell activation include:
(a) *lck* (a 56,000 MW protein, also known as p56^{lck}) which is associated with the TcR complex, and which is in the src-kinase family;
(b) *fyn* which is also in the src-kinase family;
(c) Zap-70 and syk, which share limited homology with src-kinases;
(d) *itk* kinase, which may be associated with the CD28 receptor; and
(e) csk-like kinases, and which may also negatively regulate the function of other PTKs.

Considerable evidence supports the involvement of *Ick* in T-cell activation, and suggests that inhibition of *Ick* activity is an important point of therapeutic intervention. *D.B. Strauss et al.,* 1992, determined that mutant Jurkat cells (JcaM1) that failed to show an increase in calcium levels following receptor stimulation lacked expression of functional *lck* tyrosine kinase. T.J. Molina et al., *Nature,* 357, pp. 161-164, 1992 generated an *Ick* null mutation by homologous recombination in murine embryonic stem cells. Lck-deficient mice evidenced pronounced thymic atrophy, and few CD4+, CD8+, or CD4+/CD8+ thymocytes were detected. Additionally, F. D. Goldman et al., *Journal of Clinical Investigation,* 102, pp. 421-429, 1998, have reported on an infant patient presenting a SCID (severe combined immune deficiency) phenotype in which *p59fyn* and ZAP-70 kinases were expressed at normal levels, although a marked decrease in the level of *lck* was noted. Interestingly, an alternatively spliced *lck* transcript, that lacked the kinase-encoding domain provided by exon 7 of the *lck* gene, was identified from the patient.

There are reports in the scientific literature of compounds that modulate T-cell mediated immune function, and/or which inhibit tyrosine kinases (PTKs) of the receptor and non-receptor type. For example, published international patent documents WO 98/54156 and WO 98/54157 describe quinoline and quinoxaline compounds that inhibit platelet-derived growth factor PTK and/or *lck ,* and are useful in affecting T-cell activation and proliferation. Additionally WO 97/40019 discloses 5-aminopyrazole compounds useful as selective inhibitors of *lck*. The disclosure in WO 98/11095 recites substituted 2-pyrimidineamines, and their use as inhibitors of protein kinases such as ZAP-70, protein kinase C and *lck.* The compounds are described as useful in regard to diseases or conditions involving the immune system or cellular hyperproliferation. Additional publications of note include WO 99/24035, WO 98/41525, WO 97/19065, WO 98/28281 and WO 98/18782.

The present invention provides pharmaceutical compounds useful for the treatment of clinical conditions that involve inappropriate T-cell activation. In particular, highly specific inhibitors of *Ick* tyrosine kinase are disclosed.

### Summary of Invention

Accordingly, there are provided compounds according to the formula 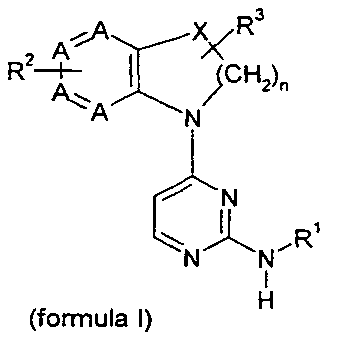
or pharmaceutically acceptable salts, solvates, or hydrates thereof; wherein
each occurrence of A is independently selected from CH or N;
X is selected from the group consisting of -CH₂-, -O-, -NH-, (C₁-C₆)alkylamino-, (C₁-C₆)alkylaminocarbonylamino-, (C₁-C₆)alkylcarbonylamino-, (C₁-C₆)alkylsulfonylamino-, phenylsulfonylamino-, carbonyl, -NH-C(O)-, -N(C₁-C₆)alkyl-C(O)-, -S(O)_{y}- where y is 0, 1 or 2, and ;
n in -(CH₂)ₙ- is 1, 2 or 3;
R¹ is selected from the groups consisting of (C₆-C₁₀)aryl-, (C₁-C₉)heteroaryl-, (C₆-C₁₀)aryl(C₆-C₁₀)aryl-, (C₆-C₁₀)aryl(C₁-C₉) heteroaryl-, (C₁-C₉)heteroaryl(C₁-C₉)heteroaryl-, (C,-C₉)heteroaryl(C₆-C₁₀)aryl-, (C₆-C₁₀)arylsulfinyl-, (C₆-C₁₀)aryl(C₆-C₁₀)arylsulfinyl-, (C,-C₉)heteroaryl(C₆-C₁₀)arylsulfinyl-, (C₆-C₁₀)arylsulfonyl-, (C₆-C₁₀)aryl(C₆-C₁₀)arylsulfonyl-, (C₁-C₉)heteroaryl(C₆-C₁₀)arylsulfonyl-, (C₁-C₉)heteroarylsulfinyl-, (C₁-C₉)heteroaryl(C₁-C₉)heteroarylsulfinyl-, (C₆-C₁₀)aryl(C₁-C₉)heteroarylsulfinyl-, (C₁-C₉)heteroarylsulfonyl-, (C₁-C₉)heteroaryl(C₁-C₉)heteroarylsulfonyl-, (C₆-C₁₀)aryl(C₁-C₉)heteroarylsulfonyl-, (R⁴)sulfinyl-, (R⁴)sulfonyl-, (C₆-C₁₀)aryl(R⁴)sulfinyl-, (C₆-C₁₀)aryl(C₆-C₁₀)aryl(R⁴)sulfinyl-, (C,-C₉)heteroaryl(C₆-C₁₀)aryl(R⁴)sulfinyl-, (C₆-C₁₀)aryl(R⁴)sulfonyl-, (C₆-C₁₀)aryl(C₆-C₁₀)aryl(R⁴)sulfonyl-, (C₁-C₉)heteroaryl(C₆-C₁₀)aryl(R⁴)sulfonyl-, (C₁-C₉)heteroaryl(R⁴)sulfinyl-, (C₆-C₁₀)aryl(C₁-C₉)heteroaryl(R⁴)sulfinyl-, (C₁-C₉)heteroaryl(C₁-C₉)heteroaryl(R⁴)sulfinyl-, (C₁-C₉)heteroaryl(R⁴)sulfonyl-, (C₆-C₁₀)aryl(C₁-C₉)heteroaryl(R⁴)sulfonyl-, (C₁-C₉)heteroaryl(C₁-C₉)heteroaryl(R⁴)sulfonyl-, (C₆-C₁₀)arylaminocarbonyl-, (C₆-C₁₀)aryl(C₆-C₁₀)arylaminocarbonyl-(C₁-C₉)heteroaryl(C₆-C₁₀)arylaminocarbonyl-, (C₁-C₉)heteroarylaminocarbonyl-, (C₆-C₁₀)aryl(C₁-C₉)heteroarylaminocarbonyl-, (C₁-C₉)heteroaryl(C₁-C₉)heteroarylaminocarbonyl-, (C₆-C₁₀)arylcarbonyl-, (C₆-C₁₀)aryl(C₆-C₁₀)arylcarbonyl-, (C₁-C₉)heteroaryl(C₆-C₁₀)arylcarbonyl-, (C₁-C₉)heteroarylcarbonyl-, (C₆-C₁₀)aryl(C₁-C₉)heteroarylcarbonyl-, (C,-C₉)heteroaryl(C₁-C₉)heteroarylcarbonyl-, (C₆-C₁₀)aryloxycarbonyl-, (C₆-C₁₀)aryl(C₆-C₁₀)aryloxycarbonyl-, (C₁-C₉)heteroaryl(C₆-C₁₀)aryloxycarbonyl-, (C₁-C₉)heteroaryloxycarbonyl-, (C₆-C₁₀)aryl(C₁-C₉)heteroaryloxycarbonyl-, (C₁-C₉)heteroaryl(C₁-C₉)heteroaryloxycarbonyl-, (R⁴)carbonyl-, (R⁴)oxycarbonyl-, (R⁴)aminocarbonyl-, (C₆-C₁₀)aryl(R⁴)carbonyl-, (C₆-C₁₀)aryl(R⁴)oxycarbonyl-, (C₆-C₁₀)aryl(R⁴)aminocarbonyl-, (C,-C₉)heteroaryl(R⁴)carbonyl-, (C₁-C₉)heteroaryl(R⁴)oxycarbonyl-, and (C₁-C₉)heteroaryl(R⁴) aminocarbonyl-;
wherein R⁴ is selected from the groups consisting of
(a) (C₁-C₆)alkyl-, (C₂-C₆)alkenyl-, or (C₂-C₆)alkynyl-, wherein the alkyl-, alkenyl- and alkynyl- groups are optionally substituted by hydroxy, halo, amino, trifluoromethyl, hydroxy(C₂-C₆)alkyl-, (C₁-C₆)alkoxy-, (C₁-C₆)acyloxy-, (C₁-C₆)alkylamino-, ((C₁-C₆)alkyl)₂amino-, (C,-C₆)acylamino-, cyano, nitro, (C₁-C₆)alkyl-, (C₂-C₆)alkenyl-, (C₂-C₆)alkynyl-. (C₁-C₆)acylamino-, cyano(C₁-C₆)alkyl-, trifluoromethyl(C₁-C₆)alkyl-, (C₁-C₃)alkyl(difluoromethylene)(C₁-C₃)alkyl-, or nitro(C₁-C₆)alkyl-;
(b) (C₃-C₁₀)cycloalkyl-, wherein the cycloalkyl- group is optionally substituted by hydroxy, halo, amino, trifluoromethyl, hydroxy(C₂-C₆)alkyl-, (C₁-C₆)alkoxy-, (C₁-C₆)acyloxy-, (C₁-C₆)alkylamino-, ((C₁-C₆)alkyl)₂amino-, (C₁-C₆)acylamino-, cyano, nitro, (C₁-C₆)alkyl-, (C₂-C₆)alkenyl-, (C₂-C₆)alkynyl-, (C₁-C₆)acylamino-, cyano(C₁-C₆)alkyl-, trifluoromethyl(C,-C₆)alkyl-, (C₁-C₃)alkyl(difluoromethylene)(C₁-C₃)alkyl-, or nitro(C₁-C₆)alkyl-; or
(c) (C₃-C₁₀)heterocycloalkyl-, wherein the heterocycloalkyl- group is optionally substituted by hydroxy, halo, amino, trifluoromethyl, hydroxy(C₂-C₆)alkyl-, (C₁-C₆)alkoxy-, (C,-C₆)acyloxy-, (C₁-C₆)alkylamino-, ((C₁-C₆)alkyl)₂amino-, (C₁-C₆)acylamino-, cyano, nitro, (C,-C₆)alkyl-, (C₂-C₆)alkenyl-, (C₂-C₆)alkynyl-, (C₁-C₆)acylamino-, cyano(C₁-C₆)alkyl-, trifluoromethyl(C₁-C₆)alkyl-, (C₁-C₃)alkyl(difluoromethylene)(C₁-C₃)alkyl-, or nitro(C₁-C₆)alkyl-; and

wherein any of said of (C₆-C₁₀)aryl- or (C₁-C₉)heteroaryl- groups of R¹ may be optionally substituted by one to five groups selected from
(a) deuterium, hydroxy, halo, amino, trifluoromethyl, carboxy, (C₁-C₆)alkoxy-, (C,-C₆)acyloxy-, (C₁-C₆)alkylamino-, ((C₁-C₆)alkyl)₂amino-, (C₁-C₆)acylamino-, cyano, nitro, (C,-C₆)alkyl-, (C₂-C₆)alkenyl-, (C₂-C₆)alkynyl-, (C₁-C₆)acylamino-, cyano(C₁-C₆)alkyl-, trifluoromethyl(C₁-C₆)alkyl-, or nitro(C₁-C₆)alkyl-, (C₁-C₃)alkyl(difluoromethylene)(C₁-C₃)alkyl-, (C₁-C₆)acylamino(C₁-C₆)alkyl-, (C₁-C₆)alkoxy(C₁-C₆)acylamino-, amino(C₁-C₆)acyl-, amino(C₁-C₆)acyl(C₁-C₆)alkyl-, (C₁-C₆)alkylamino(C₁-C₆)acyl-, ((C₁-C₆)alkyl)₂amino(C₁-C₆)acyl-, (C₃-C₁₀)cycloalkyl(C₁-C₆)alkyl-, (C₁-C₆)acyloxy(C₁-C₆)alkyl-, (C₂-C₆)alkoxy(C₁-C₆)alkyl-, piperazinyl(C₁-C₆)alkyl-, (C₁-C₆)acylamino(C₁-C₆)alkyl-, (C₆-C₁₀)aryl(C₁-C₆)alkoxy(C₁-C₆)alkyl-, (C₅-C₉)heteroaryl(C₁-C₆)alkoxy(C₁-C₆)alkyl-, (C₁-C₆)alkylthio(C₁-C₆)alkyl-, (C₆-C₁₀)arylthio(C₁-C₆)alkyl-, (C₁-C₆)alkylsulfinyl(C₁-C₆)alkyl-, (C₆-C₁₀)arylsulfinyl(C₁-C₆)alkyl-, (C,-C₆)alkylsulfonyl(C₁-C₆)alkyl-, (C₆-C₁₀)arylsulfonyl(C₁-C₆)alkyl-, amino(C₁-C₆)alkyl-, (C₁-C₆)alkylamino(C₁-C₆)alkyl-, (C₁-C₆)alkyl(difluoromethylene)-, (C₁-C₃)alkyl(difluoromethylene)(C₁-C₃)alkyl-, (C₁-C₆)alkoxy(C₁-C₆)acyl-, (C₁-C₆)alkylamino(C₁-C₆)acyl-, ((C₁-C₆)alkyl)₂amino(C₁-C₆)acyl-, (C₆-C₁₀)aryl-, (C₁-C₉)heteroaryl-, (C₆-C₁₀)aryl(C₁-C₆)alkyl-, (C₁-C₉)heteroaryl(C₁-C₆)alkyl-, (C₆-C₁₀)aryl(C₆-C₁₀)aryl-, (C₆-C₁₀)aryl(C₆-C₁₀)aryl(C₁-C₆)alkyl-, (C₃-C₁₀)cycloalkyl-, (C₃-C₆)cycloalkyl(C₁-C₆)alkyl-, (C₃-C₁₀)heterocycloalkyl-, (C₃-C₁₀)heterocycloalkyl(C₁-C₆)alkyl-, hydroxy(C₂-C₆)alkyl-, (C₁-C₆)acyloxy(C₂-C₆)alkyl-, (C₁-C₆)alkoxy(C₂-C₆)alkyl-, piperazinyl(C₁-C₆)alkyl-, (C₁-C₆)acylamino(C₁-C₆)alkyl-, (C₆-C₁₀)aryl(C₁-C₆)alkoxy(C₁-C₆)alkyl-, (C₁-C₉)heteroaryl(C₁-C₆)alkoxy(C₁-C₆)alkyl-, (C₁-C₆)alkylthio(C₁-C₆)alkyl-, (C₆-C₁₀)arylthio(C₁-C₆)alkyl-, (C₁-C₆)alkylsulfinyl(C₁-C₆)alkyl-, (C₆-C₁₀)arylsuffinyl(C₁-C₆)alkyl-, (C₁-C₆)alkylsulfonyl(C₁-C₆)alkyl-, (C₆-C₁₀)arylsulfonyl(C₁-C₆)alkyl-, amino(C₁-C₆)alkyl-, (C₁-C₆)alkylamino(C₁-C₆)alkyl-, ((C₁-C₆)alkyl)₂amino(C₁-C₆)alkyl-;
(b) R⁵OCO(C₁-C₆)alkyl-, wherein R⁵ is selected from the group consisting of hydrogen, (C₁-C₆)alkyl-, (C₆-C₁₀)aryl(C₁-C₆)alkyl-, (C₁-C₉)heteroaryl(C₁-C₆)alkyl-; or
(c) R⁶(C₀-C₆)alkyl-, wherein R⁶ is selected from the group consisting of piperazino, (C,-C₆)acylpiperazino-, (C₆-C₁₀)arylpiperazino-, (C₅-C₉)heteroarylpiperazino-, (C,-C₆)alkylpiperazino-, (C₆-C₁₀)aryl(C₁-C₆)alkylpiperazino-, (C₁-C₉)heteroaryl(C₁-C₆)alkylpiperazino-, morpholino-, (C₁-C₆)acylmorpholino-, (C₆-C₁₀)arylmorpholino-, (C,-C₉)heteroarylmorpholino-, (C₁-C₆)alkylmorpholino-, (C₆-C₁₀)aryl(C₁-C₆)alkylmorpholino-, (C₁-C₉)heteroaryl(C₁-C₆)alkylmorpholino-, thiomorpholino-, (C₁-C₆)acylthiomorpholino-, (C₆-C₁₀)arylthiomorpholino-, (C₁-C₉)heteroarylthiomorpholino-, (C₁-C₆)alkylthiomorpholino-, (C₆-C₁₀)aryl(C₁-C₆)alkylthiomorpholino-, (C₁-C₆)heteroaryl(C₁-C₆)alkylthiomorpholino-, piperidino-, (C₁-C₆)acylpiperidina, (C₆-C₁₀)arylpiperidino-, (C₁-C₉)heteroarylpiperidino-, (C₁-C₆)alkyl piperidino-, (C₆-C₁₀)aryl(C₁-C₆)piperidino-, (C₁-C₉)heteroaryl(C₁-C₆)alkylpiperidino-, pyrrolidino-, (C₁-C₆)acylpyrrolidino-, (C₆-C₁₀)aryl- pyrrolidino-, (C₁-C₉)heteroaryl- pyrrolidino-, (C₁-C₆)alkylpyrrolidino-, (C₆-C₁₀)aryl(C₁-C₆)pyrrolidino-, (C₁-C₉)heteroaryl(C₁-C₆)alkylpyrrolidino-, (C₁-C₆)alkoxy(C₁-C₆)acyl-, (C₁-C₆)alkylamino(C₆-C₁₀)aryl-, and ((C₁-C₆)alkyl₂amino(C₁-C₆)acyl-;

R² represents one to four optional substituents, each being independently selected from the members of groups (a) to (f)
(a) deuterium, halo, hydroxy, carboxy, amino, trifluoromethyl, (C₁-C₆) alkyl-, (C₂-C₆)alkenyl-, (C₂-C₆)alkynyl-, (C₁-C₆)alkylamino-. ((C₁-C₆)(alkyl)₂amino-, cyanoalkyl-, (C₃-C₁₀)cycloalkyl-. (C₃-C₁₀)heterocycloalkyl-, (C₃-C₁₀)cycloalkoxy-, (C₁-C₆)alkylthio-, (C₁-C₆)alkylsulfinyl-, (C₁-C₆)alkylsulfonyl-, amino-CO-NH- , (C₁-C₆)alkoxy-CO-NH-. (C₁-C₆)alkyl-CO-NH- , (C₁-C₆)alkyl-CO-NH-(C₁-C₆)alkyl-, (C₁-C₆)alkyl-CO-NH-(C₁-C₆)alkoxy-, (C,-C₆)alkoxycarbonyl(C₁-C₆)alkoxy-, (C₁-C₆)alkoxy-CO-NH-(C₁-C₆)alkoxy-, (C₁-C₆)alkylamino-CO-NH- , (C₁-C₆)alkylamino-CO-NH-(C₁-C₆)alkyl-, ((C₁-C₆)alkyl)₂amino-CO-NH-(C₁-C₆)alkyl-, ((C₁-C₆)alkyl)₂amino-CO-NH- carboxy, carboxy(C₁-C₆)alkyl-, carboxy(C₁-C₆)alkoxy-, benzyloxycarbonyl(C₁-C₆)alkoxy-, (C₁-C₆)alkylamino-CO-, (C₁-C₆)acylamino-, (C₁-C₆)alkoxy-, (C₁-C₆)acyl-, (C₁-C₆)acyloxy-, (C₁-C₆)acyl(C₁-C₆)alkylamino-, (C₁-C₆)alkoxyacyl-, (C,-C₆)alkylaminoacyl-, ((C₁-C₆)alkyl)₂aminoacyl-, amino(C₁-C₆)acyl-, amino(C₁-C₆)alkyl-, (C,-C₆)alkoxycarbonylamino-, (C₁-C₆)alkoxycarbonyl(C₁-C₆)alkyl-, (C₆-C₁₀)aryl(C₁-C₆)alkoxycarbonylamino-, trihalomethyl-, trihalomethyl(C₁-C₆)alkyl-, (C,-C₆)alkyldihalomethylene-, (C₁-C₃)alkyl(dihalomethylene)(C₁-C₃)alkyl-, (C₃-C₆)cycloalkyl-, (C₃-C₆)cycloalkyl(C₁-C₆)alkyl-, hydroxy(C₁-C₆)alkyl-, (C₁-C₆)acyloxy(C₁-C₆)alkyl-, (C₁-C₆)alkoxy(C₁-C₆)alkyl-, (C₁-C₆)acylamino(C₁-C₆)alkyl-, (C₁-C₆)alkylthio(C₁-C₆)alkyl-, (C,-C₆)alkoxycarbonyl-, (C₁-C₆)alkylsulfinyl(C₁-C₆)alkyl-, (C₁-C₆)alkylsulfonyl(C₁-C₆)alkyl-, (C₁-C₆)alkylsulfonyl-, (C₁-C₆)alkylsulfonylamino-, (C₁-C₆)alkylsulfonylamino(C₁-C₆)alkyl-, amino(C₁-C₆)alkyl-, (C₁-C₆)alkylamino(C₁-C₆)alkyl-, ((C₁-C₆)alkyl)₂amino(C₁-C₆)alkyl-, (C₁-C₆)CO(C₁-C₆)alkyl-;
(b) (C₆-C₁₀)aryl-, (C₁-C₉)heteroaryl-, (C₆-C₁₀)aryl(C₆-C₁₀)aryl-, (C₆-C₁₀)aryl(C₁-C₉) heteroaryl-, (C₁-C₉)heteroaryl(C₁-C₉)heteroaryl-, (C₁-C₉)heteroaryl(C₆-C₁₀)aryl-, (C₆-C₁₀)arylsulfinyl-, (C₆-C₁₀)aryl(C₆-C₁₀)arylsulfinyl-, (C₁-C₉)heteroaryl(C₆-C₁₀)arylsulfinyl-, (C₆-C₁₀)arylsulfonyl-, (C₆-C₁₀)aryl(C₆-C₁₀)arylsulfonyl-, (C₁-C₉)heteroaryl(C₆-C₁₀)arylsulfonyl-, (C₁-C₉)heteroarylsulfinyl-, (C₁-C₉)heteroaryl(C₁-C₉)heteroarylsulfinyl-, (C₆-C₁₀)aryl(C₁-C₉)heteroarylsulfinyl-, (C₁-C₉)heteroarylsulfonyl-, (C₁-C₉)heteroaryl(C₁-C₉)heteroarylsulfonyl-, (C₆-C₁₀)aryl(C₁-C₉)heteroarylsulfonyl-, (R⁴)sulfinyl-, (R⁴)sulfonyl-, (C₆-C₁₀)aryl(R⁴)sulfinyl-, (C₆-C₁₀)aryl(C₆-C₁₀)aryl(R⁴)sulfinyl-, (C₁-C₉)heteroaryl(C₆-C₁₀)aryl(R⁴)sulfinyl-, (C₆-C₁₀)aryl(R⁴)sulfonyl-, (C₆-C₁₀)aryl(C₆-C₁₀)aryl(R⁴)sulfonyl-, (C₁-C₉)heteroaryl(C₆-C₁₀)aryl(R⁴)sulfonyl-, (C₁-C₉)heteroaryl(R⁴)sulfinyl-, (C₆-C₁₀)aryl(C₁-C₉)heteroaryl(R⁴)sulfinyl-, (C₁-C₉)heteroaryl(C₁-C₉)heteroaryl(R⁴)sulfinyl-, (C₁-C₉)heteroaryl(R⁴)sulfonyl-, (C₆-C₁₀)aryl(C₅-C₉)heteroaryl(R⁴)sulfonyl-, (C₁-C₉)heteroaryl(C₁-C₉)heteroaryl(R⁴)sulfonyl-, (C₆-C₁₀)arylaminocarbonyl-, (C₆-C₁₀)aryl(C₆-C₁₀)arylaminocarbonyl-, (C₁-C₉)heteroaryl(C₆-C₁₀)arylaminocarbonyl-, (C₁-C₉)heteroarylaminocarbonyl-, (C₆-C₁₀)aryl(C₁-C₉)heteroarylaminocarbonyl-, (C₁-C₉)heteroaryl(C₁-C₉)heteroarylaminocarbonyl-, (C₆-C₁₀)arylcarbonyl-, (C₆-C₁₀)aryl(C₆-C₁₀)arylcarbonyl-, (C₁-C₉)heteroaryl(C₆-C₁₀)arylcarbonyl-, (C₁-C₉)heteroarylcarbonyl-, (C₆-C₁₀)aryl(C₁-C₉)heteroarylcarbonyl-, (C₁-C₉)heteroaryl(C₁-C₉)heteroarylcarbonyl-, (C₆-C₁₀)aryloxycarbonyl-, (C₆-C₁₀)aryl(C₆-C₁₀)aryloxycarbonyl-, (C,-C₉)heteroaryl(C₆-C₁₀)aryloxycarbonyl-, (C₁-C₉)heteroaryloxycarbonyl-, (C₆-C₁₀)aryl(C₁-C₉)heteroaryloxycarbonyl-, (C₁-C₉)heteroaryl(C₁-C₉)heteroaryloxycarbonyl-, (R⁴)carbonyl-, (R⁴)oxycarbonyl-,(R⁴) aminocarbonyl-, (C₆-C₁₀)aryl(R⁴)carbonyl-, (C₆-C₁₀)aryl(R⁴)oxycarbonyl-, (C₆-C₁₀)aryl(R⁴)aminocarbonyl-, (C₁-C₉)heteroaryl(R⁴)carbonyl-, (C₅-C₉)heteroaryl(R⁴)oxycarbonyl-, (C₁-C₉)heteroaryl(R⁴) aminocarbonyl-, wherein R⁴ is defined as above, and wherein any of said of (C₆-C₁₀)aryl- or (C₁-C₉)heteroaryl- R² groups may be optionally substituted by one to five groups independently selected from:
   (i) hydroxy, halo, amino, trifluoromethyl, carboxy, (C₁-C₆)alkoxy-, (C,-C₆)acyloxy-, (C₁-C₆)alkylamino-, ((C₁-C₆)alkyl)₂amino-, (C₁-C₆)acylamino-, cyano, nitro, (C,-C₆)alkyl-, (C₂-C₆)alkenyl-, (C₂-C₆)alkynyl-, (C₁-C₆)acylamino-, cyano(C₁-C₆)alkyl-, trifluoromethyl(C₁-C₆)alkyl-, or nitro(C₁-C₆)alkyl-, (C₁-C₃)alkyl(difluoromethylene)(C₁-C₃)alkyl-, (C₁-C₆)acylamino(C₁-C₆)alkyl-, (C₁-C₆)alkoxy(C₁-C₆)acylamino-, amino(C₁-C₆)acyl-, amino(C₁-C₆)acyl(C₁-C₆)alkyl-, (C₁-C₆)alkylamino(C₁-C₆)acyl-, ((C₁-C₆)alkyl)₂amino(C₁-C₆)acyl-, (C₃-C₁₀)cycloalkyl(C₁-C₆)alkyl-, (C₁-C₆)acyloxy(C₁-C₆)alkyl-, (C₂-C₆)alkoxy(C₁-C₆)alkyl-, piperazinyl(C₁-C₆)alkyl-, (C₁-C₆)acylamino(C₁-C₆)alkyl-, (C₆-C₁₀)aryl(C₁-C₆)alkoxy(C₁-C₆)alkyl-, (C₁-C₉)heteroaryl(C₁-C₆)alkoxy(C₁-C₆)alkyl-, (C₁-C₆)alkylthio(C₁-C₆)alkyl-, (C₆-C₁₀)arylthio(C₁-C₆)alkyl-, (C₁-C₆)alkylsulfinyl(C₁-C₆)alkyl-, (C₆-C₁₀)arylsulfinyl(C₁-C₆)alkyl-, (C,-C₆)alkylsulfonyl(C₁-C₆)alkyl-, (C₆-C₁₀)arylsulfonyl(C₁-C₆)alkyl-, amino(C₁-C₆)alkyl-, (C,-C₆)alkylamino(C₁-C₆)alkyl-, (C₁-C₆)alkyl(difluoromethylene)-, (C₁-C₃)alkyl(difluoromethylene)(C₁-C₃)alkyl-, (C₁-C₆)alkoxy(C₁-C₆)acyl-, (C₁-C₆)alkylamino(C₁-C₆)acyl-, ((C₁-C₆)alkyl)₂amino(C₁-C₆)acyl-, (C₆-C₁₀)aryl-, (C₁-C₉)heteroaryl-, (C₆-C₁₀)aryl(C₁-C₆)alkyl-, (C₁-C₉)heteroaryl(C₁-C₆)alkyl-, (C₆-C₁₀)aryl(C₆-C₁₀)aryl-, (C₆-C₁₀)aryl(C₆-C₁₀)aryl(C₁-C₆)alkyl-, (C₃-C₁₀)cycloalkyl-, (C₃-C₆)cycloalkyl(C₁-C₆)alkyl-, (C₃-C₁₀)heterocycloalkyl-, (C₃-C₁₀)heterocycloalkyl(C₁-C₆)alkyl-, hydroxy(C₂-C₆)alkyl-, (C₁-C₆)acyloxy(C₂-C₆)alkyl-, (C₁-C₆)alkoxy(C₂-C₆)alkyl-, piperazinyl(C₁-C₆)alkyl-, (C₁-C₆)acylamino(C₁-C₆)alkyl-, (C₆-C₁₀)aryl(C₁-C₆)alkoxy(C₁-C₆)alkyl-, (C₁-C₉)heteroaryl(C₁-C₆)alkoxy(C₁-C₆)alkyl-, (C₁-C₆)alkylthio(C₁-C₆)alkyl-, (C₆-C₁₀)arylthio(C₁-C₆)alkyl-, (C₁-C₆)alkylsulfinyl(C₁-C₆)alkyl-, (C₆-C₁₀)arylsulfinyl(C₁-C₆)alkyl-, (C₁-C₆)alkylsulfonyl(C₁-C₆)alkyl-, (C₆-C₁₀)arylsulfonyl(C₁-C₆)alkyl-, amino(C₁-C₆)alkyl-, (C₁-C₆)alkylamino(C₁-C₆)alkyl-, ((C₁-C₆)alkyl)₂amino(C₁-C₆)alkyl-;
   (ii) R⁵OCO(C₁-C₆)alkyl- wherein R⁵ is selected from the group consisting of hydrogen, (C₁-C₆)alkyl, (C₆-C₁₀)aryl(C₁-C₆)alkyl-, (C₁-C₉)heteroaryl(C₁-C₆)alkyl-;
   (iii) R⁶(C₂-C₆)alkyl- wherein R⁶ is selected from the group consisting of piperazino, (C₁-C₆)acylpiperazino-, (C₆-C₁₀)arylpiperazino-, (C₅-C₉)heteroarylpiperazino-, (C,-C₆)alkylpiperazino-, (C₆-C₁₀)aryl(C₁-C₆)alkylpiperazino-, (C₁-C₉)heteroaryl(C₁-C₆)alkylpiperazino-, morpholino-, (C₁-C₆)acylmorpholino-, (C₆-C₁₀)arylmorpholino-, (C₁-C₉)heteroarylmorpholino-, (C₁-C₆)alkylmorpholino-, (C₆-C₁₀)aryl(C₁-C₆)alkylmorpholino-, (C,-C₉)heteroaryl(C₁-C₆)alkylmorpholino-, thiomorpholino-, (C₁-C₆)acylthiomorpholino-, (C₆-C₁₀)arylthiomorpholino-, (C₁-C₉)heteroarylthiomorpholino-, (C₁-C₆)alkylthiomorpholino-, (C₆-C₁₀)aryl(C₁-C₆)alkylthiomorpholino-, (C₁-C₉)heteroaryl(C₁-C₆)alkylthiomorphotino-, piperidino-, (C₁-C₆)acylpiperidino-, (C₆-C₁₀)arylpiperidino-, (C₁-C₉)heteroarylpiperidino-, (C₁-C₆)alkyl piperidino-, (C₆-C₁₀)aryl(C₁-C₆)piperidino-, (C₁-C₉)heteroaryl(C₁-C₆)alkylpiperidino-, pyrrolidino-, (C₁-C₆)acylpyrrolidino-, (C₆-C₁₀)arylpyrrolidino-, (C₁-C₆)heteroarylpyrrolidino, (C₁-C₆)alkylpyrrolidino-, (C₆-C₁₀)aryl(C₁-C₆)alkylpyrrolidino-, (C₁-C₉)heteroaryl(C₁-C₆)alkylpyrrolidino-(C₁-C₆)alkoxy(C₁-C₆)acyl-, (C₁-C₆)alkylamino(C₁-C₁₀)aryl-, and ((C₁-C₆)alkyl₂amino(C₁-C₆)acyl-;
(c) R⁷, or R⁷Y-, where R⁷ is selected from the group consisting of piperazino-, (C₆-C₁₀)arylpiperazino-, (C₁-C₉)heteroarylpiperazino-, (C₁-C₆)alkylpiperazino-, (C₆-C₁₀)aryl(C₁-C₆)alkylpiperazino-, (C₁-C₉)heteroaryl(C₁-C₆)alkylpiperazino-, morpholino-, (C₆-C₁₀)arylmorpholino-, (C₁-C₉)heteroarylmorpholino-, (C₁-C₆)alkylmorpholino-, (C₆-C₁₀)aryl(C₁-C₆)alkylmorpholino-, (C₁-C₉)heteroaryl(C₁-C₆)alkylmorpholino-, thiomorpholino-, (C₆-C₁₀)arylthiomorpholino-, (C₁-C₉)heteroarylthiomorpholino-, (C₁-C₆)alkylthiomorpholino-, (C₆-C₁₀)aryl(C₁-C₆)alkylthiomorpholino-, (C₁-C₆)heteroaryl(C₁-C₆)alkylthiomorpholino-, piperidino-, (C₆-C₁₀)arylthiopiperidino-, (C₁-C₉)heteroarylthiopiperidino-, (C₁-C₆)alkylthiopiperidino-, (C₆-C₁₀)aryl(C₁-C₆)alkylthiopiperidino-, (C₁-C₉)heteroaryl(C₁-C₆)alkylthiopiperidino-, pyrolidino-, (C₆-C₁₀)arylthiopyrolidino-, (C₁-C₉)heteroarylthiopyrolidino-, (C₁-C₆)alkylthiopyrolidino-, (C₆-C₁₀)aryl(C₁-C₆)alkylthiopyrolidino-, (C₁-C₉)heteroaryl(C₁-C₆)alkylthiopyrolidino-, and Y, if present, is selected from the group consisting of (C₁-C₆)alkyl-, (C₂-C₆)alkenyl-, (C₂-C₆)alkynyl-, amino, oxygen, thio, sulfinyl, sulfonyl, halo(C₁-C₆)alkyl-, and hydroxy(C₂-C₆)alkyl-;
(d) ZR⁸ -, where R⁸ is selected from the group consisting of piperazino-, (C₆-C₁₀)arylpiperazino-, (C₁-C₉)heteroarylpiperazino-, (C₁-C₆)alkylpiperazino-, (C₆-C₁₀)aryl(C₁-C₆)alkylpiperazino-, (C₁-C₉)heteroaryl(C₁-C₆)alkylpiperazino-, morpholino-, (C₆-C₁₀)arylmorpholino-, (C₁-C₉)heteroarylmorpholino-, (C₁-C₆)alkylmorpholino-, (C₆-C₁₀)aryl(C₁-C₆)alkylmorpholino-, (C₁-C₉)heteroaryl(C₁-C₆)alkylmorpholino-, thiomorpholino-, (C₆-C₁₀)arylthiomorpholino-, (C₁-C₉)heteroarylthiomorpholino-, (C₁-C₆)alkylthiomorpholino-, (C₆-C₁₀)aryl(C₁-C₆)alkylthiomorpholino-, (C₁-C₉)heteroaryl(C₁-C₆)alkylthiomorpholino-, piperidino-, (C₆-C₁₀)arylthiopiperidino-, (C₁-C₉)heteroarylthiopiperidino-, (C₁-C₆)alkylthiopiperidino-, (C₆-C₁₀)aryl(C₁-C₆)alkylthiopiperidino-, (C₁-C₉)heteroaryl(C₁-C₆)alkylthiopiperidino-, pyrolidino-, (C₆-C₁₀)arylthiopyrolidino-, (C₁-C₉)heteroarylthiopyrolidino-, (C₁-C₆)alkylthiopyrolidino-, (C₆-C₁₀)aryl(C₁-C₆)alkylthiopyrolidino-, (C₁-C₉)heteroaryl(C₁-C₆)alkylthiopyrolidino-, and Z is selected from the group consisting of (C₁-C₆)alkyl-, (C₂-C₆)alkenyl-, (C₂-C₆)alkynyl-, amino, oxygen, thio, sulfinyl, sulfonyl, halo(C₁-C₆)alkyl-, and hydroxy(C₂-C₆)alkyl-;
(e) two or more of R², when vicinal, together to form one or more further rings of 4, 5, 6 or 7 member atoms selected from the group consisting of phenyl-, naphthyl-, furyl-, thienyl-, thiazolyl-, pyrazolyl-, isothiazolyl-, oxazolyl-, isoxazolyl-, pyrrolyl-, triazolyl-, tetrazolyl-, imidazolyl-, 1,3,5-oxadiazolyl-, 1,2,4-oxadiazolyl-, 1,2,3-oxadiazolyl-, 1,3,5-thiadiazolyl,-1,2,3-thiadiazolyl-, 1,2,4-thiadiazolyl-, pyridyl-, pyrimidyl-, pyrazinyl-, pyridazinyl-, 1,2,4-triazinyl-, 1,2,3-triazinyl-, 1,3,5-triazinyl-, pyrazolo[3,4-b]pyridinyl-, cinnolinyl-, pteridinyl-, purinyl-, 6,7-dihydro-5H-[1]pyrindinyl-, benzo[b]thiophenyl-, 5, 6, 7, 8-tetrahydro-quinolin-3-yl, benzoxazolyl-, benzothiazolyl-, benzisothiazolyl-, benzisoxazolyl-, benzimidazolyl-, thianaphthenyl-, isothianaphthenyl-, benzofuranyl-, isobenzofuranyl-, isoindolyl-, indolyl-, indolizinyl-, indazolyl-, isoquinolyl-, quinolyl-, phthalazinyl-, quinoxalinyl-, quinazolinyl-, benzoxazinyl-, and wherein said ring(s) are optionally substituted by one or more (C₁-C₆)alkyl-(C₂-C₆)alkenyl-, (C₂-C₆)alkynyl-, amino-, halo-, hydroxy-, carboxy-, thiol-, nitro-, cyano-, sulfonic-, halo(C₁-C₆)alkyl-, and hydroxy(C₂-C₆)alkyl-; and
(f) two or more of R² when vicinal, together to form one or more further rings of 3, 4, 5, 6 or 7 member atoms selected from the groups consisting of:
   (i) (C₃-C₁₀)cycloalkyl-, containing zero to two levels of unsaturation, selected from the group consisting of cyclopropyl-, cyclobutyl-, cyclopentyl-, cyclohexyl-, cycloheptyl-, cyclopropenyl-, cyclobutenyl-, cyclopentenyl-, cyclohexenyl-, cycloheptenyl-, 1,3-cyclobutadienyl-, 1,3-cyclopentadienyl-, 1,3-cyclohexadienyl-, 1,4-cyclohexadienyl-, 1,3-cycloheptadienyl-, 1,4-cycloheptadienyl-, bicyclo[3.2.1]octane-, bicyclo [2.2.1] heptane, the norborn-2-ene unsaturated form thereof, and the like, wherein said ring is optionally substituted by hydroxy-, halo-, amino-, trifluoromethyl-, hydroxy(C₂-C₆)alkyl-, (C₁-C₆)alkoxy-, (C₁-C₆)acyloxy-, (C₁-C₆)alkylamino-. ((C₁-C₆)alkyl)₂amino-, (C₁-C₆)acylamino-, cyano-, nitro-, carboxy-, thiol-, sulfonyl-, (C₁-C₆)alkyl-, (C₂-C₆)alkenyl-, (C₂-C₆)alkynyl-, (C₁-C₆)acylamino-, cyano(C₁-C₆)alkyl-, trifluoromethyl(C₁-C₆)alkyl-, (C₁-C₃)alkyl(difluoromethylene)(C₁-C₃)alkyl-, halo(C₁-C₆)alkyl- or nitro(C₁-C₆)alkyl-; and
   (ii) (C₃-C₁₀)heterocycloalkyl- selected from the group consisting of pyrrolidinyl-, tetrahydrofuranyl-, dihydrofuranyl-, tetrahydropyranyl-, pyranyl-, thiopyrany-l, aziridinyl-, oxiranyl-, methylenedioxyl-, isoxazolidinyl,- 1,3-oxazolidin-3-yl-, isothiazolidinyl-, 1,3-thiazolidin-3-yl-, 1,2-pyrazolidin-2-yl-, 1,3-pyrazolidin-1-yl-, piperidinyl-, thiomorpholinyl-, 1,2-tetrahydrothiazin-2-yl-, 1,3-tetrahydrothiazin-3-yl-, tetrahydrothiadiazinyl-, morpholinyl-, 1,2-tetrahydrodiazin-2-yl-, 1,3-tetrahydrodiazin-1-yl-, tetrahydroazepinyl-, piperazinyl-, chromenyl-, chromanyl-, where said ring is optionally substituted by hydroxy-, halo-, amino-, trifluoromethyl-, hydroxy(C₂-C₆)alkyl-, (C₁-C₆)alkoxy-, (C₁-C₆)acyloxy-, (C₁-C₆)alkylamino-, ((C₁-C₆)alkyl)₂amino-, (C₁-C₆)acylamino-, cyano-, nitro-, carboxy-, thiol-, sulfonyl-, (C,-C₆)alkyl-, (C₂-C₆)alkenyl-, (C₂-C₆)alkynyl-, (C₁-C₆)acylamino-, cyano(C₁-C₆)alkyl-, trifluoromethyl(C₁-C₆)alkyl-, (C₁-C₃)alkyl(difluoromethylene)(C₁-C₃)alkyl-, halo(C₁-C₆)alkyl- or nitro(C₁-C₆)alkyl-;

wherein any (C₁-C₆)alkyl-, (C₂-C₆)alkenyl-, (C₂-C₆)alkynyl-, (C₃-C₁₀)cycloalkyl- or (C₃-C₁₀)heterocycloalkyl- groups that are, or comprise a portion of, said one to four optional R² substituents are themselves optionally substituted by deuterium-, hydroxy-, amino-, trifluoromethyl-, cyano-, nitro-, carboxy-, (C₁-C₄)alkoxy-, (C₁-C₆)acyloxy-, (C₁-C₆)alkylamino-, ((C₁-C₆)alkyl)₂amino-, (C₁-C₆)alkyl- (C₂-C₆)alkenyl-, (C₂-C₆)alkynyl-, (C₁-C₆)acylamino-, (C₃-C₁₀)cycloalkyl-, (C₃-C₁₀)heterocycloalkyl-, cyano(C₁-C₆)alkyl-, trifluoromethyl(C₁-C₆)alkyl-, nitro(C₁-C₆)alkyl-, and (C₁-C₆)acylamino; and.
R³ represents one or more optional substituents on a ring carbon atom, including at X where X is -CH₂-, selected from the groups consisting of (C₁-C₆)alkyl- , trihalo(C₁-C₆)alkyl- that is preferably trifluoromethyl-, deuterium, and fluorine.

According to the practice of the invention, with respect to the structural component of compounds of formula I that is represented by 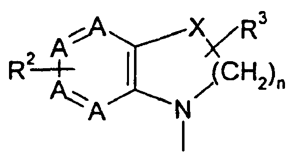
preferred examples include those where the ring structure is contributed by 1,2,3,4-tetrahydroquinoline; 1,2,3,4-tetrahydroquinoxaline; 3,4-dihydro-1H-quinoxaline-2-one (also named 2-oxo-1,2,3,4-tetrahydroquinoxaline); 3,4-dihydro-2H-benzo[1,4]oxazine; 2,3-dihydr 1H-indole; and 3,4-dihydro-2H-benzo[1,4]thiazine, respectively, as depicted below.

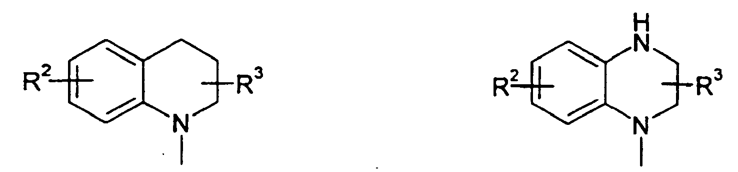

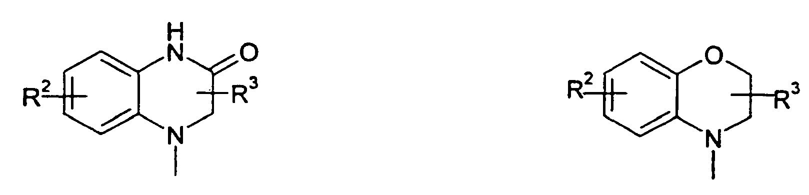

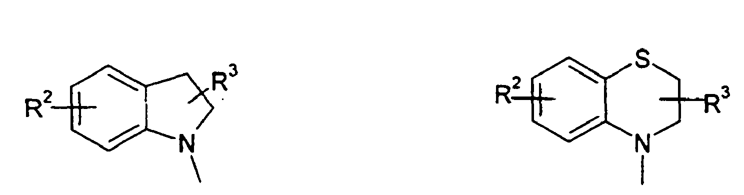

Preferred examples of the above six structures include 6-methoxy-1,2,3,4-tetrahydroquinoline; 4-methyl-1,2,3,4-tetrahydroquinoline; 7-(trifluoromethyl)-1,2,3,4-tetrahydroquinoline; 8-methyl-1,2,3,4-tetrahydroquinoline, 6-hydroxy-1,2,3,4-tetrahydroquinoline; 8-chloro-1,2,3,4-tetrahydroquinoline, 7-chloro-1,2,3,4-tetrahydroquinoline; 6-benzyloxy-7-methoxy-1,2,3,4-tetrahydroquinoline: 6,7-dimethyl-1,2,3,4-tetrahydroquinoxaline; 1,2,3,4-tetrahydroquinoxaline; 1-phenylsulfonyl-1,2,3,4-tetrahydroquinoxaline; 6-Methyl-1,2,3,4-tetrahydroquinoline; 3,4-dihydro-2H-benzo[1,4]oxazine; 5-Fluoro-2,3-dihydro-1H-indole; 1,2,3,4-tetrahydroquinoxaline; and 3,3-dimethyl-2,3-dihydro-1 H-indole.

In additional embodiments of the invention, the 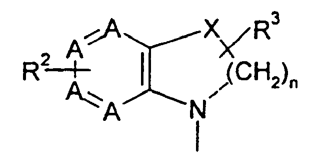
structure is defined, for example, by 2,3-Dihydro-1H-pyrrolo[2,3-b]pyridine; 2,3-Dihydro-1H-pyrrolo[2,3-c]pyridine; 2,3-Dihydro-1H-pyrrolo[3,2-c]pyridine; 2,3-Dihydro-1H-pyrrolo[3,2-b]pyridine; 6,7-Dihydro-5H-pyrrolo[3,2-d]pyrimidine; 6,7-Dihydro-5H-pyrrolo[3,2-d][1,2,3]triazine; 6,7-Dihydro-5H-pyrrolo[2,3-d][1,2,3]triazine; 1,4,5,7-Tetraaza-indan; 1,4,6,7-Tetraaza-indan; 6,7-Dihydro-5H-pyrrolo[2,3-c]pyridazine; 2,3-Dihydro-1H-pyrrolo[2,3-d]pyridazine; 6,7-Dihydro-5H-pyrrolo[3,2-c]pyridazine; 6,7-Dihydro-5H-pyrrolo[2,3-b]pyrazine; 6,7-Dihydro-5H-pyrimido[4,5-b][1,4]oxazine; 5,6,7,8-Tetrahydro-pteridine; 1,2,3,4-Tetrahydro-pyrido[2,3-b]pyrazine; 1,2,3,4-Tetrahydro-pyrido[3,4-b]pyrazine; 1,2,3,4-Tetrahydro-pyrido[3,4-b]pyrazine; 1,2,3,4-Tetrahydro-pyrido[2,3-b]pyrazine; 5,6,7,8-Tetrahydro-pyrazino[2,3-c]pyridazine; 5,6,7,8-Tetrahydro-pteridine; 1,2,3,4-Tetrahydro-pyrazino[2,3-d]pyridazine; 5,6,7,8-Tetrahydro-pyrazino[2,3-c]pyridazine; 1,2,3,4-Tetrahydro-pyrazino[2,3-b]pyrazine; 5,6,7,8-Tetrahydro-pyrazino[2,3-e][1,2,4]triazine; 5,6,7,8-Tetrahydro-pyrazino[2,3-e][1,2,4]triazine; 5,6,7,8-Tetrahydro-pyrazino[2,3-d][1,2,3]triazine; 5,6,7,8-Tetrahydro-pyrazino[2,3-d][1,2,3]triazine; 2,3-Dihydro-1H-4-oxa-1,5-diaza-naphthalene; 2,3-Dihydro-1H-4-oxa-1,6-diaza-naphthalene; 3,4-Dihydro-2H-1-oxa-4,6-diaza-naphthalene; 3,4-Dihydro-2H-1-oxa-4,5-diaza-naphthalene; 7,8-Dihydro-6H-5-oxa-1,2,8-triaza-naphthalene; 3,4-Dihydro-2H-1-oxa-4,6,7-triaza-naphthalene; 6,7-Dihydro-5H-8-oxa-1,2,5-triaza-naphthalene; 3,4-Dihydro-2H-1-oxa-4,5,8-triaza-naphthalene; 7,8-Dihydro-6H-pyrimido[5,4-b][1,4]oxazine; 6,7-Dihydro-5H-pyrimido[4,5-b][1,4]oxazine; 6,7-Dihydro-5H-8-oxa-1,2,3,5-tetraaza-naphthalene; 6,7-Dihydro-5H-8-oxa-1,2,4,5-tetraaza-naphthalene; 7,8-Dihydro-6H-5-oxa-1,2,3,8-tetraaza-naphthalene; 6,7-Dihydro-5H-8-oxa-1,2,4,5-tetraaza-naphthalene; 2,3-Dihydro-1H-pyrido[2,3-b][1,4]thiazine; 2,3-Dihydro-1H-4-thia-1,6-diaza-naphthalene; 3,4-Dihydro-2H-1-thia-4,6-diaza-naphthalene; 3,4-Dihydro-2H-pyrido[3,2-b][1,4]thiazine; 7,8-Dihydro-6H-5-thia-1,2,8-triaza-naphthalene; 3,4-Dihydro-2H-1-thia-4,6,7-triaza-naphthalene; 6,7-Dihydro-5H-8-thia-1,2,5-triaza-naphthalene; 6,7-Dihydro-5H-pyrimido[4,5-b][1,4]thiazine; 7,8-Dihydro-6H-pyrimido[5,4-b][1,4]thiazine; 3,4-Dihydro-2H-1-thia-4,5,8-triaza-naphthalene; 6,7-Dihydro-5H-8-thia-1,2,4,5-tetraaza-naphthalene; 7,8-Dihydro-6H-5-thia-1,2,4,8-tetraaza-naphthalene; 7,8-Dihydro-6H-5-thia-1,2,3,8-tetraaza-naphthalene; 6,7-Dihydro-5H-8-thia-1,2,3,5-tetraaza-naphthalene: 5,6,7,8-Tetrahydro-pyrido[3,2-d]pyrimidine; 1,2,3,4-Tetrahydro-pyrido[2,3-d]pyridazine; 5,6,7,8-Tetrahydro-pyrido[2,3-b]pyrazine; 5,6,7,8-Tetrahydro-pyrido[3,2-e][1,2,4]triazine; 5,6,7,8-Tetrahydro-pyrido[2,3-e][1,2,4]triazine; 5,6,7,8-Tetrahydro-pyrido[3,2-d][1,2,3]triazine; and 5,6,7,8-Tetrahydro-pyrido[2,3-d][1,2,3]triazine.

It is additionally preferred that one or more of substituents R² be selected from the groups consisting of:
(a) (C₁-C₆)alkyl-, (C₁-C₆)alkynl-, (C₁-C₆)alkoxy-, trihalo(C₁-C₆)alkyl- that is preferably trifluoromethyl-, (C₁-C₆)alkylamino-, ((C₁-C₆)₂)dialkylamino-, amino-, cyano, and halo-; and
(b) benzyloxy-, phenylsulfonyl-, phenylaminocarbonyl-, (C₁-C₉)heteroarylsulfonyl- and (C₁-C₉)heteroarylaminocarbonyl-, optionally substituted by one or more groups selected from the group consisting of (C₁-C₆)alkyl-, (C₂-C₆)alkynyl-, trihalo(C₁-C₆)alkyl- that is preferably trifluoromethyl- , (C₁-C₆)alkoxy-, (C₁-C₆)alkylamino-, ((C₁-C₆)₂)alkylamino-, and halo.

According to the practice of the invention, preferred examples of R¹ include (C₆-C₁₀)aryl-, and (C₁-C₉) heteroaryl- wherein said R¹ group is optionally substituted by one or more groups, each independently selected from hydroxy, halo, amino, (C₁-C₆)alkyl-, (C₁-C₆)alkoxy-, trihalo(C₁-C₆)alkyl- that is preferably trifluoromethyl-, (C₁-C₆)alkynl-, (C₁-C₆)alkylamino-, ((C₁-C₆)₂)dialkylamino-, carboxy-, (C₁-C₆)alkoxycarbonyl-, (C₁-C₆)acyloxy-,and (C₁-C₆)acylamino-.

In a preferred embodiment of the invention, R¹ is a (C₁-C₉)heteroaryl- group selected from the group consisting of pyridyl-, indazolyl-, indolyl-, 1,3-dihydro-benzoimidazol-2-one, thienyl-, oxazoyl-, 2H-pyrazolyl-, 1H-pyrazolyl-, isooxazoyl-, thiazolyl(fix,name), and isothiazoyl-, and is optionally substituted by one or more groups, each independently selected from hydroxy-, halo-, amino-, (C₁-C₆)alkyl-, (C₁-C₆)alkoxy-, trihalo(C₁-C₆)alkyl- that is preferably trifluoromethyl-, (C₁-C₆)alkynl-, (C₁-C₆)alkylamino-, ((C₁-C₆)₂)dialkylamino-, carboxy-, (C₁-C₆)alkoxycarbonyl-, (C₁-C₆)acyloxy-, and (C₁-C₆)acylamino-.

In an additionally preferred embodiment of the invention, R¹ is phenyl, optionally substituted with one to five substituents, that are each independently selected from hydroxy-, halo-, amino-, (C₁-C₆)alkyl-, (C₁-C₆)alkoxy-, trihalo(C₁-C₆)alkyl- that is preferably trifluoromethyl-, (C₁-C₆)alkynl-, (C₁-C₆)alkylamino-, ((C₁-C₆)₂)dialkylamino-, carboxy, (C,-C₆)alkoxycarbonyl-, (C₁-C₆)acyloxy-, and (C₁-C₆)acylamino-.

Particularly preferred examples of R¹ include 3,4,5-trimethoxyphenyl-; 2,3-dimethyl-1H-indol-5-yl; 3,4-dihydro-2H-quinolin-1-yl; and 6-morpholin-4-yl-pyridin-3-yl.

Representative compounds of the invention include:
(a) 1-[(2-anilino)-4-pyrimidinyl]-6-methyl-1,2,3,4-tetrahydroquinoline;
(b) 1-[2-[(4-bromophenyl)amino]-4-pyrimidinyl]-6-methyl-1,2,3,4-tetrahydroquinoline;
(c) 1-[2-[(4-methoxyphenyl)amino)-4-pyrimidinyl]-6-methyl-1,2.3,4-tetrahydroquinoline;
(d) 1-[2-[(1H-indazole-5-yl)]-4-pyrimidyl]-6-methyl-1,2,3,4-tetrahydroquinoline;
(e) 1-[2-[(4-phenoxyphenyl)amino]-4-pyrimidinyl]-6-methyl-1,2,3,4-tetrahydroquinoline;
(f) 1-[2-[(3,4-dimethoxyphenyl)amino]-4-pyrimidinyl]-6-methyl-1,2,3,4-tetrahydroquinoline;
(g) 1-[2-[(3,4,5-trimethoxyphenyl)amino]-4-pyrimidinyl]-6-methyl-1,2,3,4-tetrahydroquinoline;
(h) 1-[2-[(4,N-phenylaminophenyl)amino]-4-pyrimidinyl]-6-methyl-1,2,3,4-tetrahydroquinoline;
(i) [4-(6-Methyl-3,4-dihydro-2H-quinolin-1-yl)-pyrimidin-2-yl]-(6-morpholin-4-yl-pyridin-3-yl)-amine;
(j) 5-[4-(6-Methyl-3,4-dihydro-2H-quinolin-1-yl)-pyrimidin-2-ylamino]-1,3-dihydro-benzoimidazol-2-one;
(k) (2,3-Dimethyl-1H-indol-5-yl)-[4-(6-methyl-3,4-dihydro-2H-quinolin-1-yl)-pyrimidin-2-yl]-amine;
(l) [4-(6-Methyl-3,4-dihydro-2H-quinolin-1-yl)-pyrimidin-2-yl]-(2-methyl-2H-pyrazol-3-yl)-amine;
(m) (6-Methoxy-pyridin-3-yl)-[4-(6-methyl-3,4-dihydro-2H-quinolin-1-yl)-pyrimidin-2-yl]-amine;
(n) (4-Fluoro-3-methyl-phenyl)-[4-(6-methyl-3,4-dihydro-2H-quinolin-1-yl)-pyrimidin-2-yl]-amine;
(o) (5-Cyclopropyl-2H-pyrazol-3-yl)-[4-(6-methyl-3,4-dihydro-2H-quinolin-1-yl)-pyrimidin-2-yl]-amine;
(p) 4-Benzyl-N 3 -[4-(6-methyl-3,4-dihydro-2H-quinolin-1-yl)-pyrimidin-2-yl]-1H-pyrazole-3,5-diamine;
(q) [4-(6-Methyl-3,4-dihydro-2H-quinolin-1-yl)-pyrimidin-2-yl]-(4-methyl-thiazol-2-yl)-amine; and
(r) [4-(6-Methyl-3,4-dihydro-2H-quinolin-1-yl)-pyrimidin-2-yl]-(5-methyl-1H-pyrazol-3-yl)-amine.

Additional preferred compounds of the invention include [4-(3,4-Dihydro-2H-quinolin-1-yl)-pyrimidin-2-yl]-(6-pyrrolidin-1-yl-pyridin-3-yl)-amine; (1-Cyclopentyl-1H-indol-6-yl)-[4-(6-methyl-3,4-dihydro-2H-quinolin-1-yl)-pyrimidin-2-yl]-amine; [4-(6-Methyl-3,4-dihydro-2H-quinolin-1-yl)-pyrimidin-2-yl]-oxazol-4-yl-amine; (3,4-Dichloro-phenyl)-[4-(6-methyl-3,4-dihydro-2H-quinolin-1-yl)-pyrimidin-2-yl]-amine; and [4-(3,4-Dihydro-2H-quinolin-1-yl)-pyrimidin-2-yl]-isothiazol-3-yl-amine.

Additional preferred compounds of the invention include:
2-({5-[4-(2,3-Dihydro-benzo[1,4]oxazin-4-yl)-pyrimidin-2-ylamino]-pyridin-2-yl}-methyl-amino)-ethanol;
N-{5-[4-(3-Oxo-3,4-dihydro-2H-quinoxalin-1-yl)-pyrimidin-2-ylamino]-pyridin-2-yl}-acetamide;
3-Chloro-N-[4-(4-methyl-3-oxo-3,4-dihydro-2H-quinoxalin-1-yl)-pyrimidin-2-yl]-benzamide;
[4-(2,3-Dihydro-benzo[1,4]thiazin-4-yl)-pyrimidin-2-yl]-oxazol-4-yl-amine;
N-[4-(5-Fluoro-2,3-dihydro-indol-1-yl)-pyrimidin-2-yl]-3-methoxy-benzenesulfonamide;
[4-(5,6-Dihydro-pyrrolo[2,3-d]pyrimidin-7-yl)-pyrimidin-2-yl]-(2-trifluoromethyl-phenyl)-amine;
6-Methoxy-1-[2-(pyridazin-3-ylamino)-pyrimidin-4-yl]-2,3-dihydro-1H-quinolin-4-one;
2-{5-[4-(3,4-Dihydro-2H-quinoxalin-1-yl)-pyrimidin-2-ylamino]-indol-1-yl}-ethanol;
(2H-Pyrazol-3-yl)-[4-(7-trifluoromethyl-3,4-dihydro-2H-quinolin-1-yl)-pyrimidin-2-yl]-amine;
1-[4-(3,4-Dihydro-2H-[1,5]naphthyridin-1-yl)-pyrimidin-2-yl]-3-ethyl-urea;
1-[4-(2,3-Dihydro-benzo[1,4]oxazin-4-yl)-pyrimidin-2-yl]-3-(2-ethoxy-ethyl)-urea;
[4-(3,3-Dimethyl-2,3-dihydro-indol-1-yl)-pyrimidin-2-yl]-carbamic acid tert-butyl ester;
3-Cyano-N-[4-(7-methoxy-2,3,4,5-tetrahydro-benzo[b]azepin-1-yl)-pyrimidin-2-yl]-benzamide;
Isoxazol-4-yl-[4-(2,3,4,5-tetrahydro-benzo[b][1,4]diazepin-1-yl)-pyrimidin-2-yl]-amine;
(3,4-Dichloro-phenyl)-[4-(3,4-dihydro-2H-benzo[b][1,4]thiazepin-5-yl)-pyrimidin-2-yl]-amine;
(6-Aziridin-1-yl-pyridin-3-yl)-[4-(5-methanesulfonyl-2,3-dihydro-indol-1-yl)-pyrimidin-2-yl]-amine;
N²-Cyclopropyl-N⁵-[4-(6-fluoro-3,4-dihydro-2H-quinolin-1-yl)-pyrimidin-2-yl]-pyridine-2,5-diamine; and
Benzo[1,3]dioxole-5-carboxylic acid [4-(6-fluoro-3,4-dihydro-2H-quinolin-1-yl)-pyrimidin-2-yl]-amide

The compounds and pharmaceutical compositions of this invention include all conformational isomers of compounds of formula I (e.g., cis and trans isomers, whether or not involving double bonds). The compounds of the invention include all optical isomers of the compounds of formula I (e.g., enantiomers and diastereomers), as well as racemic, diastereomeric and other mixtures of all such isomers. This invention further relates to tautomers and stereoisomers of the compounds of formula (I), and mixtures of any of the aforementioned forms.

The present invention also relates to the pharmaceutically acceptable acid addition salts of compounds of the formula (I). The acids which are used to prepare the pharmaceutically acceptable acid addition salts of the aforementioned base compounds of this invention are those which form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [i.e., 1,1'-methylene-bis-(2-hydroxy-3- naphthoate)]salts.

The present invention also relates to the pharmaceutically acceptable base addition salts of compounds of the formula (I). The chemical bases that may be used as reagents to prepare pharmaceutically acceptable base salts of those compounds of formula I that are acidic in nature are those that form non-toxic base salts with such compounds. Such non-toxic base salts include, but are not limited to those derived from such pharmacologically acceptable cations such as alkali metal cations (e.g., potassium and sodium) and alkaline earth metal cations (e.g., calcium and magnesium), ammonium or water-soluble amine addition salts such as N-methylglucamine-(meglumine), and the lower alkanolammonium and other base salts of pharmaceutically acceptable organic amines.

The subject invention also includes isotopically-labelled compounds, which are identical to those recited in Formula (I), but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine and chlorine, such as ²H , ³H, ¹³C, ¹⁴C, ¹⁵N, ¹⁸O, ¹⁷O, ³¹P, ³²P, ³⁵S, ¹⁸F, and ³⁶Cl, respectively. Compounds of the present invention, prodrugs thereof, and pharmaceutically acceptable salts of said compounds or of said prodrugs which contain the aforementioned isotopes and/or other isotopes of other atoms are within the scope of this invention. Certain isotopically-labelled compounds of the present invention, for example those into which radioactive isotopes such as ³H and ¹⁴C are incorporated, are useful in drug and/or substrate tissue distribution assays. Tritiated, i.e., ³H. and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labelled compounds of Formula (1) of this invention and prodrugs thereof can generally be prepared by carrying out the procedures disclosed in the Schemes and/or in the Examples and Preparations below, by substituting a readily available isotopically labelled reagent for a non-isotopically labelled reagent.

In the practice of the invention, preferably the mammalian patient is a human, but the invention is broadly applicable to the treatment of other mammals, such as farm animals and companion animals.

The present invention relates to a pharmaceutical composition for treatment or prevention of conditions in a mammalian patient, where therapeutic benefit is achieved by downregulating T-cell mediated immune response, comprising an effective amount of a compound according to formula I, and a pharmaceutical carrier.

The present invention relates to a pharmaceutical composition for the treatment or prevention of transplant rejection in a mammal, comprising an effective amount of a compound according to formula I, and a pharmaceutical carrier.

The present invention relates to a pharmaceutical composition for treatment or prevention of autoimmune disease in a mammal, comprising an effective amount of a compound according to formula I, and a pharmaceutical carrier.

The present invention also relates to a pharmaceutical composition for treating inflammatory disease in a mammal, comprising an effective amount of a compound according to formula I, and a pharmaceutical carrier.

The present invention also relates to a pharmaceutical composition for treating allergy in a mammal, comprising an effective amount of a compound according to formula 1, and a pharmaceutical carrier.

The present invention also relates to a pharmaceutical composition for treating T-cell leukemias and T-cell lymphomas in a mammal, comprising an effective amount of a compound according to formula I, and a pharmaceutical carrier.

The present invention also relates to a pharmaceutical composition for the treatment of diseases in a mammal, wherein treatment can be effected by inhibiting activation of T-cells, or the results of said activation, comprising an effective amount of a compound according to formula I, and a pharmaceutical carrier.

The present invention relates to a method for treating or preventing transplant rejection in a mammal.

The present invention further relates to a method for treating or preventing autoimmune disease in a mammal.

The present invention further relates to a method for treating or preventing allergic disease in a mammal.

The present invention further relates to a method for treating or preventing inflammatory disease in a mammal.

The present invention also relates to inhibiting T-cell mediated immune response in a mammalian patient, wherein this is beneficial to the mammal nothwithstanding that said immune function was within the normal range.

In the practice of said methods, there is administered a pharmaceutical composition of the invention comprising a compound according to formula I, and a pharmaceutical carrier.

An additional embodiment of the invention includes a compound of formula (I), or a pharmaceutically acceptable salt, solvate, or hydrate of any such compound, for administration in pharmaceutically acceptable form, in combination with one or more additional agents which have an anti-inflammatory effect, or which themselves can modulate one or more components or processes of the mammalian immune system.

### Definitions

In connection with the practice of the invention, the following definitions will generally apply.

The term "treating", as used herein, refers to reversing, alleviating, inhibiting the progress of, or preventing the disorder or condition to which such term applies, or one or more symptoms of such disorder or condition. The term "treatment", as used herein, refers to the act of treating, as "treating" is defined immediately above.

The term "transplant" refers to transplanted cells, tissues, and organs or portions of organs. The term "transplant" also refers to macromolecules that are normally associated with the transplanted cells, tissues, and organs, whether intracellular, membrane associated, or extracellular in nature. In this regard, a category of macromolecules that is of particular interest refers to those associated with the extracellular matrix of a transplanted tissue. T-cell mediated immune response against such macromolecules can cause failure of the transplant as a whole. "Transplant" includes both allografts and xenografts.

The term "alkyl", as used herein, unless otherwise indicated, includes saturated monovalent hydrocarbon radicals having straight, branched or cyclic moieties or combinations thereof. Similarly, the terms "alkenyl" and "alkynl" define hydrocarbon radicals having straight, branched or cyclic moities wherein at least one double bond, or at least one triple bond, respectively, is present. Such definitions also apply when the alkyl, alkenyl or alkynyl group is present within another group, such as alkoxy or alkylamine.

The term "alkoxy", as used herein, includes O-alkyl groups wherein "alkyl" is as defined above.

The term "halo", as used herein, unless otherwise indicated, includes fluoro, chloro, bromo or iodo.

An "aryl" group as used herein, unless otherwise indicated, includes an organic radical derived from a monocyclic or bicylic (C₆-C₁₀) aromatic hydrocarbon compound by removal of a hydrogen radical from a ring carbon of the aryl compound. An aryl group is optionally substituted by one or more substituents wherein, unless otherwise indicated, selection of each optional substituent is independent of selection of any other optional substituents, and perferably the number of optional substituents is between 0 and 3, more preferably between 0 and 2. It will be appreciated that the preferred number of substituents is determined in part by facility of synthesis. Representative aryl groups are phenyl and naphthyl.

A "heteroaryl" group as used herein, unless otherwise indicated, includes an organic radical derived from a monocyclic or bicyclic (C₁-C₉) aromatic heterocyclic compound by removal of a hydrogen radical from a ring atom of the heteroaryl compound, said ring atom being uncharged in said compound. A heteroaryl group is optionally substituted by one or more substituents wherein, unless otherwise indicated, selection of each optional substituent is independent of selection of any other optional substituents, and perferably the number of optional substituents is between 0 and 3, more preferably between 0 and 2. It will be appreciated that the preferred number of substituents is determined in part by facility of synthesis. Representative heteroaryl- groups include furyl-, thienyl-, thiazolyl-, pyrazolyl-, isothiazolyl-, oxazolyl-, isoxazolyl-, pyrrolyl-, triazolyl-, tetrazolyl-, imidazolyl-, 1,3,5-oxadiazolyl-, 1,2,4-oxadiazolyl-, 1,2,3-oxadiazolyl-, 1,3,5-thiadiazolyl-, 1,2,3-thiadiazolyl-, 1,2,4-thiadiazolyl-, pyridyl-, pyrimidyl-, pyrazinyl-, pyridazinyl-, 1,2,4-triazinyl-, 1,2,3-triazinyl-, 1,3,5-triazinyl-, pyrazolo[3,4-b]pyridinyl-, cinnolinyl-, pteridinyl-, purinyl-, 6,7-dihydro-5H-[1]pyrindinyl-, benzo[b]thiophenyl-, 5, 6, 7, 8-tetrahydro-quinolin-3-yl-, benzoxazolyl-, benzothiazolyl-, benzisothiazolyl-, benzisoxazolyl-, benzimidazolyl-, thianaphthenyl-, isothianaphthenyl-, benzofuranyl-, isobenzofuranyl-, isoindolyl-, indolyl-, indolizinyl-, indazolyl-, isoquinolyl-, quinolyl-, phthalazinyl-, quinoxalinyl-, quinazolinyl-, and benzoxazinyl-; and the like.

A "cycloalkyl" group as used herein, unless otherwise indicated, includes an organic radical derived from a monocyclic (C₃-C₁₀)cycloalkyl- compound, by removal of a hydrogen radical from a ring carbon of the cycloalkyl- compound. A cycloalkyl- group is optionally substituted by one or more substituents wherein, unless otherwise indicated, selection of each optional substituent is independent of selection of any other optional substituents, and perferably the number of optional substituents is between 0 and 3, more preferably between 0 and 2. It will be appreciated that the preferred number of substituents is determined in part by facility of synthesis. Representative cycloalkyl- groups include cyclopropyl-, cyclobutyl-, cyclopentyl-, cyclohexyl-, cycloheptyl-, cyclopropenyl-, cyclobutenyl-, cyclopentenyl-, cyclohexenyl-, cycloheptenyl-, 1,3-cyclobutadienyl-, 1,3-cyclopentadienyl-, 1,3-cyclohexadienyl-, 1,4-cyclohexadienyl-, 1,3-cycloheptadienyl-, 1,4-cycloheptadienyl-, bicyclo[3.2.1]octane-, bicyclo- [2.2.1] heptane-, and the norborn-2-ene unsaturated form thereof. Thus, the term cycloalkyl- also includes cycloalkenyl- groups having one or two double bonds.

A "heterocycloalkyl" group as used herein, unless otherwise indicated, includes an organic radical derived from a monocyclic (C₃-C₁₀)heterocycloalkyl compound by removal of a hydrogen radical from a ring atom of the heterocycloalkyl compound. A heterocycloalkyl group is optionally substituted by one or more substituents wherein, unless otherwise indicated, selection of each optional substituent is independent of selection of any other optional substituents, and perferably the number of optional substituents is between 0 and 3, more preferably between 0 and 2. It will be appreciated that the preferred number of substituents is determined in part by facility of synthesis. Representative heterocycloalkyl- groups include pyrrolidinyl-, tetrahydrofuranyl-, dihydrofuranyl-, tetrahydropyranyl-, pyranyl-, thiopyranyl-, aziridinyl-, oxiranyl-, methylenedioxyl-, chromenyl-, isoxazolidinyl-, 1,3-oxazolidin-3-yl-, isothiazolidinyl-, 1,3-thiazolidin-3-yl-, 1,2-pyrazolidin-2-yl-, 1,3-pyrazolidin-1-yl-, piperidinyl-, thiomorpholinyl-, 1,2-tetrahydrothiazin-2-yl-, 1,3-tetrahydrothiazin-3-yl-, tetrahydrothiadiazinyl-, morpholinyl-, 1,2-tetrahydrodiazin-2-yl-, 1,3-tetrahydrodiazin-1-yl-, tetrahydroazepinyl-, piperazinyl-, and chromanyl-.

In connection with the terms "aryl" group, "heteroaryl" group, "cycloalkyl" group and "heterocycloalkyl" group, as herein defined, the term "optionally substituted" means that one or more chemically and pharmaceutically acceptable functional groups may be bonded thereto. Such a group contributes properties useful to production, storage, or use of the inventive compounds as pharmaceuticals, or at least does not substantially negate their pharmacological activity. Such suitable substituents may be determined by those skilled in the art. Illustrative examples of suitable substituents include, but are not limited to, hydroxy, halo, amino, trifluoromethyl, carboxy, (C₁-C₆)alkoxy-, (C₁-C₆)acyloxy-, (C₁-C₆)alkylamino-, ((C,-C₆)alkyl)₂amino-, (C₁-C₆)acylamino-, cyano, nitro, (C₁-C₆)alkyl-, (C₂-C₆)alkenyl-, (C₂-C₆)alkynyl-, (C₁-C₆)acylamino-, cyano(C₁-C₆)alkyl-, trifluoromethyl(C₁-C₆)alkyl-, nitro(C₁-C₆)alkyl-, (C₁-C₃)alkyl(difluoromethylene)(C₁-C₃)alkyl-, (C₁-C₆)acylamino(C₁-C₆)alkyl-, (C₁-C₆)alkoxy(C₁-C₆)acylamino-, amino(C₁-C₆)acyl-, amino(C₁-C₆)acyl(C₁-C₆)alkyl-, (C₁-C₆)alkylamino(C₁-C₆)acyl-, ((C₁-C₆)alkyl)₂amino(C₁-C₆)acyl-, (C₃-C₁₀)cycloalkyl(C₁-C₆)alkyl-, (C₁-C₆)acyloxy(C₁-C₆)alkyl-, (C₂-C₆)alkoxy(C₁-C₆)alkyl-, piperazinyl(C₁-C₆)alkyl-, (C,-C₆)acylamino(C₁-C₆)alkyl-, (C₆-C₁₀)aryl(C₁-C₆)alkoxy(C₁-C₆)alkyl-, (C₂-C₉)heteroaryl(C₁-C₆)alkoxy(C₁-C₆)alkyl-, (C₁-C₆)alkylthio(C₁-C₆)alkyl-, (C₆-C₁₀)arylthio(C₁-C₆)alkyl-, (C₁-C₆)alkylsulfinyl(C₁-C₆)alkyl- (C₆-C₁₀)arylsulfinyl(C₁-C₆)alkyl-, (C₁-C₆)alkylsulfonyl(C₁-C₆)alkyl-, (C₆-C₁₀)arylsulfonyl(C₁-C₆)alkyl-, amino(C₁-C₆)alkyl-, (C₁-C₆)alkylamino(C₁-C₆)alkyl-, (C₁-C₆)alkyl(difluoromethylene)-, (C₁-C₃)alkyl(difluoromethylene)(C₁-C₃)alkyl-, (C₁-C₆)alkoxy(C₁-C₆)acyl-, (C₁-C₆)alkylamino(C₁-C₆)acyl-, ((C₁-C₆)alkyl)₂amino(C₁-C₆)acyl-, (C₆-C₁₀)aryl-, (C₅-C₉)heteroaryl-, (C₆-C₁₀)aryl(C₁-C₆)alkyl-, (C₂-C₉)heteroaryl(C₁-C₆)alkyl-, (C₆-C₁₀)aryl(C₆-C₁₀)aryl-. (C₆-C₁₀)aryl(C₆-C₁₀)aryl(C₁-C₆)alkyl- (C₃-C₁₀)cycloalkyl-, (C₃-C₆)cycloalkyl(C₁-C₆)alkyl-, (C₃-C₁₀)heterocycloalkyl-, (C₃-C₁₀)heterocycloalkyl(C₁-C₆)alkyl-, hydroxy(C₂-C₆)alkyl-, (C₁-C₆)acyloxy(C₂-C₆)alkyl-, (C₁-C₆)alkoxy(C₂-C₆)alkyl-, piperazinyl(C₁-C₆)alkyl-, (C₁-C₆)acylamino(C₁-C₆)alkyl-, (C₆-C₁₀)aryl(C₁-C₆)alkoxy(C₁-C₆)alkyl-, (C₂-C₉)heteroaryl(C₁-C₆)alkoxy(C₁-C₆)alkyl-, (C₁-C₆)alkylthio(C₁-C₆)alkyl-, (C₆-C₁₀)arylthio(C₁-C₆)alkyl-, (C₁-C₆)alkylsulfinyl(C₁-C₆)alkyl-, (C₆-C₁₀)arylsulfinyl(C₁-C₆)alkyl-, (C₁-C₆)alkylsulfonyl(C₁-C₆)alkyl-, (C₆-C₁₀)arylsulfonyl(C₁-C₆)alkyl-, amino(C₁-C₆)alkyl-, (C₁-C₆)alkylamino(C₁-C₆)alkyl-, and ((C₁-C₆)alkyl)₂amino(C₁-C₆)alkyl.

The present invention, and additional embodiments thereof, are further described in the detailed description of the invention which follows directly.

### Detailed Description of the Invention

### Practice of the Invention

Characterized in its broadest sense, the present invention is directed to recognizing conditions where therapeutic benefit can be achieved by downregulating T-cell mediated immune response. Depending on the involved clinical condition, the immune response that is downregulated may be normal or abnormal, or otherwise beneficial. In a preferred embodiment of the invention, therapeutic modulation of T-cell mediated processes is achieved in a mammalian patient through the administration of compounds that interefere with, or otherwise modify, T-cell activation, and/or other T-cell functions that result from such activation. Generally speaking, such events binding of an antigen (including a self-antigen) to a T-cell..

T-cell mediated immune responses are involved, for example, in delayed-type hypersensitivity, lysis of tumor cells or cells that express viral antigens, resistance to intracellular pathogens, allergic contact dermatitis, rejection of allografts and xenografts, graft versus host reactions, certain autoimmune diseases, and various types of allergy. Preventing activation of T-cells thus represents an important point of intervention for cell mediated immune responses when this is therapeutically appropriate.

For purposes of description, clinical conditions that can be treated according to the practice of the present invention may be divided into three principal categories:
(1) prevention of transplant rejection, where cells, tissues, or organs (or parts thereof) have been transplanted into a patient, and the normal and otherwise proper immune response against the transpant must be prevented;
(2) treatment of various disease states where "normal" immune system action leads, directly or indirectly, to clinical manifestations that are not desired, for example is circumstances involving damaging inflammation, and allergy ; and
(3) various disease states, characterized in whole or part as autoimmune diseases, wherein an immune response is mounted against the body's own tissues.

The present invention is practiced with respect to all of the diseases, clinical conditions, and the like, that are discussed below.

Of course, given the complex nature of many disease states or clinical conditions, more than one of the above categories may be relevant in particular circumstances. It must be emphasized that these categories are arbitrary and merely descriptive. For example, with respect to insulin dependent (juvenile/type I) diabetes (see below), treatment at onset is best characterized as prevention of autoimmune disease, whereas immune suppression in the mature disease may be for the purpose of protecting transplanted pancreatic beta cells.

An additional category relates to suppression of an immune response against a therapeutic macromolecule that is administered to (or expressed in) a patient, wherein said macromolecule is otherwise foreign to the patient. Examples include proteins expressed from gene therapy and covalently modified proteins, such as PEGylated proteins. It should be noted that, on occasion, immune response can also occur even against a protein that has an amino acid sequence identical to that encoded by a patient's own genome. Under these circumstances, the protein may have been expressed in the body at levels, or in places, that are atypical, or in an atypical combination with other macromolecules, or the immune response may occur for unknown reasons.

In elaboration of these demonstrative categories, examples of circumstances where it is appropriate to prevent activation of T-cells, and/or to down regulate T-cell mediated immune response, are as follows.
(1) Organ, tissue, and cell transplants between individuals of the same species (allograft transplantations) are an important medical procedure for which there is often no substitute, since the complicated functions of the kidney, heart, bone marrow, lung, or liver, for example, cannot be duplicated. Unfortunately, transplants between individuals very often end in rejection of the transplant. For example, if an allograft of donor skin is positioned on an excised area of a recipient patient, the graft will at first successfully vascularize and proliferate. However, after a brief period of time (perhaps 7-10 days), the site typically becomes subject to severe inflammation, and the transplanted skin withers and is sloughed. A repeat transplant from the same donor is subject to more rapid rejection. It is well known that such events are mediated by transplantation antigens including the major histocompatibility complex of glycoproteins (MHC, also termed HLA in humans) which are expressed from perhaps 20 genes. The HLA protein products, when expressed on cell surfaces, play a major role in presenting peptide fragments of antigen to T-cells at their antigen-specific receptors, the TcR. The HLA glycoproteins vary tremendously from individual to individual, and when recognized by receptive T-cells, are responsible for the typically complete rejection of the donor tissue. Interfering with activation of the T-cells, will permit a wider variety of transplant procedures to be successfully performed.
   Xenograft transplants are also subject to rejection. Important examples of such transplants include primate-to-human and pig-to-human, and involve numerous organs and tissues including, without limitation, heart and heart valve, kidney, skin, pancreas, and the like.
(2) It is well known that antigens can elicit inflammatory responses having an intensity that does not necessary correlate with the level of circulating antibodies. Delayed-type hypersensitivity (DTH) reactions are an example of such responses, in which activated T-cells participate.
   In a general sense, inflammation is a protective response to local injury or other abnormal condition, and involves blood vessels, cells that circulate in the blood vessels , and nearby connective tissue. The early phase of an inflammatory response typically begins with hyperemia, edema, and margination of circulating white blood cells. The white blood cells (including phagocytic leukocytes, and lymphocytes) then penetrate between the endothelial cells of the blood vessel wall, and enter the tissue. The leakage of water and protein into the damaged area (edema) also permits entry of antibodies, facilitates washing away of toxic substances and debris, and permits direct contact of defending white cells, including phagocytic cells, with infecting agents. Local inflammatory responses are also associated with systemic changes including fever, and an increase in the number of circulating leukocytes.
   A large number of additional cellular components participate in the inflammatory response. In this regard, the complement system should be mentioned. Complement consists of about 25-30 proteins, some of which circulate in blood plasma, and some of which are membrane bound. Some complement proteins bind, in an ordered sequence, to antigen-antibody complexes on target cells, facilitating cell lysis. Other complement proteins facilitate clearance of antibody-antigen complexes from the body, others prevent cell lysis or excessive inflammation, while peptide fragments of other complement proteins stimulate inflammation.
   Particular proinflammatory activities of activated complement proteins (and their fragments) include: release of histamine and other vasoactive mediators from mast cells to increase permeability of the capillaries at an affected site; attracting polymorphonuclear leukocytes and macrophages to sites of inflammation and enhancing the activity thereof; lysis of gram negative bacteria, and damage to the membranes of many other types of targeted cells, including self cells, bearing foreign antigens; and facilitating adherence of leukocytes and macrophages to the surface of cells targeted for ingestion (such as bacteria and viruses, or self cells) via antigen-antibody complexes.
   General interrelationships between the immune system, the complement system, and inflammatory conditions are well recognized in the art. For example, CD4+ T-cells are known to release lymphokines, such as λ-interferon, which stimulate macrophages to release substances that increase inflammation at an affected site, permitting destruction of invading pathogens. It is thus apparent that the inflammatory response, and cell mediated immune processes, reflect a complex set of interrelated mechanisms that permit response to injury, and infection. Unfortunately, the component pathways of this complex system occasionally work in ways adverse to the body, preventing appropriate response to disease states, or actually causing the disease states themselves. As a result, the specificity of the compounds of the invention contribute to their therapeutic value.
   Particular inflammatory diseases that may be treated according to the practice of the invention include psoriasis, and inflammatory diseases of the gastrointestinal tract such as ulcerative colitis and Crohn's disease.
   An additional category of inappropriate immune response includes those processes involving antibody-mediated (intermediate-type) hypersensitivity reactions (typically involving IgE antibodies), and which are often termed allergies. Generally speaking, allergy or hypersensitivity may be defined as an altered state, induced by an antigen, in which pathologic reactions can be subsequently elicited by exposure to that antigen , or to structurally similar substances. Representative examples include asthma, hay fever, hives, infantile eczema, atopic dermatitis, and gastrointestinal disturbances. Activated T-cells are also involved in these inappropriate immune responses.
(3) A considerable number of disease states involve circumstances where an individual produces antibodies and reactive T-cells against his or her own proteins or cells. Such circumstances are a significant exception to the general principle of self-tolerance, whereinby self-molecules do not trigger an immune response. A significant number of mechanisms are recognized whereinby an autoimmune response to a self-antigen can be triggered. Self-antigens may represent self-proteins that are denatured or otherwise modified after being produced on ribosomes, thus exposing novel epitopes (immune-recognized domains, typically short peptide or carbohydrate sequences) which are then taken up and processed by antigen-presenting cells and made available to receptive T-cells. Loss of thyroid function following chronic inflammation of the thyroid (Hashimoto's disease) may involve such an autoimmune pathway. Another thyroid pathology, thyroiditis followed by hyperthyroidism, may be explained by immune recognition of the cell surface receptor for thyroid stimulating hormone, however with the less typical result that antibody binding to the recognized cells results in their stimulation, not death.

Similarly, an autoimmune response may be mounted against an altered distribution of self-antigen. For example, self antigen from an organ may be exposed only after serious injury, and immune-mediated inflammation (see below) may then enhance and perpetuate the primary response. Persistent viral infections may also trigger autoimmune-like disease. It is not uncommon for host antibodies to bind to viral particles without neutralizing them, and it is possible that the resulting virus-antibody complexes may, over time, result in the production of what appear to be anti-self antibodies. Additionally, it is thought that certain T-cells termed Supressor Cells(Tₛ) may act to suppress immune response to particular self-antigens. Defective production of such Tₛ cells could permit activation of autoreactive T-cells whose action would need to be suppressed by therapeutic intervention.

Finally, many of the most widespread and serious autoimmune diseases may have their origin in the phenomenon of antigenic mimicry. The epitopes (immune-recognized domains) of antigens of infecting bacteria and viruses may bear considerable resemblance to similar structural motifs (for example peptide sequences) in mammalian proteins. Thus, an immune response intended to be specific against structural features of an invading pathogen may unfortunately also target identical or nearly identical macromolecular structural elements of self proteins.

Infections with some viruses are statistically associated with the onset of myasthenia gravis and insulin-dependent (juvenile/ type 1) diabetes. In type I diabetic disease, the panceatic beta (islet) cells that produce insulin are selectively destroyed. The human disease appears to depend on activated CD4+ T-cells and corrleates with the inherited presence in patients of specific HLA alleles (for example, DR3 or DR4 homozygotes, and DR4/DR3 heterozygotes have a high probability of contracting the disease). Although the exact beta cell autoantigen and autoantigen epitope(s) are unknown, homology with a Coxsackie B virus protein is suspected. It has been proposed that in susceptible individuals, having particular HLA alleles, presentation of viral antigen to T-cells unfortunately leads to cross-recognition of beta cell surface proteins by the immune system, with the result of gradual death of the entire beta cell population.

Thus, as the type I disease progresses, the patient becomes insulin dependent. However, diagnostic procedures for susceptibility to type I disease are known in the art, and the at-risk patient can be placed on a program of life-long immune suppression to prevent full onset of the autoimmune disease (thereby protecting surviving insulin-producing pancreatic cells). In those cases where no insulin producing cells survive (full type I disease), the disease may be treated by transplantation of pancreatic islet cells. In such case, the approach of the present invention is best characterized as prevention of transplant rejection.

The mechanisms of causation and progression for rheumatoid arthritis appear to share in-common features with those for type I diabetes. In rheumatoid arthritis, synovial membranes enclosing joint spaces are subject to very pronounced infiltration by lymphocytes, macrophages, and other cells. Compared to a control population, rheumatoid arthritis patients tend to express DR4, DR1, and DRw10 HLA haplotypes at high frequency. It is again likely that presentation of a self peptide by particular surface HLA molecules to receptive T-cells is essential to development of the disease. Consequently, suppression of resultant T-cell activation, and downstream signalling events, is an important strategy for therapeutic intervention. Additional autoimmune diseases that can be treated according to the practice of the invention include lupus (including systemic lupus erythematosus and lupus nephritis), pemphigus vulgaris (in which the recognized self-antigen is found in epidermal cells), thrombocytopenic purpura (in which the level of functional platelets falls to very low levels), and multiple sclerosis (wherein demyelinating activity may result from infection of the nervous system by a virus, bringing appropriate antigen in contact with T-cells).

The present invention provides highly specific inhibitors of T-cell *lck* tyrosine kinase. Administration of such compounds interferes with T-cell activation, and subsequent signalling events, thereby providing an effective means of intervention in the above-identified cell mediated immune responses.

The present invention also provides a method of treating or preventing T-cell leukemias, T-cell lymphomas, and other T-cell malignancies, whether the affected cells are primarily circulating or non-circulating. In this embodiment of the invention, it is not necessary that the involved T cells be activated.

The present invention also provides a method of treating the multifaceted pathology of Alzheimer's disease, and the complications thereof.

### Pharmaceutical formulations

The compounds of the present invention that are basic in nature are capable of forming a wide variety of different salts with various inorganic and organic acids. Although such salts must be pharmaceutically acceptable for administration to animals, it is often desirable in practice to initially isolate the compound of the present invention from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the base compounds of this invention are readily prepared, for example, by treating the base compound with a substantially equivalent amount of the chosen mineral or organic acid in an aqueous solvent medium or in a suitable organic solvent, such as methanol or ethanol. Upon careful evaporation of the solvent, the desired solid salt is readily obtained. The desired acid salt can also be precipitated from a solution of the free base in an organic solvent by adding to the solution an appropriate mineral or organic acid.

Those compounds of the present invention that are acidic in nature, are capable of forming base salts with various pharmacologically acceptable cations. Examples of such salts include the alkali metal or alkaline-earth metal salts and particularly, the sodium and potassium salts. These salts are all prepared by conventional techniques. The chemical bases which are used as reagents to prepare the pharmaceutically acceptable base salts of this invention are those which form non-toxic base salts with the acidic compounds of the present invention. Such non-toxic base salts include those derived from such pharmacologically acceptable cations as sodium, potassium calcium and magnesium, etc. These salts can easily be prepared by treating the corresponding acidic compounds with an aqueous solution containing the desired pharmacologically acceptable cations, and then evaporating the resulting solution to dryness, preferably under reduced pressure. Alternatively, they may also be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum yields of the desired final product.

The compositions of the present invention may be formulated in a conventional manner using one or more pharmaceutically acceptable carriers. Thus, the active compounds of the invention may be formulated for oral, buccal, intranasal, parenteral (e.g., intravenous, intramuscular or subcutaneous) or rectal administration or in a form suitable for administration by inhalation or insufflation. The active compounds of the invention may also be formulated for sustained delivery.

For oral administration, the pharmaceutical compositions may take the form of, for example, tablets or capsules prepared by conventional means with pharmaceutically acceptable excipients such as binding agents (e.g., pregelatinized maize starch, polyvinylpyrrolidone or hydroxypropyl methylcellulose); fillers (e.g., lactose, microcrystalline cellulose or calcium phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, methyl cellulose or hydrogenated edible fats); emulsifying agents (e.g., lecithin or acacia); non-aqueous vehicles (e.g., almond oil, oily esters or ethyl alcohol); and preservatives (e.g., methyl or propyl p-hydroxybenzoates or sorbic acid).

For buccal administration, the composition may take the form of tablets or lozenges formulated in conventional manner.

The active compounds of the invention may be formulated for parenteral administration by injection, including using conventional catheterization techniques or infusion. Formulations for injection may be presented in unit dosage form, e.g., in ampules or in multi-dose containers, with an added preservative. The compositions may take such forms as suspensions, solutions or emulsions in oily or aqueous vehicles, and may contain formulating agents such as suspending, stabilizing and/or dispersing agents. Alternatively, the active ingredient may be in powder form for reconstitution with a suitable vehicle, e.g., sterile pyrogen-free water, before use.

The active compounds of the invention may also be formulated in rectal compositions such as suppositories or retention enemas, e.g., containing conventional suppository bases such as cocoa butter or other glycerides.

For intranasal administration or administration by inhalation, the active compounds of the invention are conveniently delivered in the form of a solution or suspension from a pump spray container that is squeezed or pumped by the patient or as an aerosol spray presentation from a pressurized container or a nebulizer, with the use of a suitable propellant, e.g., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. The pressurized container or nebulizer may contain a solution or suspension of the active compound. Capsules and cartridges (made, for example, from gelatin) for use in an inhaler or insufflator may be formulated containing a powder mix of a compound of the invention and a suitable powder base such as lactose or starch.

A proposed dose of the active compounds of the invention for oral, parenteral or buccal administration to the average adult human for the treatment of the conditions referred to above (e.g., rheumatoid arthritis) is 0.1 to 1000 mg of the active ingredient per unit dose which could be administered, for example, 1 to 4 times per day.

Aerosol formulations for treatment of the conditions referred to above (e.g., asthma) in the average adult human are preferably arranged so that each metered dose or "puff" of aerosol contains 20 pg to 1000 µg of the compound of the invention. The overall daily dose with an aerosol will be within the range 0.1 mg to 1000 mg. Administration may be several times daily, for example 2, 3, 4 or 8 times, giving for example, 1, 2 or 3 doses each time.

As is well recognized, the precise dose, and method and timing of administration thereof, are capable of determination by those skilled in the art, and depend upon numerous factors including the activity of the therapeutic compound, the properties of the formulation thereof, the nature and location of the target tissue, and the particulars of the disease state as it exists in a particular patient.

A compound of formula (I) administered in a pharmaceutically acceptable form either alone or in combination with one or more additional agents which modulate a mammlian immune system or with one or more anti-inflammatory agents. Such additional agents may include, but are not limited to, cyclosporin A (e.g. Sandimmune® or Neoral®), rapamycin, FK-506 (tacrolimus), leflunomide, CD40L Ab, methotrexate, FTY720, deoxyspergualin and analogs thereof, mycophenolate (e.g. Cellcept®), azathioprine (e.g. Imuran®), daclizumab (e.g. Zenapax®), OKT3 (e.g. Orthocolone®), AtGam, aspirin, acctaminophen, ibuprofen, naproxen, piroxicam, and antiinflmmatory steroids (e.g. prednisolone or dexamethasone). Such agents may be administered as part of the same or ofg separate dosage forms, via the same or different routes of administration, and on the same or different administration schedules according to standard pharmaceutical practice.

As examples, FK506 (Tacrolimus) may be given orally at 0.10-0.15 mg/kg body weight, every 12 hours, within first 48 hours postoperative, for example. Dose is monitored by measurement of serum Tacrolimus trough levels.

Cyclosporin A (Sandimmune® oral or intravenous formulation, or Neoral®, oral solution or capsules) may be given orally at 5 mg/kg body weight, every 12 hours within 48 hours postoperative. Dose is monitored by measurement of blood cyclosporin A trough levels.

The compounds of the present invention can be formulated for sustained delivery according to methods well known to those of ordinary skill in the art. Examples of such formulations can be found in U. S. Patents 3,538,214, 4,060,598, 4,173,626, 3,119,742, and 3,492,397. Additionally, the compounds of the present invention can be formulated using tehcnologies that provide continuous dosing via the digestive tract including, for example, osmotic systems, such as described in U.S. Patent 4,612.008.

### Synthesis of Compounds of the Invention

The following reaction schemes illustrate preparation of compounds of the present invention.

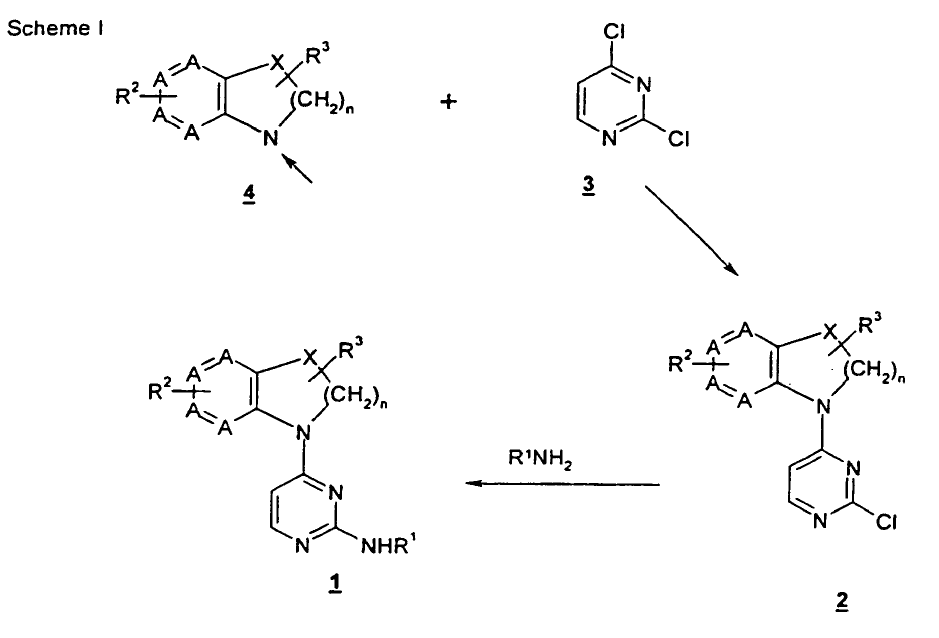

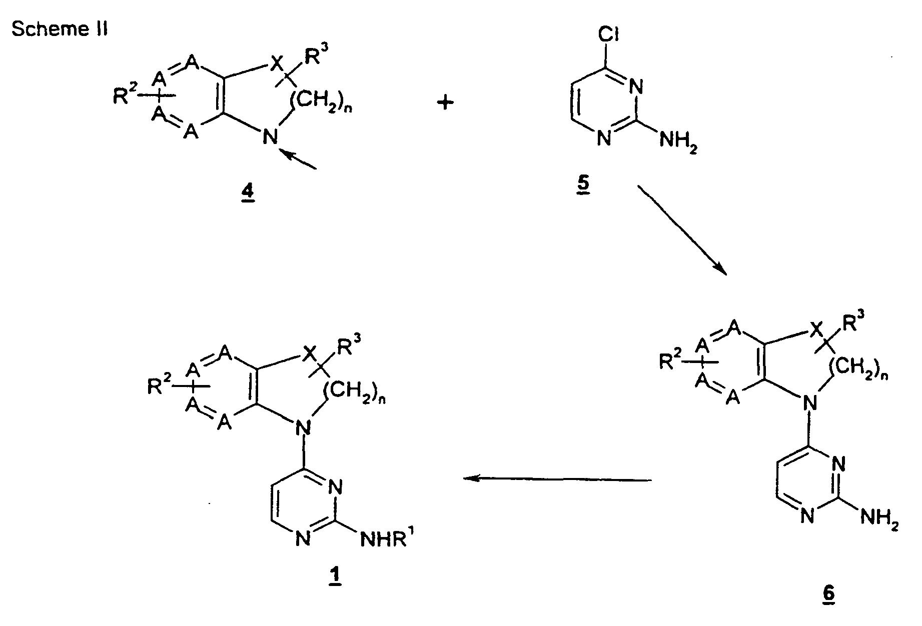

### General reaction conditions

Generally speaking, the compounds of the invention are made in a two step process First, the reactive nitrogen atom of compound **4** (indicated by an arrow above) preferentially displaces the 4-chloro group of 2,4-dichloropyrimidine (compound **3)** under basic conditions to form compound **2.** In a second step, generally in the presence of an acid catalyst, compound **2** is treated with an amine to form compound **1,** wherein the amine nitrogen displaces the 2-chloro atom of the pyrimidine.

The reaction of compounds **4** and **3** is best conducted under basic conditions. Examples of suitable conditions include refluxing with a trialkylamine such as triethylamine in an alcohol solvent such as ethanol. As aforementioned, the 4-chloro group in 2,4-dichloropyrimidine is selectively displaced in the formation of compounds **2**.

Subsequent treatment of compound **2** with an amine yields compound **1** as product. Selection of the appropriate amine is determined by the required structure of the product. The reaction solvent is chosen to both facilitate the solubility of the amine, and its subsequent reaction. For example, in the case of aniline or substituted anilines, the amine may be dissolved in an acetone/water solution in the presence of a catalytic amount of HCl, followed by heating for 18 hours at 50°C, for example. Mixtures of THF/water also provide combinations of solvent and reaction conditions that are generally useful. Conditions suited to reaction of any particular amine are readily determined.

Some compounds of the invention are made in a two step process shown in Scheme II where, in the first step, the reactive nitrogen of a compound **4** displaces the 4-chloro group of 2-amino-4-chloropyrimidine (compound 5) under basic conditions to form compound **6**. In a second step, optionally in the presence of a tertiary amine base such as triethylamine, compound **6** is treated with a suitable agent, such as an acylating or sulfonylating agent to give compound **1.** Examples of suitable acylating agents include, but are not limited to, acid chlorides, sulfonyl chlorides such as aryl or heteroarylsulfonyl chlorides, carbamoyl chlorides, chloroformates, and isocyanates, or a carboxylic acid in the presence of a suitable coupling agent such as a 1-(3-dimethylaminopropyl)-3-ethylcarbodiimide hydrochloride in a suitable solvent such as THF. In this regard, it is understood that the acylating agent is chosen to deliver R¹ according to the general formula I.

Both compound **3** (2,4-dichloropyrimidine) and compound **5** (2-amino-4-chloropyrimidine) are readily prepared and commercially available.

Compounds **4** are also readily prepared, or are commercially available, and may contain one or more optional substituents R², and one or more optional substituents R³ (where it is understood that the R³ group is attached to a ring carbon (including at X, if X is a methylene group), as indicated below.

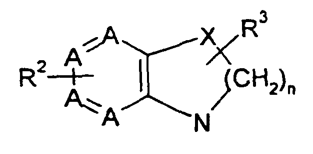

Representative ring structures for compound **4** include 1,2,3,4-tetrahydroquinoline; 1,2,3,4-tetrahydroquinoxaline; 3,4-Dihydro-1H-quinoxaline-2-one (2-oxo-1,2,3,4-tetrahydroquinoxaline); 3,4-Dihydro-2H-benzo[1,4]oxazine (3,4-2H-2H-benzo(1,4)oxazin-6-ol); 2,3-Dihydro-1H-indole; and 3,4-Dihydro-2H-benzo[1,4]thiazine, respectively, as shown below.

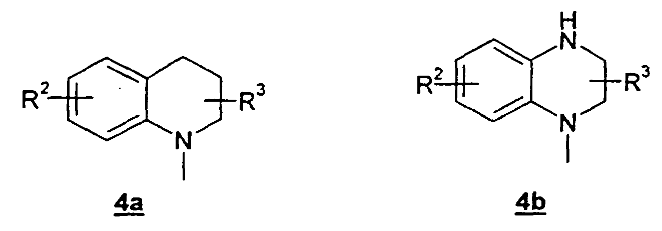

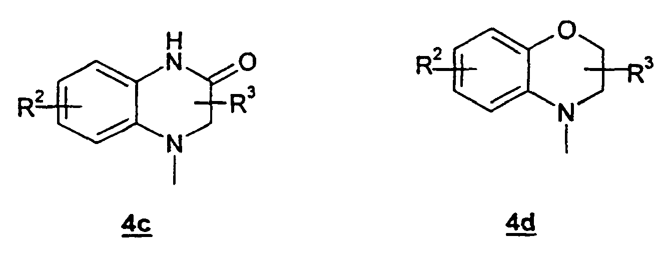

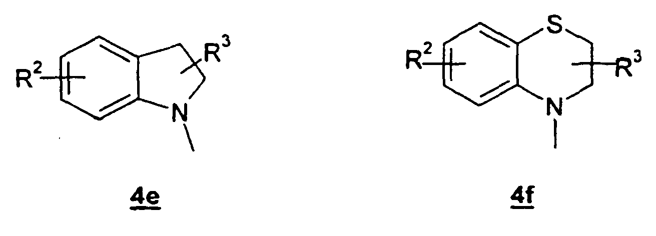

Structures 4a to 4f above, for example, are commercially available where R² and R³ are hydrogen. Additional species that are readily available in commerce include 7-methyl-1,2,3,4-tetrahydroquinoline; 6-methyl-1,2,3,4-tetrahydroquinoline; 5-methyl-1,2,3,4-tetrahydroquinoline; 6-methoxy-1,2,3,4-tetrahydroquinoline; 7-trifluoromethyl-1,2,3,4-tetrahydroquinoline; 6-fluoro-2-methyl-1,2,3,4-tetrahydroquinoline; 2-methyl-1,2,3,4-tetrahydroquinoline; 2,3-dihydro-1H-quinolin-4-one; 6-methoxy-2,3-dihydro-1H-quinolin-4-one; 2-methyl-2,3-dihydro-1H-indole; 2,3-dimethyl-2,3-dihydro-1H-indole; 5-fluoro-2,3-dihydro-1H-indole; 5-bromo-2,3-dihydro-1H-indole; 5-methanesulfinyl-2,3-dihydro-1H-indole; 5-methanesulfonyt-2.3-dihydro-1H-indofe; and 2,3-dihydrobenzothiazole.

With respect to structures of type 4, additional synthetic approaches include the following:
(1) with respect to synthesis of the 2,3-dihydro-1H-indole ring system 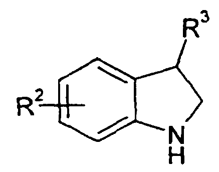 methods are disclosed in E. C. Taylor et al., Tetrahedron, 43, p.5145 (1987).
(2) with respect to synthesis of the 3,4-Dihydro-1H-quinoxalin-2-one ring system 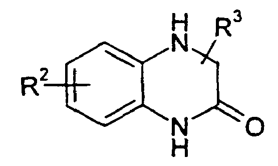 methods are disclosed in R.W. Holley, et al., J. A. Chem. Soc., 74, p. 3069, 1952 and R.E. TenBrink, J. Med. Chem. 37, p. 758, 1994;
(3) with respect to synthesis of the 1,2,3,4-tetrahydro-[1,5]naphthyridine ring system 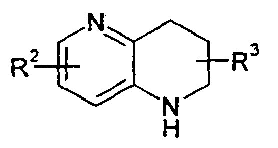 methods are disclosed in C. E. Neipp et al., Tetrahedron Letters, 38, p. 7499, 1997;
(4) with respect to synthesis of the 6,7,8,9-tetrahydro-5H-pyrido[2,3-b]azepine ring system 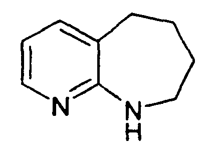 methods are disclosed in E.M. Hawes et al., Tetrahedron, 10, p. 39, 1973;
(5) with respect to synthesis of the 5,6,7,8-tetrahydropyrido {2,3-*d*]pyrimidine ring system, 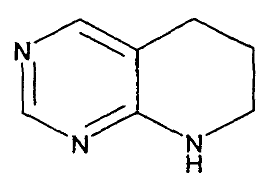 methods are disclosed in S. Kobayashi, Bull. Chem. Soc. Jpn., 46, p. 2835, 1973, and involve reaction of δ-valerolactam with formamide;
(6) with respect to synthesis of the 5,6,7,8-tetrahydropyrrolo {2.3-*d*]pyrimidine ring system; 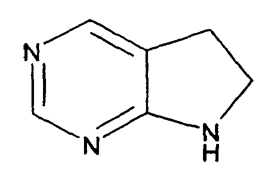 methods are disclosed in S. Kobayashi, Bull. Chem. Soc. Jpn., 46, p. 2835, 1973, and involve reaction of γ-butyrolactam with formamide;
(7) a large series of pyridopyridines are commercially available that can be reduced to the corresponding cyclic amines using known reduction methods including those cited in N. Ikekawa, et al., Chem. Pharm Bull., 6, p.408, 1958; W.L.F. Armarego, J. Chem. Soc. (C), VIOL?, p. 377, 1967, and H. Rapoport et al., J. Org. Chem., 28, p. 1753. 1963. For example, 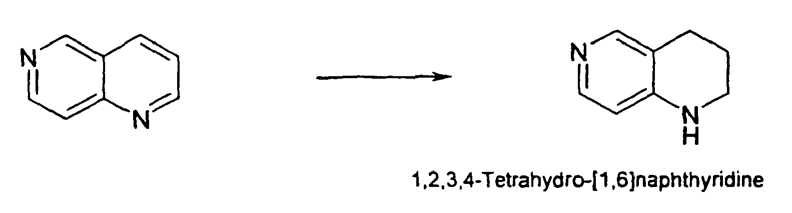 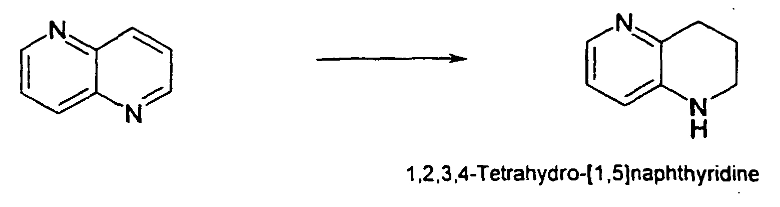 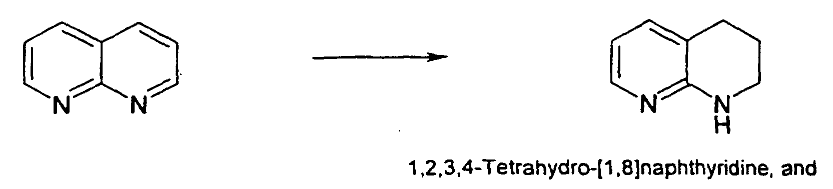 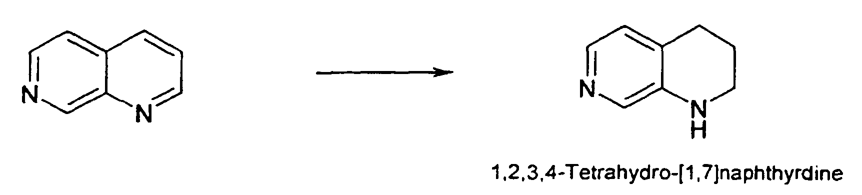
(8) a large series of cyclic amides and diamides are commercially available that can be reduced with lithium aluminum hydride, or other suitable reducing agents as recognized in the art, to give the corresponding amines. For example, 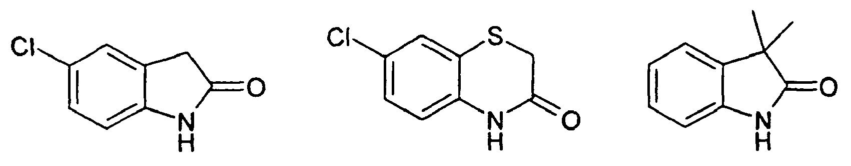 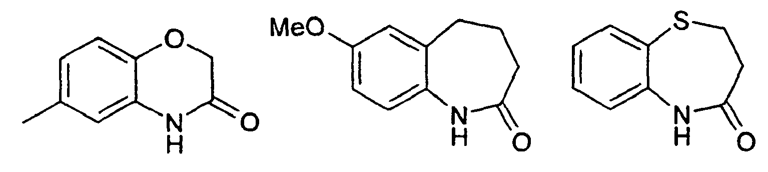 and

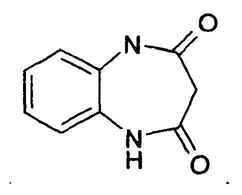

As aforementioned, preferred R² groups include halo, trifluoromethyl, (C₁-C₆)alkyl, hydroxy, (C₁-C₆)alkoxy-, and benzyloxy, for example, and compounds **4** in Schemes I and II containing them are commercially available or are readily synthesized.

Additionally, in the case where compound **4** is a 1,2,3,4-tetrahydroquinoxaline **(4b),** a ring nitrogen atom thereof may be substituted by (C₁-C₆)alkyl, (C₁-C₆)alkylsulfonyl, or phenylsulfonyl, and the like. In such cases it may be preferred to attach this substituent after the completion of all other chemistry, for example using an (C₁-C₆)alkylbromide, (C₁-C₆)alkylsulfonylchloride, or phenylsulfonylchloride.

The present invention is evidenced by the following examples.

### Examples

### Example 1 preparation of 1-[(2-anilino)-4-pyrimidinyl]-6-methyl-1,2,3,4-tetrahydroquinoline.

6-Methyl-1,2,3,4 tetrahydroquinoline (33.7 mmol) was added to a mixture of 2,4-dichloropyrimidine (33.5 mmol) and triethylamine (37 mmot) in EtOH (62 mL). The reaction mixture was refluxed for 3 h, cooled to room temperature, and the volatiles removed by rotary evaporation. The remaining solid was extracted with EtOAc/H₂O. The EtOAc layers were combined, dried over MgSO₄, filtered and the volatiles were then removed rotary evaporation. The residual solid was recrystallized from EtOAc/hexane to give compound 6, as depicted below, 1-(2-Chloro-pyrimidin-4-yl)-6-methyl-1,2,3,4-tetrahydroquinoline. [¹H-NMR(DMSO-d₆): 8.87 (d, J=6, 1 H) 7.28 (d, J=8,1H) 7.04 (s, 1 H) 7.00 (d, J=8, 1H) 6.93 (d, J=6, 1 H) 3.79 (m, 2H) 2.65 (m, 2H) 1.85 (m. 2H); m/z 260 (M+1)] The 4-chloro group in 2,4-dichloropyrimidine was selectively displaced.

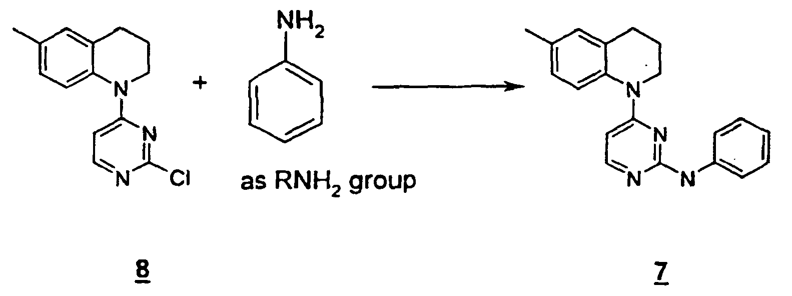

Subsequent treatment of compound 8 with the appropriate amine (in this case aniline) yields the product 7, in which the 2-chloro atom on the pyrimidine is replaced by the intended substituent. Aniline (0.173 mmol) was added to 1-(2-chloro-pyrimidin-4-yl)-6-methyl-1,2,3,4 tetrahyrdoquinoline (0.154 mmol) in 3 mL of acetone/water/ hydrochloric acid (10:15:0.2) and heated to 50°C for 18 h. The reaction mixture was cooled to room temperature, and the precipitated solid was then filtered and recrystallized from ethyl acetate to give 1-[(2-anilino)-4-pyrimidinyl]-6-methyl-1,2,3,4-tetrahydroquinoline [¹H-NMR (DMSO-d₆): 7.95 (d, J=8, 1H) 7.54 (d, J=8, 2H) 7.35 (m, 3H) 7.14 (m, 1 H) 7.08 (m, 2H) 6.60 (d, J=8, 1 H) 3.91 (m, 2H) 2.67 (m, 2H) 2.28 (s, 3H) 1.93 (m, 2H) m/z: 317(M+1)]

In Examples 2-8 below, synthetic procedures were very similar except for selection of the appropriate amine to react at the 2-chloro position of the pyrimidine ring.

### Example 2 -1-[2-[(4-bromophenyl)amino]-4-pyrimidinyl]-6-methyl-1,2,3,4 tetrahydroquinoline.

¹H-NMR(DMSO-d₆): 7.96 (d, J=7, 1H) 7.53 (m, 4H) 7.31 (d, J=8 1H) 7.09 (s, 1H) 7.07 (d, J=7 1H) 6.60 (d, J=8, 1H) 3.90 (m, 2H) 2.66 (m, 2H) 2.28 (s, 3H) 1.91 (m, 2H) m/z: 395,397(M+1)

### Example 3 1-[2-[(4-methoxyphenyl)amino]-4-pyrimidinyl]-6-methyl-1,2,3,4-tetrahydroquinoline.

¹H-NMR(DMSO-d₆): 7.88 (b, 1H) 7.41 (d, J=8, 2H) 7.30 (d, J=8, 1H) 7.09 (s, 1H) 7.06 (d, J=8, 1 H) 6.95 (d, J=8, 2H) 6.55 (d, J=8, 1H) 3.89 (m, 2H) 3.74 (s, 3H) 2.67 (m, 2H) 2.28 (s, 3H) 1.91 (m, 2H) m/z: 347(M+1)

### Example 4 1-[2-[(1H-indazole-5-yl)]-4-pyrimidyl]-6-methyl-1,2,3,4-tetrahydroquinoline

¹H-NMR(DMSO-d₆): 9.11 (s, 1H) 8.17 (s, 1H) 7.95 (d, J=6, 1H) 7.84 (s, 1H) 7,49 (d, J=8. 1 H) 7.35 (d, J=8, 1 H) 7.27 (d, J=8, 1 H) 6.99 (s, 1 H) 6.96 (d, J=8, 1 H) 6.34 (d, J=7, 1H) 3.83 (m, 2H) 2.64 (m,2H) 2.22 (s, 3H) 1.85 (m,2H) m/z: 357 (M+1)

### Example 5 1-[2-[(4-phenoxyphenyl)amino]-4-pyrimidinyl]-6-methyl-1,2,3,4-tetrahydroquinoline.

¹H-NMR(DMSO-d₆): 9.20 (s,1H) 7.94 (d, J=8, 1H) 7.71 (d, J=9 2H) 7.31 (m; 2H) 7.24 (d, J=8, 1 H) 7.03 (t, J=8, 1H) 6.98 (s, 1 H) 6.95 (d, J=8 1 H) 6.90 (m, 4H) 6.37 (d, J=7, 1H) 3.83 (m, 2H) 2.64 (m, 2H) 2.22 (s, 3H) 1.85 (m, 2H). m/z: 409 (M=1)

### Example 6 1-[2-[(3,4-dimethoxyphenyl)amino]-4-pyrimidinyl]-6-methyl-1,2,3,4-tetrahydroquinoline.

m/z: 377 (M+1)

### Example 7 1-[2-[(3,4,5-trimethoxyphenyl)amino]-4-pyrimidinyl]-6-methyl-1,2,3,4-tetrahydroquinoline.

m/z: 407 (M+1)

### Example 8 1-(2-[(4,N-phenylaminophenyl)amino]-4-pyrimidinyl]-6-methyl-1,2,3,4-tetrahydroquinoline.

m/z: 408 (M+1)

### Example 9 [4-(6-Methyl-3,4-dihydro-2H-quinolin-1-yl)-pyrimidin-2-yl]-(6-morpholin-4-yl-pyridin-3-yl)-amine.

m/z 403 (M+1)

### Example 10 5-[4-(6-Methyl-3,4-dihydro-2H-quinolin-1-yl)-pyrimidin-2-ylamino]-1,3-dihydro-benzoimidazol-2-one.

m/z 373 (M+1)

### Example 11 (2,3-Dimethyl-1H-indol-5-yl)-[4-(6-methyl-3,4-dihydro-2H-quinolin-1-yl)-pyrimidin-2-yl]-amine.

m/z 384 (M+1)

### Example 12 [4-(6-Methyl-3,4-dihydro-2H-quinolin-1-yl)-pyrimidin-2-yl]-(2-methyl-2H-pyrazol-3-yl)-amine.

m/z 321 (M+1)

### Example 13 (6-Methoxy-pyridin-3-yl)-[4-(6-methyl-3,4-dihydro-2H-quinolin-1-yl)-pyrimidin-2-yl]-amine.

m/z 348 (M+1)

### Example 14 (4-Fluoro-3-methyl-phenyl)-[4-(6-methyl-3,4-dihydro-2H-quinolin-1-yl)-pyrimidin-2-yl]-amine.

m/z 349 (M+1)

### Example 15 (5-Cyclopropyl-2H-pyrazol-3-yl)-[4-(6-methyl-3,4-dihydro-2H-quinolin-1-yl)-pyrimidin-2-yl]-amine.

m/z 347 (M+1)

### Example 16 4-Benzyl-N%38-[4-(6-methyl-3,4-dihydro-2H-quinolin-1-yl)-pyrimidin-2-yl]-1H-pyrazote-3,5-diamine.

### Example 17 [4-(6-Methyl-3,4-dihydro-2H-quinolin-1-yl)-pyrimidin-2-yl]-(4-methyl-thiazol-2-yl)-amine.

m/z 338 (M+1)

### Example 18 [4-(6-Methyl-3,4-dihydro-2H-quinolin-1-yl)-pyrimidin-2-yl]-(5-methyl-1 H-pyrazol-3-yl)-amine.

m/z 321 (M+1)

The capability of the compounds of formula (I) to downregulate immune system function is demonstrated by the following further examples.

### Example 19 Short term whole cell assay for compounds than inhibit lck, ZAP-70 and itk enzymes.

The present assay measures interleukin-2 (IL-2) secreted from stimulated T-cells following binding to the cells (at the TcR) by known agonists, anti-CD3 and anti-CD28 antibodies. PTK-inhibitory compounds prevent downstream signalling and activation of the target cells by inhibiting phosphorylation of T-cell polypeptides that is necessary for the downstream signalling events (following binding to TcR) that otherwise result from antigen binding.

In the assay, Jurkat cells are incubated with candidate drug for one hour, and then stimulated with anti-CD3 and anti-CD28 antibodies provided on recoverable magnetic beads. After 18 hours of stimulation, cell supernatants are assayed for interleukin 2 by immunoassay. The following reagents are used in the assay:
(a) Dynabeads® M-450 coated with sheep-antimouse IgG (Dynal Co., product No. 110.02);
(b) anti-CD3 monoclonal antibody such as "OKT3", that is capable of signaling through the T-cell receptor complex when crosslinked;
(c) anti-CD28 monoclonal antibody, that is capable of signaling through the T-cell receptor complex when crosslinked;
(d) RPMI medium (Gibco);
(e) supplemented RPMI, to which 10% fetal calf serum, non essential amino acids (Gibco # 00467, final conc. is 1/100 that of stock), sufficient penicillin/streptomycin and optionally, % (w/w) of L-glutamine have been added;
(f) human IL-2 assay kit, (R&D Systems, catalog No. D2050);
(g) dimethylsulfoxide (DMSO), Sigma Chemical Co., catalog No. D2650;
(h) 96 well flat bottom plates (Costar, catalog No. 3596); and
(i) 96-well polypropylene plates (U-bottom, Costar Catalog No. 3365).

The Dynabeads® are prepared for assay by adding 6 micrograms of the anti-CD28 antibody and 120micrograms of the anti-CD3 antibody to 4×10⁸ beads, in a I ml volumne of Supplemented RPMI solution, followed by incubation for 1-3 hours at room temperature with gentle rocking. The fully complexed beads are then washed 3 times with 1 ml of RPMI medium, and then diluted to a bead density of 2.5 ×10⁷/ml in supplemented RPMI. The beads may be stored at 4°C. It will be appreciated that the human CD3 and CD28 glycoprotein surface antigens have may epitopes against which monoclonal antibodies can be generated, and which possess sufficient affinity to permit proper running of the assay. Generally, it is preferred that the antibodies have a K_{A} of about 10⁻⁸ or lower. Additional anti-human CD3 and anti-CD28 antibodies are known in the art, and/or are available for purchase.

Prior to assay, drug dilution plates must also be prepared. Test compounds are serially diluted (in triplicate) from 960 micromolar to 960 nanomolar on 96-well polypropylene plates using ½ log diiutions. The diluting solutions contain DMSO at concentrations appropriate to ensure that test compounds are maintained at 9.6% DMSO (v/v) during the dilutions. The assay itself is inhibited by DMSO and it is essential that concentration of DMSO be kept constant.

The test protocol is then as follows. In a typical assay, 5 microliters of test compound (the concentration range in the final dilution plates is between 960 micromolar and 960 nanomolar) is transfered from the final dilution plate to a Jurkat cell test plate (96-well flat bottom plate) which is brought up to 150 microliters final volume with Supplemented RPMI. At the resultant dilution of 1 to 30, the final DMSO concentration is 0.32% (v/v). 1.25x10⁵ Jurkat cells are then added to each well (via 125 microliters of Supplemented RPMI medium containing the cells at a density therein of 1x10⁶/ml). The cells are incubated with the test inhibitor compounds for 1 hour at 37°C.

Following this incubation, a 20 microliter quantity of the fully complexed bead suspension (at a bead density of 2.5 x10⁷/ml in supplemented RPMI) is added to each test well (as a result, 5x10⁵ beads/well are used), and the incubation is continued for 18 hours at 37°C. The supernatants from each well are transferred to 96-well V-bottom plates in order to pellet the cells and beads. The supernatants are then assayed for interleukin-2 with the human IL-2 kit (R&D, #D2050) according to instructions contained therein.

The assay does not specifically discriminate between inhibition of T-cell activation caused by inhibition of *lck,* ZAP-70 and *itk* PTK enzymes, or any combination thereof, but serves as a useful screen of promising compounds. Data are analyzed by polynomial regression and analzyed using a MACRO program. An IC₅₀ value of less than about 5 µM is preferred.

### Example 20 Screen for immunosuppressive compounds that inhibit the kinase activity of Ick enzyme

In the following assay, the potency of a test compound is determined as an IC₅₀ value, that is, the concentration of compound needed, under assay conditions, to inhibit 50% of *lck* phosphorylation activity. In the present assay, the *lck* substrate is "PGT", poly(glu-tyr) as sodium salt. The following reagents are used in the assay:
(a) DMSO (Sigma, catalog No. D2650);
(b) Dulbecco's medium diluted 1:1 with PBS (Sigma, catalog No. 14190―136).
(c) Tween-20 detergent (Sigma, catalog No. P1379);
(d) bovine serum albumin (Sigma, catalog No. A-7030;
(e) ATP (Sigma, catalog No. A5394);
(f) PGT (Sigma, catalog No. P-0275);
(g) Nunc Maxisorp plates (Van Waters &Rogers, catalog No. 62409-004);
(h) *lck*-GST enzyme (a fusion protein of *lck*/glutathione -S-transferase, expressed from a *Baculovirus* vector system, and purified on a glutathione affinity column);
(i) plate coating buffer (100 µg/ml PGT in PBS);
(j) blocking buffer (3% bovine serum albumin in PBS);
(k) phosphorylation buffer (50mM Hepes, pH 7.4, 125 mM NaCl, 24 mM MgCl₂);
(l) assay buffer (0.3 µM ATP in buffer (k));
(m) wash buffer (0.05% Tween-20 in PBS):
(n) for the detection antibody, the anti-phosphotyrosine antibody, PY-20, provided as a horseradish peroxidase ("HRP") conjugate (ICN catalog No. 69-151-1).
(o) TMB Microwell Peroxidase substrate (Kirkegaard and Perry, catalog No. 50-76-05);
(p) stop solution (0.09M H₂SO₄); and
(q) 96-well polypropylene plates (Costar, U-bottom, catalog No. 3365)

### Serial Dilution of Test Compounds on Plates

The test compounds are solubilized in DMSO (100%) and brought to 10mM as stock solutions. In this representative design, each 96-well polpypropylene drug dilution plate contains 3 compounds which are serially diluted 8 times, with a dilution factor of four for each dilution. The dilutions are performed in 50% DMSO, and set up such that each serial dilution is done in triplicate.

Prior to dilution 1, the test compound is present at 250µM (prepared by adding 5 µl of 10mM compound to 195µl of 50% DMSO). From this point, consecutive four-fold dilutions are made. For example, dilution 2 is made by mixing 25µl from dilution 1 with 75µl of 50% DMSO, and dilution 3 is made by mixing 25µl from dilution 2 with 75µl DMSO, and the like. Thus, consecutive serial four-fold dilutions will be made at 250µM, 62.5µM. 15.6µM, 3.9µM, 0.98µM, 0.24µM, 0.06µM. and 0.015µM. Accordingly, for test compound 1, the consecutive serial dilutions proceed from wells A(1-3), to A(4-6), to A(7-9), to A(10-12), to B(1-3), to B(4-6), to B(7-9), to B(10-12). For test compound 2, consecutive serial dilutions proceed from wells C(1-3), to C(4-6), to C(7-9), to C(10-12), to D(4-6), to D(10-12), to E(1-3), to E(4-6). For test compound 3, consecutive serial dilutions proceed from wells E(7-9), to E(10-12), to F(1-3), to F(4-6), to F(7-9), to F(10-12), to G(1-3), to G(4-6). Additionally, wells D(1-3) and D(7-9) contain 50% DMSO only (no compound) and are used as positive and negative controls. All other wells on the plate, G(7-12) and H(1-12) are left unused. Then, an additional 25-fold dilution is accomplished when resultant test compound samples (5µl) are transfered from the drug dilution plate to the assay plate wells (see below), each containing 120µl of assay components. Thus, the concentrations of test compounds in the present assay are 10µM, 2.5µM, 0.625µM, 0.156µM, 0.039µM, 0.0098µM, 0.0024µM, and 0.0006µM. Following the above preparations, the assay itself is performed as follows.

The Maxisorp assay plates are coated with 100µl of plate coating buffer, covered to prevent evaporation, and incubated overnight at 37°C. It should be noted that the concentration of PGT used is saturating. Following the overnight incubation, the assay plates are rinsed 3 times with wash buffer (350µl/rinse).

From the test compound plate, 5µl samples of test compound solution are added to the appropriate wells. Then 100µl of assay buffer is added to each well (assay buffer is prepared by adding ATP to the phosphorylation buffer just prior to assay). Finally, an appropriate amount of LCK, determined by titration, is added to each well in a volume of 20µl (generally, the LCK level should be near the top of the linear response range, i.e., about 80% thereof). The loaded assay plates are then shaken gently (covering is not necessary) at room temperature for 30 minutes, after which the plates are again washed three times with wash buffer.

To each plate well, 150µl of blocking buffer is then added, and blocking is performed for 30 minutes at 37°C, during which time the plates are shaken and covered to prevent evaporation. The plates are again washed 3 times with wash buffer.

The detection antibody stock solution is then diluted 1:2000 in blocking buffer, and a 50µl quantity thereof is added to each well, after which the plates are again shaken (at room temperature for 25 min), but with no covering needed. The procedure of washing the wells with wash buffer, three times, is repeated.

50µl of TMB Microwell Peroxidase Substrate is then added to each well, and blue color is allowed to develop (about 1-5 minutes) until the OD value for the positive control (450 nm) is about 1.0. At this point, 50µl of stop solution is added to each well and the plate is read on a plate reader (Softmax Pro) at 450nm.

IC₅₀ values are determined by polynomial regression and analzyed using a MACRO program. An IC₅₀ value of less than about 3 µM is preferred.

## Claims

1. A compound according to the formula 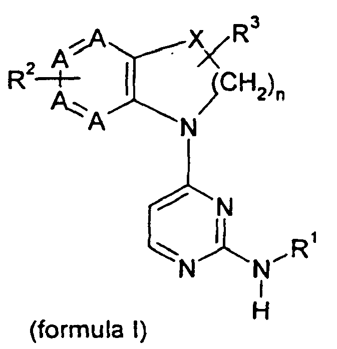 or a pharmaceutically acceptable salt, solvate, or hydrate thereof; wherein
each occurrence of A is independently selected from CH or N;
X is selected from the group consisting of -CH₂-, -O-, -NH-, (C₁-C₆)alkylamino-, (C₁-C₆)alkylaminocarbonylamino-, (C₁-C₆)alkylcarbonylamino-, (C₁-C₆)alkylsulfonylamino-, phenylsulfonylamino-, carbonyl, -NH-C(O)-, -N(C₁-C₆)alkyl-C(O)-, -S(O)y- where y is 0, 1 or 2, and ;
n in -(CH₂)ₙ- is 1, 2 or 3;
R¹ is selected from the groups consisting of (C₆-C₁₀)aryl-, (C₁-C₉)heteroaryl-, (C₆-C₁₀)aryl(C₆-C₁₀)aryl-, (C₆-C₁₀)aryl(C₁-C₉) heteroaryl-, (C₁-C₉)heteroaryl(C₁-C₉)heteroaryl-, (C₁-C₉)heteroaryl(C₆-C₁₀)aryl-, (C₆-C₁₀)arylsulfinyl-, (C₆-C₁₀)aryl(C₆-C₁₀)arylsulfinyl-, (C₁-C₉)heteroaryl(C₆-C₁₀)arylsulfinyl-, (C₆-C₁₀)arylsulfonyl-, (C₆-C₁₀)aryl(C₆-C₁₀)arylsulfonyl-, (C₁-C₉)heteroaryl(C₆-C₁₀)arylsulfonyl-, (C₁-C₉)heteroarylsulfinyl-, (C₁-C₉)heteroaryl(C₁-C₉)heteroarylsulfinyl-, (C₆-C₁₀)aryl(C₁-C₉)heteroarylsulfinyl-, (C₁-C₉)heteroarylsulfonyl-, (C₁-C₉)heteroaryl(C₁-C₉)heteroarylsulfonyl-, (C₆-C₁₀)aryl(C₁-C₉)heteroarylsulfonyl-, (R⁴)sulfinyl-, (R⁴)sulfonyl-, (C₆-C₁₀)aryl(R⁴)sulfinyl-, (C₆-C₁₀)aryl(C₆-C₁₀)aryl(R⁴)sulfinyl-, (C₁-C₉)heteroaryl(C₆-C₁₀)aryl(R⁴)sulfinyl-, (C₆-C₁₀)aryl(R⁴)sulfonyl-, (C₆-C₁₀)aryl(C₆-C₁₀)aryl(R⁴)sulfonyl-, (C₁-C₉)heteroaryl(C₆-C₁₀)aryl(R⁴)sulfonyl-, (C₁-C₉)heteroaryl(R⁴)sulfinyl-. (C₆-C₁₀)aryl(C₁-C₉)heteroaryl(R⁴)sulfinyl-, (C₁-C₉)heteroaryl(C₁-C₉)heteroaryl(R⁴)sulfinyl-, (C,-C₉)heteroaryl(R⁴)sulfonyl-, (C₆-C₉)aryl(C₁-C₉)heteroaryl(R⁴)sulfonyl-, (C₁-C₉)heteroaryl(C₁-C₉)heteroaryl(R⁴)sulfonyl-, (C₆-C₁₀)arylaminocarbonyl-, (C₆-C₁₀)aryl(C₆-C₁₀)arylaminocarbonyl-, (C₁-C₉)heteroaryl(C₆-C₁₀)arylaminocarbonyl-, (C₁-C₉)heteroarylaminocarbonyl-, (C₆-C₁₀)aryl(C₁-C₉)heteroarylaminocarbonyl-, (C₁-C₉)heteroaryl(C₁-C₉)heteroarylaminocarbonyl-, (C₆-C₁₀)arylcarbonyl-, (C₆-C₁₀)aryl (C₆-C₁₀)arylcarbonyl-, (C₁-C₉)heteroaryl(C₆-C₁₀)arylcarbonyl-, (C₁-C₉)heteroarylcarbonyl-, (C₆-C₁₀)aryl(C₁-C₉)heteroarylcarbonyl-, (C₁-C₉)heteroaryl(C₁-C₉)heteroarylcarbonyl-, (C₆-C₁₀)aryloxycarbonyl-, (C₆-C₁₀)aryl(C₆-C₁₀)aryloxycarbonyl-, (C₁-C₉)heteroaryl(C₆-C₁₀)aryloxycarbonyl-, (C₁-C₉)heteroaryloxycarbonyl-, (C₆-C₁₀)aryl(C₁-C₉)heteroaryloxycarbonyl-, (C₁-C₉)heteroaryl(C₁-C₉)heteroaryloxycarbonyl-, (R⁴)carbonyl-, (R⁴)oxycarbonyl-, (R⁴)aminocarbonyl-, (C₆-C₁₀)aryl(R⁴)carbonyl-, (C₆-C₁₀)aryl(R⁴)oxycarbonyl-, (C₆-C₁₀)aryl(R⁴)aminocarbonyl-, (C₁-C₉)heteroaryl(R⁴)carbonyl-, (C₁-C₉)heteroaryl(R⁴)oxycarbonyl-, and (C₁-C₉)heteroaryl(R⁴) aminocarbonyl-;
wherein R⁴ is selected from the groups consisting of
(a) (C₁-C₆)alkyl-, (C₂-C₆)alkenyl-, or (C₂-C₆)alkynyl-, wherein the alkyl-, alkenyl- and alkynyl- groups are optionally substituted by hydroxy, halo, amino, trifluoromethyl, hydroxy(C₂-C₆)alkyl-, (C₁-C₆)alkoxy-, (C₁-C₆)acyloxy-, (C₁-C₆)alkylamino-, ((C₁-C₆)alkyl)₂amino-, (C,-C₆)acylamino-, cyano, nitro, (C₁-C₆)alkyl-, (C₂-C₆)alkenyl-, (C₂-C₆)alkynyl-, (C₁-C₆)acylamino-, cyano(C₁-C₆)alkyl-, trifluoromethyl(C₁-C₆)alkyl-, (C₁-C₃)alkyl(difluoromethylene)(C₁-C₃)alkyl-, or nitro(C₁-C₆)alkyl-;
(b) (C₃-C₁₀)cycloalkyl-, wherein the cycloalkyl- group is optionally substituted by hydroxy, halo, amino, trifluoromethyl, hydroxy(C₂-C₆)alkyl-, (C₁-C₆)alkoxy-, (C₁-C₆)acyloxy-, (C₁-C₆)alkylamino-, ((C₁-C₆)alkyl)₂amino-, (C₁-C₆)acylamino-, cyano, nitro, (C₁-C₆)alkyl-, (C₂-C₆)alkenyl-, (C₂-C₆)alkynyl-, (C₁-C₆)acylamino-, cyano(C₁-C₆)alkyl-, trifluoromethyl(C₁-C₆)alkyl-, (C₁-C₃)alkyl(difluoromethylene)(C₁-C₃)alkyl-, or nitro(C₁-C₆)alkyl-; or
(c) (C₃-C₁₀)heterocycloalkyl-, wherein the heterocycloalkyl- group is optionally substituted by hydroxy, halo, amino, trifluoromethyl, hydroxy(C₂-C₆)alkyl-, (C₁-C₆)alkoxy-, (C₁-C₆)acyloxy-, (C₁-C₆)alkylamino-, ((C₁-C₆)alkyl)₂amino-, (C₁-C₆)acylamino-, cyano, nitro, (C,-C₆)alkyl-, (C₂-C₆)alkenyl-, (C₂-C₆)alkynyl-, (C₁-C₆)acylamino-, cyano(C₁-C₆)alkyl-, trifluoromethyl(C₁-C₆)alkyl-, (C₁-C₃)alkyl(difluoromethylene)(C₁-C₃)alkyl-, or nitro(C₁-C₆)alkyl-; and
wherein any of said of (C₆-C₁₀)aryl- or (C₁-C₉)heteroaryl- groups of R¹ may be optionally substituted by one to five groups selected from
(a) deuterium, hydroxy, halo, amino, trifluoromethyl, carboxy, (C₁-C₆)alkoxy-, (C,-C₆)acyloxy-, (C₁-C₆)alkylamino-, ((C₁-C₆)alkyl)₂amino-, (C₁-C₆)acylamino-, cyano, nitro, (C₁-C₆)alkyl-, (C₂-C₆)alkenyl-, (C₂-C₆)alkynyl-, (C₁-C₆)acylamino-, cyano(C₁-C₆)alkyl-, trifluoromethyl(C₁-C₆)alkyl-, or nitro(C₁-C₆)alkyl-, (C₁-C₃)alkyl(difluoromethylene)(C₁-C₃)alkyl-, (C₁-C₆)acylamino(C₁-C₆)alkyl-, (C₁-C₆)alkoxy(C₁-C₆)acylamino-, amino(C₁-C₆)acyl-, amino(C,-C₆)acyl(C₁-C₆)alkyl-, (C₁-C₆)alkylamino(C₁-C₆)acyl-, ((C₁-C₆)alkyl)₂amino(C₁-C₆)acyl-, (C₃-C₁₀)cycloalkyl(C₁-C₆)alkyl-, (C₁-C₆)acyloxy(C₁-C₆)alkyl-, (C₂-C₆)alkoxy(C₁-C₆)alkyl-, piperazinyl(C₁-C₆)alkyl-, (C₁-C₆)acylamino(C₁-C₆)alkyl-, (C₆-C₁₀)aryl(C₁-C₆)alkoxy(C₁-C₆)alkyl-, (C₅-C₉)heteroaryl(C₁-C₆)alkoxy(C₁-C₆)alkyl-, (C₁-C₆)alkylthio(C₁-C₆)alkyl-, (C₆-C₁₀)arylthio(C₁-C₆)alkyl-, (C₁-C₆)alkylsulfinyl(C₁-C₆)alkyl-, (C₆-C₁₀)arylsulfinyl(C₁-C₆)alkyl-, (C₁-C₆)alkylsulfonyl(C₁-C₆)alkyl-, (C₆-C₁₀)arylsulfonyl(C₁-C₆)alkyl-, amino(C₁-C₆)alkyl-, (C,-C₆)alkylamino(C₁-C₆)alkyl-, (C₁-C₆)alkyl(difluoromethylene)-, (C₁-C₃)alkyl(difluoromethylene)(C₁-C₃)alkyl-, (C₁-C₆)alkoxy(C₁-C₆)acyl-, (C₁-C₆)alkylamino(C₁-C₆)acyl-, ((C₁-C₆)alkyl)₂amino(C₁-C₆)acyl-, (C₆-C₁₀)aryl-, (C₁-C₉)heteroaryl-, (C₆-C₁₀)aryl(C₁-C₆)alkyl-, (C₁-C₉)heteroaryl(C₁-C₆)alkyl-, (C₆-C₁₀)aryl(C₆-C₁₀)aryl-, (C₆-C₁₀)aryl(C₆-C₁₀)aryl(C₁-C₆)alkyl-, (C₃-C₁₀)cycloalkyl-, (C₃-C₆)cycloalkyl(C₁-C₆)alkyl-, (C₃-C₁₀)heterocycloalkyl-, (C₃-C₁₀)heterocycloalkyl(C₁-C₆)alkyl-, hydroxy(C₂-C₆)alkyl-, (C₁-C₆)acyloxy(C₂-C₆)alkyl-, (C,-C₆)alkoxy(C₂-C₆)alkyl-, piperazinyl(C₁-C₆)alkyl-, (C₁-C₆)acylamino(C₁-C₆)alkyl-, (C₆-C₁₀)aryl(C₁-C₆)alkoxy(C₁-C₆)alkyl-, (C₁-C₉)heteroaryl(C₁-C₆)alkoxy(C₁-C₆)alkyl-, (C₁-C₆)alkylthio(C₁-C₆)alkyl-, (C₆-C₁₀)arylthio(C₁-C₆)alkyl-, (C₁-C₆)alkylsulfinyl(C₁-C₆)alkyl-, (C₆-C₁₀)arylsulfinyl(C₁-C₆)alkyl-, (C₁-C₆)alkylsulfonyl(C₁-C₆)alkyl-, (C₆-C₁₀)arylsulfonyl(C₁-C₆)alkyl-, amino(C₁-C₆)alkyl-, (C₁-C₆)alkylamino(C₁-C₆)alkyl-, ((C₁-C₆)alkyl)₂amino(C₁-C₆)alkyl-;
(b) R⁵OCO(C₁-C₆)alkyl-, wherein R⁵ is selected from the group consisting of hydrogen, (C₁-C₆)alkyl-, (C₆-C₁₀)aryl(C₁-C₆)alkyl-, (C₁-C₉)heteroaryl(C₁-C₆)alkyl-; or
(c) R⁶(C₀-C₆)alkyl-, wherein R⁶ is selected from the group consisting of piperazino, (C,-C₆)acylpiperazino-, (C₆-C₁₀)arylpiperazino-, (C₅-C₉)heteroarylpiperazino-, (C₁-C₆)alkylpiperazino-, (C₆-C₁₀)aryl(C₁-C₆)alkylpiperazino-, (C₁-C₉)heteroaryl(C₁-C₆)alkylpiperazino-, morpholino-, (C₁-C₆)acylmorpholino-, (C₆-C₁₀)arylmorpholino-, (C₁-C₉)heteroarylmorpholino-, (C₁-C₆)alkylmorpholino-, (C₆-C₁₀)aryl(C₁-C₆)alkylmorpholino-, (C,-C₉)heteroaryl(C₁-C₆)alkylmorpholino-, thiomorpholino-, (C₁-C₆)acylthiomorpholino-, (C₆-C₁₀)arylthiomorpholino-, (C₁-C₉)heteroarylthiomorpholino-, (C₁-C₆)alkylthiomorpholino-, (C₆-C₁₀)aryl(C₁-C₆)alkylthiomorpholino-, (C₁-C₉)heteroaryl(C₁-C₆)alkylthiomorpholino-, piperidino-, (C₁-C₆)acylpiperidino-, (C₆-C₁₀)arylpiperidino-, (C₁-C₉)heteroarylpiperidino-, (C₁-C₆)alkyl piperidino-, (C₆-C₁₀)aryl(C₁-C₆)piperidino-, (C₁-C₉)heteroaryl(C₁-C₆)alkylpiperidino-, pyrrolidino-, (C₁-C₆)acylpyrrolidino-, (C₆-C₁₀)aryl- pyrrolidino-, (C₁-C₉)heteroaryl- pyrrolidino-, (C₁-C₆)alkylpyrrolidino-, (C₆-C₁₀)aryl(C₁-C₆)pyrrolidino-, (C₁-C₉)heteroaryl(C₁-C₆)alkylpyrrolidino-, (C₁-C₆)alkoxy(C₁-C₆)acyl-, (C₁-C₆)alkylamino(C₆-C₁₀)aryl-, and ((C₁-C₆)alkyl₂amino(C₁-C₆)acyl-;
R² represents one to four optional substituents, each being independently selected from the members of groups (a) to (f)
(a) deuterium, halo, hydroxy, carboxy, amino, trifluoromethyl, (C₁-C₆) alkyl-, (C₂-C₆)alkenyl-, (C₂-C₆)alkynyl-, (C₁-C₆)alkylamino-, ((C₁-C₆)(alkyl)₂amino-, cyanoalkyl-, (C₃-C₁₀)cycloalkyl-, (C₃-C₁₀)heterocycloalkyl-, (C₃-C₁₀)cycloalkoxy-, (C₁-C₆)alkylthio-, (C,-C₆)alkylsulfinyl-, (C₁-C₆)alkylsulfonyl-, amino-CO-NH- , (C₁-C₆)alkoxy-CO-NH-, (C₁-C₆)alkyl-CO-NH- (C₁-C₆)alkyl-CO-NH-(C₁-C₆)alkyl-, (C₁-C₆)alkyl-CO-NH-(C₁-C₆)alkoxy-, (C₁-C₆)alkoxycarbonyl(C₁-C₆)alkoxy-, (C₁-C₆)alkoxy-CO-NH-(C₁-C₆)alkoxy-, (C₁-C₆)alkylamino-CO-NH- , (C₁-C₆)alkylamino-CO-NH-(C₁-C₆)alkyl-, ((C₁-C₆)alkyl)₂amino-CO-NH-(C₁₋C₆)alkyl-, ((C₁-C₆)alkyl)₂amino-CO-NH- carboxy, carboxy(C₁-C₆)alkyl-, carboxy(C₁-C₆)alkoxy-, benzyloxycarbonyl(C₁-C₆)alkoxy-, (C₁-C₆)alkylamino-CO-, (C₁-C₆)acylamino-, (C₁-C₆)alkoxy-, (C₁-C₆)acyl-, (C₁-C₆)acyloxy-, (C₁-C₆)acyl(C₁-C₆)alkylamino-, (C₁-C₆)alkoxyacyl-, (C₁-C₆)alkylaminoacyl-, ((C₁-C₆)alkyl)₂aminoacyl-, amino(C₁-C₆)acyl-, amino(C₁-C₆)alkyl-, (C,-C₆)alkoxycarbonylamino-, (C₁-C₆)alkoxycarbonyl(C₁-C₆)alkyl-, (C₆-C₁₀)aryl(C₁-C₆)alkoxycarbonylamino-, trihalomethyl-, trihalomethyl(C₁-C₆)alkyl-, (C₁-C₆)alkyldihalomethylene-, (C₁-C₃)alkyl(dihalomethylene)(C₁-C₃)alkyl-, (C₃-C₆)cycloalkyl-, (C₃-C₆)cycloalkyl(C₁-C₆)alkyl-, hydroxy(C₁-C₆)alkyl-, (C₁-C₆)acyloxy(C₁-C₆)alkyl-, (C₁-C₆)alkoxy(C₁-C₆)alkyl-, (C₁-C₆)acylamino(C₁-C₆)alkyl-, (C₁-C₆)alkylthio(C₁-C₆)alkyl-, (C₁-C₆)alkoxycarbonyl-, (C₁-C₆)alkylsulfinyl(C₁-C₆)alkyl-, (C₁-C₆)alkylsulfonyl(C₁-C₆)alkyl-, (C₁-C₆)alkylsulfonyl-, (C₁-C₆)alkylsulfonylamino-, (C₁-C₆)alkylsulfonylamino(C₁-C₆)alkylamino(C₁-C₆)alkyl-, (C₁-C₆)alkylamino(C₁-C₆)alkyl-, ((C₁-C₆)alkyl)₂amino(C₁-C₆)alkyl-, (C₁-C₆)CO(C₁-C₆)alkyl-;
(b) (C₆-C₁₀)aryl-, (C₁-C₉)heteroaryl-, (C₆-C₁₀)aryl(C₆-C₁₀)aryl-, (C₆-C₁₀)aryl(C₁-C₉) heteroaryl-, (C₁-C₉)heteroaryl(C₁-C₉)heteroaryl-, (C₁-C₉)heteroary)(C₆-C₁₀)aryl-, (C₆-C₁₀)arylsulfinyl-, (C₆-C₁₀)aryl(C₆-C₁₀)arylsulfinyl-, (C₁-C₉)heteroaryl(C₆-C₁₀)arylsulfinyl-, (C₆-C₁₀)arylsulfonyl-, (C₆-C₁₀)aryl(C₆-C₁₀)arylsulfonyl-, (C₁-C₉)heteroaryl(C₆-C₁₀)arylsulfonyl-, (C,-C₉)heteroarylsulfinyl-, (C₁-C₉)heteroaryl(C₁-C₉)heteroarylsulfinyl, (C₆-C₁₀)aryl(C₁-C₉)heteroarylsulfinyl-, (C₁-C₉)heteroarylsulfonyl-, (C₁-C₉)heteroaryl(C₁-C₉)heteroarylsulfonyl-, (C₆-C₁₀)aryl(C₁-C₉)heteroarylsulfonyl-, (R⁴)sulfinyl-, (R⁴)sulfonyl-, (C₆-C₁₀)aryl(R⁴)sulfinyl-, (C₆-C₁₀)aryl(C₆-C₁₀)aryl(R⁴)sulfinyl-, (C₁-C₉)heteroaryl(C₆-C₁₀)aryl(R⁴)sulfinyl-, (C₆-C₁₀)aryl(R⁴)sulfonyl-, (C₆-C₁₀)aryl(C₆-C₁₀)aryl(R⁴)sulfonyl-, (C₁-C₉)heteroaryl(C₆-C₁₀)aryl(R⁴)sulfonyl-, (C₁-C₉)heteroaryl(R⁴)sulfinyl-, (C₆-C₁₀)aryl(C₁-C₉)heteroaryl(R⁴)sulfinyl-, (C₁-C₉)heteroaryl(C₁-C₉)heteroaryl(R⁴)sulfinyl-, (C₁-C₉)heteroaryl(R⁴)sulfonyl-, (C₆-C₁₀)aryl(C₅-C₉)heteroaryl(R⁴)sulfonyl-, (C₁-C₉)heteroaryl(C₁-C₉)heteroaryl(R⁴)sulfonyl-, (C₆-C₁₀)arylaminocarbonyl-, (C₆-C₁₀)aryl(C₆-C₁₀)arylaminocarbonyl-, (C₁-C₉)heteroaryl(C₆-C₁₀)arylaminocarbonyl-, (C₁-C₉)heteroarylaminocarbonyl-, (C₆-C₁₀)aryl(C₁-C₉)heteroarylaminocarbonyl-, (C₁-C₉)heteroaryl(C₁-C₉)heteroarylaminocarbonyl-, (C₆-C₁₀)arylcarbonyl-, (C₆-C₁₀)aryl(C₆-C₁₀)arylcarbonyl-, (C₁-C₉)heteroaryl(C₆-C₁₀)arylcarbonyl-, (C₁-C₉)heteroarylcarbonyl-, (C₆-C₁₀)aryl(C₁-C₉)heteroarylcarbonyl-, (C₁-C₉)heteroaryl(C₁-C₉)heteroarylcarbonyl-, (C₆-C₁₀)aryloxycarbonyl-, (C₆-C₁₀)aryl(C₆-C₁₀)aryloxycarbonyl-, (C₁-C₉)heteroaryl(C₆-C₁₀)aryloxycarbonyl-, (C₁-C₉)heteroaryloxycarbonyl-, (C₆-C₁₀)aryl(C₁-C₉)heteroaryloxycarbonyl-, (C₁-C₉)heteroaryl(C₁-C₉)heteroaryloxycarbonyl-, (R⁴)carbonyl-, (R⁴)oxycarbonyl-, (R⁴)aminocarbonyl-, (C₆-C₁₀)aryl(R⁴)carbonyl-, (C₆-C₁₀)aryl(R⁴)oxycarbonyl-, (C₆-C₁₀)aryl(R⁴)aminocarbonyl-, (C₁-C₉)heteroaryl(R⁴)carbonyl-, (C₅-C₉)heteroaryl(R⁴)oxycarbonyl-, (C₁-C₉)heteroaryl(R⁴) aminocarbonyl-, wherein R⁴ is defined as above, and wherein any of said of (C₆-C₁₀)aryl- or (C₁-C₉)heteroaryl- R² groups may be optionally substituted by one to five groups independently selected from:
(i) hydroxy, halo, amino, trifluoromethyl, carboxy, (C₁-C₆)alkoxy-, (C₁-C₆)acyloxy-, (C₁-C₆)alkylamino-, ((C₁-C₆)alkyl)₂amino-, (C₁-C₆)acylamino-, cyano, nitro, (C,-C₆)alkyl-, (C₂-C₆)alkenyl-, (C₂-C₆)alkynyl-, (C₁-C₆)acylamino-, cyano(C₁-C₆)alkyl-, trifluoromethyl(C₁-C₆)alkyl-, or nitro(C₁-C₆)alkyl-, (C₁-C₃)alkyl(difluoromethylene)(C₁-C₃)alkyl-, (C₁-C₆)acylamino(C₁-C₆)alkyl-, (C₁-C₆)alkoxy(C₁-C₆)acylamino-, amino(C₁-C₆)acyl-, amino(C,-C₆)acyl(C₁-C₆)alkyl-, (C₁-C₆)alkylamino(C₁-C₆)acyl-, ((C₁-C₆)alkyl)₂amino(C₁-C₆)acyl-, (C₃-C₁₀)cycloalkyl(C₁-C₆)alkyl-, (C₁-C₆)acyloxy(C₁-C₆)alkyl-, (C₂-C₆)alkoxy(C₁-C₆)alkyl-, piperazinyl(C₁-C₆)alkyl-, (C₁-C₆)acylamino(C₁-C₆)alkyl-, (C₆-C₁₀)aryl(C₁-C₆)alkoxy(C₁-C₆)alkyl-, (C₁-C₉)heteroaryl(C₁-C₆)alkoxy(C₁-C₆)alkyl-, (C₁-C₆)alkylthio(C₁-C₆)alkyl-, (C₆-C₁₀)arylthio(C₁-C₆)alkyl-, (C₁-C₆)alkylsulfinyl(C₁-C₆)alkyl-, (C₆-C₁₀)arylsulfinyl(C₁-C₆)alkyl-, (C,-C₆)alkylsulfonyl(C₁-C₆)alkyl-, (C₆-C₁₀)arylsulfonyl(C₁-C₆)alkyl-, amino(C₁-C₆)alkyl-, (C,-C₆)alkylamino(C₁-C₆)alkyl-, (C₁-C₆)alkyl(difluoromethylene)-, (C₁-C₃)alkyl(difluoromethylene)(C₁-C₃)alkyl-, (C₁-C₆)alkoxy(C₁-C₆)acyl-, (C₁-C₆)alkylamino(C₁-C₆)acyl-, ((C₁-C₆)alkyl)₂amino(C₁-C₆)acyl-, (C₆-C₁₀)aryl-, (C₁-C₉)heteroaryl-, (C₆-C₁₀)aryl(C₁-C₆)alkyl-, (C₁-C₉)heteroaryl(C₁-C₆)alkyl-, (C₆-C₁₀)aryl(C₆-C₁₀)aryl-, (C₆-C₁₀)aryl(C₆-C₁₀)aryl(C₁-C₆)alkyl-, (C₃-C₁₀)cycloalkyl-, (C₃-C₆)cycloalkyl(C₁-C₆)alkyl-, (C₃-C₁₀)heterocycloalkyl-, (C₃-C₁₀)heterocycloalkyl(C₁-C₆)alkyl-, hydroxy(C₂-C₆)alkyl-, (C₁-C₆)acyloxy(C₂-C₆)alkyl-, (C,-C₆)alkoxy(C₂-C₆)alkyl-, piperazinyl(C₁-C₆)alkyl-, (C₁-C₆)acylamino(C₁-C₆)alkyl-, (C₆-C₁₀)aryl(C₁-C₆)alkoxy(C₁-C₆)alkyl-, (C₁-C₉)heteroaryl(C₁-C₆)alkoxy(C₁-C₆)alkyl-, (C₁-C₆)alkylthio(C₁-C₆)alkyl-, (C₆-C₁₀)arylthio(C₁-C₆)alkyl-, (C₁-C₆)alkylsulfinyl(C₁-C₆)alkyl-, (C₆-C₁₀)arylsulfinyl(C₁-C₆)alkyl-, (C₁-C₆)alkylsulfonyl(C₁-C₆)alkyl-, (C₆-C₁₀)arylsulfonyl(C₁-C₆)alkyl-, amino(C₁-C₆)alkyl-, (C₁-C₆)alkylamino(C₁-C₆)alkyl-, ((C₁-C₆)alkyl)₂amino(C₁-C₆)alkyl-;
(ii) R⁵OCO(C₁-C₆)alkyl- wherein R⁵ is selected from the group consisting of hydrogen, (C₁-C₆)alkyl, (C₆-C₁₀)aryl(C₁-C₆)alkyl-, (C₁-C₉)heteroaryl(C₁-C₆)alkyl-;
(iii) R⁶(C₂-C₆)alkyl- wherein R⁶ is selected from the group consisting of piperazino, (C₁-C₆)acylpiperazino-, (C₆-C₁₀)arylpiperazino-, (C₅-C₉)heteroarylpiperazino-, (C₁-C₆)alkylpiperazino-, (C₆-C₁₀)aryl(C₁-C₆)alkylpiperazino-. (C₁-C₉)heteroaryl(C₁-C₆)alkylpiperazino-, morpholino-, (C₁-C₆)acylmorpholino-, (C₆-C₁₀)arylmorpholino-, (C₁-C₉)heteroarylmorpholino-, (C₁-C₆)alkylmorpholino-, (C₆-C₁₀)aryl(C₁-C₆)alkylmorpholino-, (C,-C₉)heteroaryl(C₁-C₆)alkylmorpholino-, thiomorpholino-, (C₁-C₆)acylthiomorpholino-, (C₆-C₁₀)arylthiomorpholino-, (C₁-C₉)heteroarylthiomorpholino-, (C₁-C₆)alkylthiomorpholino-, (C₆-C₁₀)aryl(C₁-C₆)alkylthiomorpholino-, (C₁-C₉)heteroaryl(C₁-C₆)alkytthiomorpholino-, piperidino-, (C₁-C₆)acylpiperdino-, (C₆-C₁₀)arylpiperidino-, (C₁-C₉)heteroarylpiperidino-, (C₁-C₆)alkyl piperidino-, (C₆-C₁₀)aryl(C₁-C₆)piperidino-, (C₁-C₉)heteroaryl(C₁-C₆)alkylpiperidino-, pyrrolidino-, (C₁-C₆)acylpyrrolidino-, (C₆-C₁₀)arylpyrrolidino-, (C₁-C₉)heteroarylpyrrolidino-, (C₁-C₆)alkylpyrrolidino-, (C₆-C₁₀)aryl(C₁-C₆)alkylpyrrolidino-, (C₁-C₉)heteroaryl(C₁-C₆)alkylpyrrolidino-, (C₁-C₆)alkoxy(C₁-C₆)acyl-, (C₁-C₆)alkylamino(C₆-C₁₀)aryl-, and ((C₁-C₆)alkyl₂amino(C₁-C₆)acyl-;
(c) R⁷, or R⁷Y-, where R⁷ is selected from the group consisting of piperazino-, (C₆-C₁₀)arylpiperazino-, (C₁-C₉)heteroarylpiperazino-, (C₁-C₆)alkylpiperazino-, (C₆-C₁₀)aryl(C₁-C₆)alkylpiperazino-, (C₁-C₉)heteroaryl(C₁-C₆)alkylpiperazino-, morpholino-, (C₆-C₁₀)arylmorpholino-, (C₁-C₉)heteroarylmorpholino-, (C₁-C₆)alkylmorpholino-, (C₆-C₁₀)aryl(C₁-C₆)alkylmorpholino-, (C₁-C₉)heteroaryl(C₁-C₆)alkylmorpholino-, thiomorpholino-, (C₆-C₁₀)arylthiomorpholino-, (C₁-C₉)heteroarylthiomorpholino-, (C₁-C₆)alkylthiomorpholino-, (C₆-C₁₀)aryl(C₁-C₆)alkylthiomorpholino-, (C₁-C₉)heteroaryl(C₁-C₆)alkylthiomorpholino-, piperidino-, (C₆-C₁₀)arylthiopiperidino-, (C₁-C₉)heteroarylthiopiperidino-, (C₁-C₆)alkylthiopiperidino-, (C₆-C₁₀)aryl(C₁-C₆)alkylthiopiperidino-, (C₁-C₉)heteroaryl(C₁-C₆)alkylthiopiperidino-, pyrolidino-, (C₆-C₁₀)arylthiopyrolidino-, (C₁-C₉)heteroarylthiopyrolidino-, (C₁-C₆)alkylthiopyrolidino-, (C₆-C₁₀)aryl(C₁-C₆)alkylthiopyrolidino-, (C₁-C₉)heteroaryl(C₁-C₆)alkylthiopyrolidino-, and Y, if present, is selected from the group consisting of (C₁-C₆)alkyl-, (C₂-C₆)alkenyl-, (C₂-C₆)alkynyl-amino, oxygen, thio, sulfinyl, sulfonyl, halo(C₁-C₆)alkyl-, and hydroxy(C₂-C₆)alkyl-;
(d) ZR⁸ -, where R⁸ is selected from the group consisting of piperazino-, (C₆-C₁₀)arylpiperazino-, (C₁-C₉)heteroarylpiperazino-, (C₁-C₆)alkylpiperazino-, (C₆-C₁₀)aryl(C₁-C₆)alkylpiperazino-, (C₁-C₉)heteroaryl(C₁-C₆)alkylpiperazino-, morpholino-, (C₆-C₁₀)arylmorpholino-, (C₁-C₉)heteroarylmorpholino-, (C₁-C₆)alkylmorpholino-, (C₆-C₁₀)aryl(C₁-C₆)alkylmorpholino-, (C₁-C₉)heteroaryl(C₁-C₆)alkylmorpholino-, thiomorpholino-, (C₆-C₁₀)arylthiomorpholino-, (C₁-C₉)heteroarylthiomorpholino-, (C₁-C₆)alkylthiomorpholino-, (C₆-C₁₀)aryl(C₁-C₆)alkylthiomorpholino-, (C₁-C₉)heteroaryl(C₁-C₆)alkylthiomorpholino-, piperidino-, (C₆-C₁₀)arylthiopiperidino-, (C₁-C₉)heteroarylthiopiperidino-, (C₁-C₆)alkylthiopiperidino-, (C₆-C₁₀)aryl(C₁-C₆)alkylthiopiperidino-, (C₁-C₉)heteroaryl(C₁-C₆)alkylthiopiperidino-, pyrolidino-, (C₆-C₁₀)arylthiopyrolidino-, (C₁-C₉)heteroarylthiopyrolidino-, (C₁-C₆)alkylthiopyrolidino-, (C₆-C₁₀)aryl(C₁-C₆)alkylthiopyrolidino-, (C₁-C₉)heteroaryl(C₁-C₆)alkylthiopyrolidino-, and Z is selected from the group consisting of (C₁-C₆)alkyl-, (C₂-C₆)alkenyl-, (C₂-C₆)alkynyl-, amino, oxygen, thio, sulfinyl, sulfonyl, halo(C₁-C₆)alkyl-, and hydroxy(C₂-C₆)alkyl-;
(e) two or more of R² , when vicinal, together to form one or more further rings of 4, 5, 6 or 7 member atoms selected from the group consisting of phenyl-, naphthyl-, furyl-, thienyl-, thiazolyl-, pyrazolyl-, isothiazolyl-, oxazolyl-, isoxazolyl-, pyrrolyl-, triazolyl-, tetrazolyl-, imidazolyl-, 1,3,5-oxadiazolyl-, 1,2,4-oxadiazolyl-, 1,2,3-oxadiazolyl-, 1,3,5-thiadiazolyl,-1,2,3-thiadiazolyl-, 1,2,4-thiadiazolyl-, pyridyl-, pyrimidyl-, pyrazinyl-, pyridazinyl-, 1,2,4-triazinyl-, 1,2,3-triazinyl-, 1,3,5-triazinyl-, pyrazolo[3,4-b]pyridinyl-, cinnolinyl-, pteridinyl-, purinyl-, 6,7-dihydro-5H-[1]pyrindinyl-, benzo[b]thiophenyl-, 5, 6, 7, 8-tetrahydro-quinolin-3-yl, benzoxazolyl-, benzothiazolyl-, benzisothiazolyl-, benzisoxazolyl-, benzimidazolyl-, thianaphthenyl-, isothianaphthenyl-, benzofuranyl-, isobenzofuranyl-, isoindolyl-, indolyl-, indolizinyl-, indazolyl-, isoquinolyl-, quinolyl-, phthalazinyl-, quinoxalinyl-, quinazolinyl-, benzoxazinyl-, and wherein said ring(s) are optionally substituted by one or more (C₁-C₆)alkyl-(C₂-C₆)alkenyl-, (C₂-C₆)alkynyl-, amino-, halo-, hydroxy-, carboxy-, thiol-, nitro-, cyano-, sulfonic-, halo(C₁-C₆)alkyl-, and hydroxy(C₂-C₆)alkyl-; and
(f) two or more of R² when vicinal, together to form one or more further rings of 3, 4, 5, 6 or 7 member atoms selected from the groups consisting of:
(i) (C₃-C₁₀)cycloalkyl-, containing zero to two levels of unsaturation, selected from the group consisting of cyclopropyl-, cyclobutyl-, cyclopentyl-, cyclohexyl-, cycloheptyl-, cyclopropenyl-, cyclobutenyl-, cyclopentenyl-, cyclohexenyl-, cycloheptenyl-, 1,3-cyclobutadienyl-, 1,3-cyclopentadienyl-, 1,3-cyctohexadienyl-, 1,4-cyclohexadienyl-, 1,3-cycloheptadienyl-, 1,4-cycloheptadienyl-, bicyclo[3.2.1]octane-, bicyclo [2.2.1] heptane, the norborn-2-ene unsaturated form thereof, and the like, wherein said ring is optionally substituted by hydroxy-, halo-, amino-, trifluoromethyl-, hydroxy(C₂-C₆)alkyl-, (C₁-C₆)alkoxy-, (C₁-C₆)acyloxy-, (C₁-C₆)alkylamino-, ((C₁-C₆)alkyl)₂amino-, (C₁-C₆)acylamino-, cyano-, nitro-, carboxy-, thiol-, sulfonyl-, (C₁-C₆)alkyl-, (C₂-C₆)alkenyl-, (C₂-C₆)alkynyl-, (C₁-C₆)acylamino-, cyano(C₁-C₆)alkyl-, trifluoromethyl(C₁-C₆)alkyl-, (C₁-C₃)alkyl(difluoromethylene)(C₁-C₃)alkyl-, halo(C₁-C₆)alkyl- or nitro(C₁-C₆)alkyl-; and
(ii) (C₃-C₁₀)heterocycloalkyl- selected from the group consisting of pyrrolidinyl-, tetrahydrofuranyl-, dihydrofuranyl-, tetrahydropyranyl-, pyranyl-, thiopyrany-1, aziridinyl-, oxiranyl-, methylenedioxyl-, , isoxazolidinyl.- 1,3-oxazolidin-3-yl-, isothiazolidinyl-, 1,3-thiazolidin-3-yl-, 1,2-pyrazolidin-2-yl-, 1,3-pyrazolidin-1-yl-, piperidinyl-, thiomorpholinyl-, 1,2-tetrahydrothiazin-2-yl-, 1,3-tetrahydrothiazin-3-yl-, tetrahydrothiadiazinyl-, morpholinyl-, 1,2-tetrahydrodiazin-2-yl-, 1,3-tetrahydrodiazin-1-yl-, tetrahydroazepinyl-, piperazinyl-, chromenyl-, chromanyl-, where said ring is optionally substituted by hydroxy-, halo-, amino-, trifluoromethyl-, hydroxy(C₂-C₆)alkyl-, (C₁-C₆)alkoxy-, (C₁-C₆)acyloxy-, (C₁-C₆)alkylamino-, ((C₁-C₆)alkyl)₂amino-, (C₁-C₆)acylamino-, cyano-, nitro-, carboxy-, thiol-, sulfonyl-, (C₁-C₆)alkyl-, (C₂-C₆)alkenyl-, (C₂-C₆)alkynyl-, (C₁-C₆)acylamino-, cyano(C₁-C₆)alkyl-, trifluoromethyl(C₁-C₆)alkyl-, (C₁-C₃)alkyl(difluoromethylene)(C₁-C₃)alkyl-, halo(C₁-C₆)alkyl- or nitro(C₁-C₆)alkyl-;
wherein any (C₁-C₆)alkyl-, (C₂-C₆)alkenyl-, (C₂-C₆)alkynyl-, (C₃-C₁₀)cycloalkyl- or (C₃-C₁₀)heterocycloalkyl- groups that are, or comprise a portion of, said one to four optional R² substituents are themselves optionally substituted by deuterium-, hydroxy-, amino-, trifluoromethyl-, cyano-, nitro-, carboxy-, (C₁-C₄)alkoxy-, (C₁-C₆)acyloxy-, (C₁-C₆)alkylamino-, ((C₁-C₆)alkyl)₂amino-, (C₁-C₆)alkyl- (C₂-C₆)alkenyl-, (C₂-C₆)alkynyl-, (C₁-C₆)acylamino-, (C₃-C₁₀)cycloalkyl-, (C₃-C₁₀)heterocycloalkyl-, cyano(C₁-C₆)alkyl-, trifluoromethyl(C₁-C₆)alkyl-, nitro(C₁-C₆)alkyl-, and (C₁-C₆)acylamino; and.
R³ represents one or more optional substituents on a ring carbon atom, including at X where X is -CH₂-, selected from the groups consisting of (C₁-C₆)alkyl- , trihalo(C₁-C₆)alkyl- that is preferably trifluoromethyl-, deuterium, and fluorine.

2. A compound according to claim 1, wherein the structural component thereof that is represented by 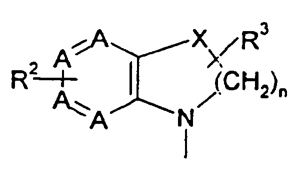 is contributed by 1,2,3,4-tetrahydroquinoline; 1,2,3,4-tetrahydroquinoxaline; 3,4-Dihydro-1H-quinoxaline-2-one; 3,4-Dihydro-2H-benzo[1,4]oxazine; 2,3-Dihydro-1H-indole; or 3,4-Dihydro-2H-benzo[1,4]thiazine.

3. A compound according to claim 2, that comprises a structure selected from the group consisting of 6-methoxy-1,2,3,4-tetrahydroquinoline; 4-methyl-1,2,3,4-tetrahydroquinoline; 7-(trifluoromethyl)-1,2,3,4-tetrahydroquinoline; 8-methyl-1,2,3,4-tetrahydroquinoline; 6-hydroxy-1,2,3,4-tetrahydroquinoline; 8-chloro-1,2,3,4-tetrahydroquinoline; 7-chloro-1,2,3,4-tetrahydroquinoline; 6-benzyloxy-7-methoxy-1,2,3,4-tetrahydroquinoline; 6,7-dimethyl-1,2,3,4-tetrahydroquinoxaline; 1,2,3,4-tetrahydroquinoxaline; 1-phenylsuffonyl-1,2,3,4-tetrahydroquinoxaline; 6-Methyl-1,2,3,4-Tetrahydroquinoline; 3,4-Dihydro-2H-benzo[1,4]oxazine; 5-Fluoro-2,3-dihydro-1H-indole; and 3,3-Dimethyl-2,3-dihydro-1 H-indole.

4. A compound according to claim 1 wherein R¹ is a (C₁-C₉)heteroaryl- group selected from the group consisting of pyridyl-, indazolyl-, indolyl-, 1,3-dihydro-benzoimidazol-2-one, thienyl-, oxazoyl-, 2H-pyrazolyl-, 1H-pyrazolyl-, isooxazoyl-, thiazolyl(fix,name), and isothiazoyl-; and is optionally substituted by one or more groups, each independently selected from hydroxy-, halo-, amino-, (C₁-C₆)alkyl-, (C₁-C₆)alkoxy-, trihalomethyl-, (C₁-C₆)alkynl-, (C,-C₆)alkylamino-, ((C₁-C₆)₂)dialkylamino-, carboxy-, (C₁-C₆)alkoxycarbonyl-, (C₁-C₆)acyloxy-, and (C₁-C₆)acylamino-.

5. A compound according to claim 1 wherein R¹ is phenyl, optionally substituted with one to five substituents, that are each independently selected from hydroxy-, halo-, amino-, (C₁-C₆)alkyl-, (C₁-C₆)alkoxy-, trihalo(C₁-C₆)alkyl- that is preferably trifluoromethyl-, (C₁-C₆)alkynl-, (C₁-C₆)alkylamino-, ((C₁-C₆)₂)dialkylamino-, carboxy, (C₁-C₆)alkoxycarbonyl-, (C₁-C₆)acyloxy-, and (C₁-C₆)acylamino-.

6. A compound according to claim 1 wherein R¹ is selected from the group consisting of 3,4,5-trimethoxyphenyl-, 2,3-dimethyl-1H-indol-5-yl; 3,4-dihydro-2H-quinolin-1-yl; and 6-morpholin-4-yl-pyridin-3-yl.

7. A compound according to claim 1 wherein one or more of substituents R² is selected from the groups consisting of
(a) (C₁-C₆)alkyl-, (C₁-C₆)alkynl-, (C₁-C₆)alkoxy-, trihalo(C₁-C₆)alkyl- that is preferably trifluoromethyl-, (C₁-C₆)alkylamino-, ((C₁-C₆)₂)dialkylamino-, amino-, cyano, and halo-; and
(b) benzyloxy-, phenylsulfonyl-, phenylaminocarbonyl-, (C₁-C₉)heteroarylsulfonyl- and (C₁-C₉)heteroarylaminocarbonyl-, optionally substituted by one or more groups selected from the group consisting of (C₁-C₆)alkyl-, (C₂-C₆)alkynyl-, trihalo(C₁-C₆)alkyl- that is preferably trifluoromethyl- , (C₁-C₆)alkoxy-, (C₁-C₆)alkylamino-, ((C₁-C₆)₂)alkylamino-, and halo.

8. A compound according to claim 1 wherein one or more of substituents R³ is selected from the groups consisting of (C₁-C₆)alkyl- , trihalo(C₁-C₆)alkyl-, deuterium, and fluorine.

9. A compound according to claim 8 wherein R³ is trifluoromethyl.

10. A compound according to claim 1, selected from the group consisting of
(a) 1-[(2-anilino)-4-pyrimidinyl]-6-methyl-1,2,3,4-tetrahydroquinoline;
(b) 1-[2-[(4-bromophenyl)amino]-4-pyrimidinyl]-6-methyl-1,2,3,4-tetrahydroquinoline;
(c) 1-[2-[(4-methoxyphenyl)amino]-4-pyrimidinyl]-6-methyl-1,2,3,4-tetrahydroquinoline;
(d) 1-[2-[(1H-indazole-5-yl)]-4-pyrimidyl]-6-methyl-1,2,3,4-tetrahydroquinoline;
(e) 1-[2-[(4-phenoxyphenyl)amino]-4-pyrimidinyl]-6-methyl-1,2,3,4-tetrahydroquinoline;
(f) 1-[2-[(3,4-dimethoxyphenyl)amino]-4-pyrimidinyl]-6-methyl-1,2,3,4-tetrahydroquinoline;
(g) 1-[2-[(3,4,5-trimethoxyphenyl)amino]-4-pyrimidinyl]-6-methyl-1,2,3,4-tetrahydroquinoline;
(h) 1-[2-[(4,N-phenylaminophenyl)amino]-4-pyrimidinyl]-6-methyl-1,2,3,4-tetrahydroquinoline;
(i) [4-(6-Methyl-3,4-dihydro-2H-quinolin-1-yl)-pyrimidin-2-yl]-(6-morpholin-4-yl-pyridin-3-yl)-amine;
(j) 5-[4-(6-Methyl-3,4-dihydro-2H-quinolin-1-yl)-pyrimidin-2-ylamino]-1,3-dihydro-benzoimidazol-2-one;
(k) (2,3-Dimethyl-1H-indol-5-yl)-[4-(6-methyl-3,4-dihydro-2H-quinolin-1-yl)-pyrimidin-2-yl]-amine;
(l) [4-(6-Methyl-3,4-dihydro-2H-quinolin-1-yl)-pyrimidin-2-yl]-(2-methyl-2H-pyrazol-3-yl)-amine;
(m) (6-Methoxy-pyridin-3-yl)-[4-(6-methyl-3,4-dihydro-2H-quinolin-1-yl)-pyrimidin-2-yl]-amine;
(n) (4-Fluoro-3-methyl-phenyl)-[4-(6-methyl-3,4-dihydro-2H-quinolin-1-yl)-pyrimidin-2-yl]-amine;
(o) (5-Cyclopropyl-2H-pyrazol-3-yl)-[4-(6-methyl-3,4-dihydro-2H-quinolin-1-yl)-pyrimidin-2-yl]-amine;
(p) 4-Benzyl-N 3 -[4-(6-methyl-3,4-dihydro-2H-quinolin-1-yl)-pyrimidin-2-yl]-1H-pyrazole-3,5-diamine;
(q) [4-(6-Methyl-3,4-dihydro-2H-quinolin-1-yl)-pyrimidin-2-yl]-(4-methyl-thiazol-2-yl)-amine; and
(r) [4-(6-Methyl-3,4-dihydro-2H-quinolin-1-yl)-pyrimidin-2-yl]-(5-methyl-1H-pyrazol-3-yl)-amine.

11. A compound according to claim 1, selected from the group consisting of
(a) [4-(3,4-Dihydro-2H-quinolin-1-yl)-pyrimidin-2-yl]-(6-pyrrolidin-1-yl-pyridin-3-yl)-amine;
(b) (1-Cyclopentyl-1H-indol-6-yl)-[4-(6-methyl-3,4-dihydro-2H-quinolin-1-yl)-pyrimidin-2-yl]-amine;
(c) [4-(6-Methyl-3,4-dihydro-2H-quinolin-1-yl)-pyrimidin-2-yl]-oxazol-4-yl-amine;
(d) (3,4-Dichloro-phenyl)-[4-(6-methyl-3,4-dihydro-2H-quinolin-1-yl)-pyrimidin-2-yl]-amine; and
(e) [4-(3,4-Dihydro-2H-quinolin-1-yl)-pyrimidin-2-yl]-isothiazol-3-yl-amine.

12. A compound according to claim 1, selected from the group consisting of
(a) 2-({5-[4-(2,3-Dihydro-benzo[1,4]oxazin-4-yl)-pyrimidin-2-ylamino]-pyridin-2-yl}-methyl-amino)-ethanol;
(b) N-{5-[4-(3-Oxo-3,4-dihydro-2H-quinoxalin-1-yl)-pyrimidin-2-ylamino]-pyridin-2-yl}-acetamide;
(c) 3-Chloro-N-[4-(4-methyl-3-oxo-3,4-dihydro-2H-quinoxalin-1-yl)-pyrimidin-2-yl]-benzamide;
(d) [4-(2,3-Dihydro-benzo[1,4]thiazin-4-yl)-pyrimidin-2-yl]-oxazol-4-yl-amine;
(e) N-[4-(5-Fluoro-2,3-dihydro-indol-1-yl)-pyrimidin-2-yl]-3-methoxy-benzenesulfonamide;
(f) [4-(5,6-Dihydro-pyrrolo[2,3-d]pyrimidin-7-yl)-pyrimidin-2-yl]-(2-trifluoromethyl-phenyl)-amine;
(g) 6-Methoxy-1-[2-(pyridazin-3-ylamino)-pyrimidin-4-yl]-2,3-dihydro-1H-quinolin-4-one;
(h) 2-{5-[4-(3,4-Dihydro-2H-quinoxalin-1-yl)-pyrimidin-2-ylamino]-indol-1-yl}-ethanol;
(i) (2H-Pyrazol-3-yl)-[4-(7-trifluoromethyl-34-dihydro-2H-quinolin-1-yl)-pyrimidin-2-yl]-amine;
(j) 1-[4-(3,4-Dihydro-2H-[1,5]naphthyridin-1-yl)-pyrimidin-2-yl]-3-ethyl-urea;
(k) 1-[4-(2,3-Dihydro-benzo[1,4]oxazin-4-yl)-pyrimidin-2-yl]-3-(2-ethoxy-ethyl)-urea;
(l) [4-(3,3-Dimethyl-2,3-dihydro-indol-1-yl)-pyrimidin-2-yl]-carbamic acid tert-butyl ester;
(m) 3-Cyano-N-[4-(7-methoxy-2,3,4,5-tetrahydro-benzo[b]azepin-1-yl)-pyrimidin-2-yl]-benzamide;
(n) Isoxazol-4-yl-[4-(2,3,4,5-tetrahydro-benzo[b][1,4]diazepin-1-yl)-pyrimidin-2-yl]-amine;
(o) (3,4-Dichloro-phenyl)-[4-(3,4-dihydro-2H-benzo[b][1,4]thiazepin-5-yl)-pyrimidin-2-yl]-amine;
(p) (6-Aziridin-1-yl-pyridin-3-yl)-[4-(5-methanesulfonyl-2,3-dihydro-indol-1-yl)-pyrimidin-2-yl]-amine;
(q) N²-Cyclopropyl-N⁵-[4-(6-fluoro-3,4-dihydro-2H-quinolin-1-yl)-pyrimidin-2-yl]-pyridine-2,5-diamine; and
(r) Benzo[1,3]dioxole-5-carboxylic acid [4-(6-fluoro-3,4-dihydro-2H-quinolin-1-yl)-pyrimidin-2-yl]-amide.

13. A compound according to claim 1, wherein X is methylene.

14. A compound according to claim 1, wherein structure 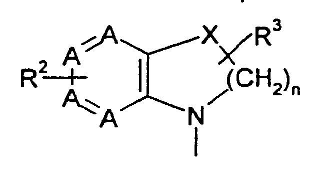 comprises a group selected from the group consisting of 2,3-Dihydro-1H-pyrrolo[2,3-b]pyridine; 2,3-Dihydro-1H-pyrrofo[2,3-c]pyridine; 2,3-Dihydro-1H-pyrrolo[3,2-c]pyridine; 2,3-Dihydro-1H-pyrrolo[3,2-b]pyridine; 6,7-Dihydro-5H-pyrrolo[3,2-d]pyrimidine; 6,7-Dihydro-5H-pyrrolo[3,2-d][1,2,3]triazine; 6,7-Dihydro-5H-pyrrolo[2,3-d][1,2,3]triazine; 1,4,5,7-Tetraaza-indan; 1,4,6,7-Tetraaza-indan; 6,7-Dihydro-5H-pyrrolo[2,3-c]pyridazine; 2,3-Dihydro-1H-pyrrolo[2,3-d]pyridazine; 6,7-Dihydro-5H-pyrrolo[3,2-c]pyridazine; 6,7-Dihydro-5H-pyrrolo[2,3-b]pyrazine; 6,7-Dihydro-5H-pyrimido[4,5-b][1,4]oxazine; 5,6,7,8-Tetrahydro-pteridine; 1,2,3,4-Tetrahydro-pyrido[2,3-b]pyrazine; 1,2,3,4-Tetrahydro-pyrido[3,4-b]pyrazine; 1,2,3,4-Tetrahydro-pyrido[3,4-b]pyrazine; 1,2,3,4-Tetrahydro-pyrido[2,3-b]pyrazine; 5,6,7,8-Tetrahydro-pyrazino[2,3-c]pyridazine; 5,6,7,8-Tetrahydro-pteridine; 1,2,3,4-Tetrahydro-pyrazino[2,3-d]pyridazine; 5,6,7,8-Tetrahydro-pyrazino[2,3-c]pyridazine; 1,2,3,4-Tetrahydro-pyrazino[2,3-b]pyrazine; 5,6,7,8-Tetrahydro-pyrazino[2,3-e][1,2,4]triazine; 5,6,7,8-Tetrahydro-pyrazino[2,3-e][1,2,4]triazine; 5,6,7,8-Tetrahydro-pyrazino[2,3-d][1,2,3]triazine; 5,6,7,8-Tetrahydro-pyrazino[2,3-d][1,2,3]triazine; 2.3-Dihydro-1H-4-oxa-1,5-diaza-naphthalene; 2,3-Dihydro-1H-4-oxa-1,6-diaza-naphthalene; 3,4-Dihydro-2H-1-oxa-4,6-diaza-naphthalene; 3,4-Dihydro-2H-1-oxa-4,5-diaza-naphthalene; 7,8-Dihydro-6H-5-oxa-1,2,8-triaza-naphthalene; 3,4-Dihydro-2H-1-oxa-4,6,7-triaza-naphthalene; 6,7-Dihydro-5H-8-oxa-1,2,5-triaza-naphthalene; 3,4-Dihydro-2H-1-oxa-4,5,8-triaza-naphthalene; 7,8-Dihydro-6H-pyrimido[5,4-b][1,4]oxazine; 6,7-Dihydro-5H-pyrimido[4,5-b][1,4]oxazine; 6,7-Dihydro-5H-8-oxa-1,2,3,5-tetraaza-naphthalene; 6,7-Dihydro-5H-8-oxa-1,2,4,5-tetraaza-naphthalene; 7,8-Dihydro-6H-5-oxa-1,2,3,8-tetraaza-naphthalene; 6,7-Dihydro-5H-8-oxa-1,2,4,5-tetraaza-naphthatene; 2,3-Dihydro-1H-pyrido[2,3-b][1,4]thiazine; 2,3-Dihydro-1H-4-thia-1,6-diaza-naphthalene; 3,4-Dihydro-2H-1-thia-4,6-diaza-naphthalene; 3,4-Dihydro-2H-pyrido[3,2-b][1,4]thiazine; 7,8-Dihydro-6H-5-thia-1,2,8-triaza-naphthalene; 3,4-Dihydro-2H-1-thia-4,6,7-triaza-naphthalene; 6,7-Dihydro-5H-8-thia-1,2,5-triaza-naphthalene; 6,7-Dihydro-5H-pyrimido[4,5-b][1,4]thiazine; 7,8-Dihydro-6H-pyrimido[5,4-b][1,4]thiazine; 3,4-Dihydro-2H-1-thia-4,5,8-triaza-naphthalene; 6,7-Dihydro-5H-8-thia-1,2,4,5-tetraaza-naphthalene; 7,8-Dihydro-6H-5-thia-1,2,4,8-tetraaza-naphthalene; 7,8-Dihydro-6H-5-thia-1,2,3,8-tetraaza-naphthalene; 6,7-Dihydro-5H-8-thia-1,2,3,5-tetraaza-naphthalene; 5,6,7,8-Tetrahydro-pyrido[3,2-d]pyrimidine; 1,2,3,4-Tetrahydropyrido[2,3-d]pyridazine; 5,6,7,8-Tetrahydro-pyrido[2,3-b]pyrazine; 5,6,7,8-Tetrahydropyrido[3,2-e][1,2,4]triazine; 5,6,7,8-Tetrahydro-pyrido[2,3-e][1,2,4]triazine; 5,6,7,8-Tetrahydropyrido[3,2-d][1,2,3]triazine; and 5,6,7,8-Tetrahydro-pyrido[2,3-d][1,2,3]triazine.

15. A pharmaceutical composition comprising an effective amount of a compound according to claim 1, and a pharmaceutical carrier.

16. A pharmaceutical composition according to claim 15, for treatment or prevention of a condition in a mammal wherein therapeutic benefit is achieved by modulation of a T-cell mediated cellular process.

17. A pharmaceutical composition according to claim 15, for treatment or prevention of transplant rejection in a mammal.

18. A pharmaceutical composition according to claim 15, for treatment or prevention of autoimmune disease in a mammal.

19. A pharmaceutical composition according to claim 15, for treatment or prevention of inflammatory disease in a mammal.

20. A pharmaceutical composition according to claim 15, for treatment or prevention of allergy in a mammal.

21. A pharmaceutical composition according to claim 15, for treating T-cell leukemia or T-cell lymphoma in a mammal.

22. A pharmaceutical composition according to claim 15, for the treatment of a disease in a mammal wherein treatment can be effected by inhibiting activation of T-cells, or the results of said activation.

23. A pharmaceutical composition according to claim 15, further comprising an additional agent which modulates the mammalian immune system or which is an anti-inflammatory agent.

24. A pharmaceutical composition according to claim 15, useful in the treatment of a disorder or condition in a mammal selected from the group consisting of asthma, hay fever, hives, infantile eczema, atopic dermatitis, and other allergic diseases involving antibody-mediated (intermediate-type) hypersensitivity reactions, transplant rejection, psoriasis, ulcerative colitis, Crohn's disease, lupus, multiple sclerosis, rheumatoid arthritis, type I diabetes, autoimmune thyroid disorders, T-cell malignancies including T-cell leukemia and T-cell lymphoma, and Alzheimer's disease.

25. A pharmaceutical composition according to claim 15, useful to inhibit tyrosine kinase Lck in a mammal.

26. A pharmaceutical composition according to claim 15, wherein said compound is provided as a pharmaceutically acceptable salt, solvate or hydrate thereof.

27. A compound, salt, soware or hydrate according to any one of claims 1 to 14, for use in medicine.

28. The use of a compound salt , soware or hydrate according to any one of claims 1 to 14 in the manufacture of a medicament for the treatment of acondition in a mammal wherein therapeutic benefit is achieved by modulation of a T-cell mediated cellular process, transplant rejection in a mammal, autoimmune disease in a mammal, inflammatory disease in a mammal, allergy in a mammal, T-cell leukemia or T-cell lymphoma in a mammal, a disease in a mammal wherein treatment can be effected by inhibiting activation of T-cells, or the results of said activation, asthma, hay fever, hives, infantile eczema, atopic dermatitis, and other allergic diseases involving antibody-mediated (intermediate-type) hypersensitivity reactions, transplant rejection, psoriasis, ulcerative colitis, Crohn's disease, lupus, multiple sclerosis, rheumatoid arthritis, type I diabetes, autoimmune thyroid disorders. T-cell malignancies including T-cell leukemia and T-cell lymphoma, Alheimer's disease.

## Patentansprüche

1. Verbindung der Formel 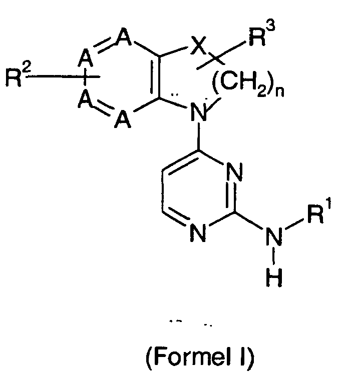 oder deren pharmazeutisch akzeptables Salz, Solvat oder Hydrat, worin jedes A jeweils unabhängig ausgewählt ist aus CH oder N;
X ausgewählt ist aus der Gruppe, enthaltend -CH₂-, -O-, -NH-, (C₁-C₆)-Alkylamino-, (C₁-C₆)-Alkylaminocarbonylamino-, (C₁-C₆)-Alkylcarbonylamino-, (C₁-C₆)-Alkylsulfonylamino-, Phenylsulfonylamino-, Carbonyl, -NH-C(O)-, -N-(C₁-C₆)-Alkyl-C(O)-, -S(O)_{y}-, wobei y 0, 1 oder 2 bedeutet, und
n in der Gruppe -(CH₂)ₙ- 1, 2 oder 3 bedeutet;
R¹ ausgewählt ist aus der Gruppe, enthaltend (C₆-C₁₀)-Aryl-, (C₁-C₉)-Heteroaryl-, (C₆-C₁₀)-Aryl-(C₆-C₁₀)-aryl-, (C₆-C₁₀)-Aryl-(C₁-C₉)-heteroaryl, (C₁-C₉)-Heteroaryl-(C₁-C₉)-heteroaryl-, (C₁-C₉)-Heteroaryl-(C₆-C₁₀)-aryl-, (C₆-C₁₀)-Arylsulfinyl-, (C₆-C₁₀)-Aryl-(C₆-C₁₀)-arylsulfinyl-, (C₁-C₉)-Heteroaryl-(C₆-C₁₀)-arylsulfinyl, (C₆-C₁₀)-Arylsulfonyl-, (C₆-C₁₀)-Aryl-(C₆-C₁₀)-arylsulfonyl-, (C₁-C₉)-Heteroaryl-(C₆-C₁₀)-arylsulfonyl-, (C₁-C₉)-Heteroarylsulfinyl-, (C₁-C₉)-Heteroaryl-(C₁-C₉)-heteroarylsulfinyl-, (C₆-C₁₀)-Aryl-(C₁-C₉)-heteroarylsulfinyl-, (C₁-C₉)-Heteroarylsulfonyl-, (C₁-C₉)- Heteroaryl-(C₁-C₉)-heteroarylsulfonyl-, (C₆-C₁₀)-Aryl-(C₁-C₉)-heteroarylsulfonyl-, (R⁴)-Sulfinyl-, (R⁴)-Sulfonyl-, (C₆-C₁₀)-Aryl-(R⁴)-sulfinyl-, (C₆-C₁₀)-Aryl-(C₆-C₁₀)-aryl-(R⁴)-sulfinyl-, (C₁-C₉)-Heteroaryl-(C₆-C₁₀)-aryl-(R⁴)-sulfinyl-, (C₆-C₁₀)-Aryl-(R⁴)-sulfonyl-, (C₆-C₁₀)-Aryl-(C₆-C₁₀)-aryl-(R⁴)-sulfonyl-, (C₁-C₉)-Heteroaryl-(C₆-C₁₀)-aryl-(R⁴)-sulfonyl-, (C₁-C₉)-Hetero-aryl-(R⁴)-sulfinyl-, (C₆-C₁₀)-Aryl-(C₁-C₉)-heteroaryl-(R⁴)-sulfinyl-, (C₁-C₉)-Heteroaryl-(C₁-C₉)-heteroaryl-(R⁴)-sulfinyl-, (C₁-C₉)-Heteroaryl-(R⁴)-sulfonyl-, (C₆-C₁₀)-Aryl-(C₁-C₉)-heteroaryl-(R⁴)-sulfonyl-, (C₁-C₉)-Heteroaryl-(C₁-C₉)-heteroaryl-(R⁴)-sulfonyl-, (C₆-C₁₀)-Arylaminocarbonyl-, (C₆-C₁₀)-Aryl-(C₆-C₁₀)-arylaminocarbonyl-, (C₁-C₉)-Heteroaryl-(C₆-C₁₀)-arylaminocarbonyl-, (C₁-C₉)-Heteroarylaminocarbonyl-, (C₆-C₁₀)-Aryl-(C₁-C₉)-heteroarylaminocarbonyl-, (C₁-C₉)-Heteroaryl-(C₁-C₉)-heteroarylaminocarbonyl-, (C₆-C₁₀)-Arylcarbonyl-, (C₆-C₁₀)-Aryl-(C₆-C₁₀)-arylcarbonyl-, (C₁-C₉)-Heteroaryl-(C₆-C₁₀)-arylcarbonyl-, (C₁-C₉)-Heteroarylcarbonyl-, (C₆-C₁₀)-Aryl-(C₁-C₉)-heteroarylcarbonyl-, (C₁-C₉)-Heteroaryl-(C₁-C₉)-heteroarylcarbonyl-, (C₆-C₁₀)-Aryloxycarbonyl-, (C₆-C₁₀)-Aryl-(C₆-C₁₀)-aryloxycarbonyl-, (C₁-C₉)-Heteroaryl-(C₆-C₁₀)-aryloxycarbonyl-, (C₁-C₉)-Heteroaryloxycarbonyl-, (C₆-C₁₀)-Aryl-(C₁-C₉)-heteroaryloxycarbonyl-, (C₁-C₉)-Heteroaryl-(C₁-C₉)-heteroaryloxycarbonyl-, (R⁴)-Carbonyl-, (R⁴)-Oxycarbonyl-, (R⁴)-Aminocarbonyl-, (C₆-C₁₀)-Aryl-(R⁴)-carbonyl-, (C₆-C₁₀)-Aryl-(R⁴)-oxycarbonyl-, (C₆-C₁₀)-Aryl-(R⁴)-aminocarbonyl-, (C₁-C₉)-Heteroaryl-(R⁴)-carbonyl-, (C₁-C₉)-Heteroaryl-(R⁴)-oxycarbonyl- und (C₁-C₉)-Heteroaryl-(R⁴)-aminocarbonyl-; worin R⁴ ausgewählt ist aus den Gruppen, enthaltend
(a) (C₁-C₆)-Alkyl-, (C₂-C₆)-Alkenyl- und (C₂-C₆)-Alkinyl-, wobei die Alkyl-, Alkenyl- und Alkinylgruppen optional substituiert sind durch Hydroxy, Halogen, Amino, Trifluormethyl, Hydroxy-(C₂-C₆)-alkyl-, (C₁-C₆)-Alkoxy-, (C₁-C₆)-Acyloxy-, (C₁-C₆)-Alkylamino-, ((C₁-C₆)-Alkyl)₂-amino-, (C₁-C₆)-Acylamino-, Cyano, Nitro, (C₁-C₆)-Alkyl-, (C₂-C₆)-Alkenyl-, (C₂-C₆)-Alkinyl-, (C₁-C₆)-Acylamino-, Cyano-(C₁-C₆)-alkyl-, Trifluormethyl-(C₁-C₆)-alkyl-, (C₁-C₃)-Alkyl(difluormethylen)-(C₁-C₃)-alkyl- oder Nitro-(C₁-C₆)-alkyl-;
(b) (C₃-C₁₀)-Cycloalkyl-, worin die Cycloalkylgruppe optional substituiert ist durch Hydroxy, Halogen, Amino, Trifluormethyl, Hydroxy-(C₂-C₆)- alkyl-, (C₁-C₆)-Alkoxy-, (C₁-C₆)-Acyloxy-, (C₁-C₆)-Alkylamino-, ((C₁-C₆)-Alkyl)₂-amino-, (C₁-C₆)-Acylamino-, Cyano, Nitro, (C₁-C₆)-Alkyl-, (C₂-C₆)-Alkenyl-, (C₂-C₆)-Alkinyl-, (C₁-C₆)-Acylamino-, Cyano-(C₁-C₆)-alkyl-, Trifluormethyl-(C₁-C₆)-alkyl-, (C₁-C₃)-Alkyl(difluormethylen)-(C₁-C₃)-alkyl- oder Nitro-(C₁-C₆)-alkyl-; oder
(c) (C₃-C₁₀)-Heterocycloalkyl-, worin die Heterocycloalkylgruppe optional substituiert ist durch Hydroxy,-Halogen, Amino, Trifluormethyl, Hydroxy-(C₂-C₆)-alkyl-, (C₁-C₆)-Alkoxy-, (C₁-C₆)-Acyloxy-, (C₁-C₆)-Alkylamino-, ((C₁-C₆)-Alkyl)₂-amino-, (C₁-C₆)-Acylamino-, Cyano, Nitro, (C₁-C₆)-Alkyl-, (C₂-C₆)-Alkenyl-, (C₂-C₆)-Alkinyl-, (C₁-C₆)-Acylamino-, Cyano-(C₁-C₆)-alkyl-, Trifluormethyl-(C₁-C₆)-alkyl-, (C₁-C₃)-Alkyl(difluormethylen)-(C₁-C₃)-alkyl- oder Nitro-(C₁-C₆)-alkyl-; und
wobei jede der für R¹ genannten (C₆-C₁₀)-Aryl- oder (C₁-C₉)-Heteroaryl-Gruppen optional ein- bis fünffach substituiert ist durch
(a) Deuterium, Hydroxy, Halogen, Amino, Trifluormethyl, Carboxy, (C₁-C₆)-Alkoxy-, (C₁-C₆)-Acyloxy-, (C₁-C₆)-Alkylamino-, ((C₁-C₆)-Alkyl)₂-amino-, (C₁-C₆)-Acylamino-, Cyano, Nitro, (C₁-C₆)-Alkyl-, (C₂-C₆)-Alkenyl-, (C₂-C₆)-Alkinyl-, (C₁-C₆)-Acylamino-, Cyano-(C₁-C₆)-alkyl-, Trifluormethyl-(C₁-C₆)-alkyl- oder Nitro-(C₁-C₆)-alkyl-, (C₁-C₃)-Alkyl(difluormethylen)-(C₁-C₃)-alkyl-, (C₁-C₆)-Acylamino-(C₁-C₆)-alkyl-, (C₁-C₆)-Alkoxy-(C₁-C₆)-acylamino, Amino-(C₁-C₆)-acyl-, Amino-(C₁-C₆)-acyl-(C₁-C₆)-alkyl-, (C₁-C₆)-Alkylamino-(C₁-C₆)-acyl-, ((C₁-C₆)-alkyl)₂-amino-(C₁-C₆)-acyl-, (C₃-C₁₀)-Cycloalkyl-(C₁-C₆)-alkyl-, (C₁-C₆)-Acyloxy-(C₁-C₆)-alkyl-, (C₂-C₆)-Alkoxy-(C₁-C₆)-alkyl-, Piperazinyl-(C₁-C₆)-alkyl-, (C₁-C₆)-Acylamino-(C₁-C₆)-alkyl, (C₆-C₁₀)-Aryl-(C₁-C₆)-alkoxy-(C₁-C₆)-alkyl-, (C₅-C₉)-Heteroaryl-(C₁-C₆)-alkoxy-(C₁-C₆)-alkyl-, (C₁-C₆)-Alkylthio-(C₁-C₆)-alkyl-, (C₆-C₁₀)-Arylthio-(C₁-C₆)-alkyl-, (C₁-C₆)-Alkylsulfinyl-(C₁-C₆)-alkyl-, (C₆-C₁₀)-Arylsulfinyl-(C₁-C₆)-alkyl-, (C₁-C₆)-Alkylsulfonyl-(C₁-C₆)-alkyl-, (C₆-C₁₀)-Arylsulfonyl-(C₁-C₆)-alkyl-, Amino-(C₁-C₆)-alkyl-, (C₁-C₆)-Alkylamino-(C₁-C₆)-alkyl-, (C₁-C₆)-Alkyl(difluormethylen)-, (C₁-C₃)-Alkyl(difluormethylen)-(C₁-C₃)-alkyl-, (C₁-C₆)-Alkoxy-(C₁-C₆)-acyl-, (C₁-C₆)-Alkylamino-(C₁-C₆)-acyl-, ((C₁-C₆)-Alkyl)₂-amino-(C₁-C₆)-acyl-, (C₆-C₁₀)-Aryl-, (C₁-C₉)-Heteroaryl-, (C₆-C₁₀)- Aryl-(C₁-C₆)-alkyl-, (C₁-C₉)-Heteroaryl-(C₁-C₆)-alkyl-, (C₆-C₁₀)-Aryl-(C₆-C₁₀)-aryl-, (C₆-C₁₀)-Aryl-(C₆-C₁₀)-aryl-(C₁-C₆)-alkyl-, (C₃-C₁₀)-Cycloalkyl-, (C₃-C₆)-Cycloalkyl-(C₁-C₆)-alkyl-, (C₃-C₁₀)-Heterocycloalkyl-, (C₃-C₁₀)-Heterocycloalkyl-(C₁-C₆)-alkyl-, Hydroxy-(C₂-C₆)-alkyl-, (C₁-C₆)-Acyloxy-(C₂-C₆)-alkyl-, (C₁-C₆)-Alkoxy-(C₂-C₆)-alkyl-, Piperazinyl-(C₁-C₆)-alkyl-, (C₁-C₆)-Acylamino-(C₁-C₆)-alkyl-, (C₆-C₁₀)-Aryl-(C₁-C₆)-alkoxy-(C₁-C₆)-alkyl-, (C₁-C₉)-Heteroaryl-(C₁-C₆)-alkoxy-(C₁-C₆)-alkyl-, (C₁-C₆)-Alkylthio-(C₁-C₆)-alkyl-, (C₆-C₁₀)-Arylthio-(C₁-C₆)-alkyl-, (C₁-C₆)-Alkylsulfinyl-(C₁-C₆)-alkyl-, (C₆-C₁₀)-Arylsulfinyl-(C₁-C₆)-alkyl-, (C₁-C₆)-Alkylsulfonyl-(C₁-C₆)-alkyl-, (C₆-C₁₀)-Arylsulfonyl-(C₁-C₆)-alkyl-, Amino-(C₁-C₆)-alkyl-, (C₁-C₆)-Alkylamino-(C₁-C₆)-alkyl-, ((C₁-C₆)-Alkyl)₂-amino-(C₁-C₆)-alkyl-;
(b) R⁵OCO(C₁-C₆)-Alkyl-, wobei R⁵ ausgewählt ist aus der Gruppe, enthaltend Wasserstoff, (C₁-C₆)-Alkyl-, (C₆-C₁₀)-Aryl-(C₁-C₆)-alkyl-, (C₁-C₉)-Heteroaryl-(C₁-C₆)-alkyl; oder
(c) R⁶(C₀-C₆)-Alkyl-, wobei R⁶ ausgewählt ist aus der Gruppe, enthaltend Piperazino, (C₁-C₆)-Acylpiperazino-, (C₆-C₁₀)-Arylpiperazino-, (C₅-C₉)-Heteroarylpiperazino-, (C₁-C₆)-Alkylpiperazino-, (C₆-C₁₀)-Aryl-(C₁-C₆)-alkylpiperazino-, (C₁-C₉)-Heteroaryl-(C₁-C₆)-alkylpiperazino-, Morpholino-, (C₁-C₆)-Acylmorpholino-, (C₆-C₁₀)-Arylmorpholino-, (C₁-C₉)-Heteroarylmorpholino-, (C₁-C₆)-Alkylmorpholino-, (C₆-C₁₀)-Aryl-(C₁-C₆)-alkylmorpholino-, (C₁-C₉)-Heteroaryl-(C₁-C₆)-alkylmorpholino-, Thiomorpholino-, (C₁-C₆)-Acylthiomorpholino-, (C₆-C₁₀)-Arylthiomorpholino-, (C₁-C₉)-Heteroarylthiomorpholino-, (C₁-C₆)-Alkylthiomorpholino-, (C₆-C₁₀)-Aryl-(C₁-C₆)-alkylthiomorpholino-, (C₁-C₉)-Heteroaryl-(C₁-C₆)-alkylthiomorpholino-, Piperidino-, (C₁-C₆)-Acylpiperidino-, (C₆-C₁₀)-Arylpiperidino-, (C₁-C₉)-Heteroarylpiperidino-, (C₁-C₆)-Alkylpiperidino-, (C₆-C₁₀)-Aryl-(C₁-C₆)-piperidino-, (C₁-C₉)-Heteroaryl-(C₁-C₆)-alkylpiperidino-, Pyrrolidino-, (C₁-C₆)-Acylpyrrolidino-, (C₆-C₁₀)-Aryl-pyrrolidino-, (C₁-C₉)-Heteroarylpyrrolidino-, (C₁-C₆)-Alkylpyrrolidino-, (C₆-C₁₀)-Aryl-(C₁-C₆)-pyrrolidino-, (C₁-C₉)-Heteroaryl-(C₁-C₆)-alkylpyrrolidino-, (C₁-C₆)-Alkoxy- (C₁-C₆)-acyl-, (C₁₋C₆)-Alkylamino-(C₆-C₁₀)-aryl- und ((C₁-C₆)-Alkyl)₂-amino-(C₁-C₆)-acyl-;
R² für 1 - 4 optionale Substituenten steht, die jeweils unabhängig aus den Gruppen (a) bis (f) ausgewählt sind:
(a) Deuterium, Halogen, Hydroxy, Carboxy, Amino, Trifluormethyl, (C₁-C₆)-Alkyl-, (C₂-C₆)-Alkenyl-, (C₂-C₆)-Alkinyl-, (C₁-C₆)-Alkylamino-, ((C₁-C₆)Alkyl)₂-amino-, Cyanoalkyl-, (C₃-C₁₀)-Cycloalkyl-, (C₃-C₁₀)-Heterocycloalkyl-, (C₃-C₁₀)-Cycloalkoxy-, (C₁-C₆)-Alkylthio-, (C₁-C₆)-Alkylsulfinyl-, (C₁-C₆)-Alkylsulfonyl-, Amino-CO-NH-, (C₁-C₆)-Alkoxy-CO-NH-, (C₁-C₆)-Alkyl-CO-NH-, (C₁-C₆)-Alkyl-CO-NH-(C₁-C₆)-alkyl-, (C₁-C₆)-Alkyl-CO-NH-(C₁-C₆)-alkoxy-, (C₁-C₆)-Alkoxycarbonyl-(C₁-C₆)-alkoxy-, (C₁-C₆)-Alkoxy-CO-NH-(C₁-C₆)-alkoxy-, (C₁-C₆)-Alkylamino-CO-NH-, (C₁-C₆)-Alkylamino-CO-NH-(C₁-C₆)-Alkyl-, ((C₁-C₆)-Alkyl)₂-amino-CO-NH-(C₁-C₆)-alkyl-, ((C₁-C₆)-Alkyl)₂-amino-CO-NH-, Carboxy, Carboxy-(C₁-C₆)-alkyl-, Carboxy-(C₁-C₆)-alkoxy-, Benzyloxycarbonyl-(C₁-C₆)-alkoxy-, (C₁-C₆)-Alkylamino-CO-, (C₁-C₆)-Acylamino-, (C₁-C₆)-Alkoxy-, (C₁-C₆)-Acyl-, (C₁-C₆)-Acyloxy-, (C₁-C₆)-Acyl-(C₁-C₆)-alkylamino-, (C₁-C₆)-Alkoxyacyl-, (C₁-C₆)-Alkylaminoacyl-, ((C₁-C₆)-Alkyl)₂-aminoacyl-, Amino-(C₁-C₆)-acyl-, Amino-(C₁-C₆)-alkyl-, (C₁-C₆)-Alkoxycarbonylamino-, (C₁-C₆)-Alkoxycarbonyl-(C₁-C₆)-alkyl-, (C₆-C₁₀)-Aryl-(C₁-C₆)-alkoxycarbonylamino-, Trihalomethyl-, Trihalomethyl-(C₁-C₆)-alkyl-, (C₁-C₆)-Alkyldihalomethylen-, (C₁-C₃)-Alkyl(dihalomethylen)-(C₁-C₃)-alkyl-, (C₃-C₆)-Cycloalkyl-, (C₃-C₆)-Cycloalkyl-(C₁-C₆)-alkyl-, Hydroxy-(C₁-C₆)-alkyl-, (C₁-C₆)-Acyloxy-(C₁-C₆)-alkyl-, (C₁-C₆)-Alkoxy-(C₁-C₆)-alkyl-, (C₁-C₆)-Acylamino-(C₁-C₆)-alkyl-, (C₁-C₆)-Alkylthio-(C₁-C₆)-alkyl-, (C₁-C₆)-Alkoxycarbonyl-, (C₁-C₆)-Alkylsulfinyl-(C₁-C₆)-alkyl-, (C₁-C₆)-Alkylsulfonyl-(C₁-C₆)-alkyl-, (C₁-C₆)-Alkylsulfonyl-, (C₁-C₆)-Alkylsulfonylamino-, (C₁-C₆)-Alkylsulfonylamino-(C₁-C₆)-alkyl-, Amino-(C₁-C₆)-alkyl-, (C₁-C₆)-Alkylamino-(C₁-C₆)-alkyl-, ((C₁-C₆)-Alkyl)₂-amino-(C₁-C₆)-alkyl-, (C₁-C₆)-CO-(C₁-C₆)-Alkyl-;
(b) (C₆-C₁₀)-Aryl-, (C₁-C₉)-Heteroaryl-, (C₆-C₁₀)-Aryl-(C₆-C₁₀)-aryl-, (C₆-C₁₀)-Aryl-(C₁-C₉)-heteroaryl-, (C₁-C₉)-Heteroaryl-(C₁-C₉)-heteroaryl-, (C₁-C₉)-Heteroaryl-(C₆-C₁₀)-aryl-, (C₆-C₁₀)-Arylsulfinyl-, (C₆-C₁₀)-Aryl- (C₆-C₁₀)-arylsulfinyl-, (C₁-C₉)-Heteroaryl-(C₆-C₁₀)-arylsulfinyl-, (C₆-C₁₀)-Arylsulfonyl-, (C₆-C₁₀)-Aryl-(C₆-C₁₀)-arylsulfonyl-, (C₁-C₉)-Heteroaryl-(C₆-C₁₀)-arylsulfonyl-, (C₁-C₉)-Heteroarylsulfinyl-, (C₁-C₉)-Heteroaryl-(C₁-C₉)-heteroarylsulfinyl-, (C₆-C₁₀)-Aryl-(C₁-C₉)-heteroarylsulfinyl, (C₁-C₉)-Heteroarylsulfonyl-, (C₁-C₉)-Heteroaryl-(C₁-C₉)-heteroarylsulfonyl-, (C₆-C₁₀)-Aryl-(C₁-C₉)-heteroarylsulfonyl-, (R⁴)-Sulfinyl-, (R⁴)-Sulfonyl-, (C₆-C₁₀)-Aryl-(R⁴)-sulfinyl-, (C₆-C₁₀)-Aryl-(C₆-C₁₀)-aryl-(R⁴)-sulfinyl-, (C₁-C₉)-Heteroaryl-(C₆-C₁₀)-aryl-(R⁴)-sulfinyl, (C₆-C₁₀)-Aryl-(R⁴)-sulfonyl, (C₆-C₁₀)-Aryl-(C₆-C₁₀)-Aryl-(R⁴)-sulfonyl-, (C₁-C₉)-Heteroaryl-(C₆-C₁₀)-aryl-(R⁴)-sulfonyl, (C₁-C₉)-Heteroaryl-(R⁴)-sulfinyl-, (C₆-C₁₀)-Aryl-(C₁-C₉)-heteroaryl-(R⁴)-sulfinyl-, (C₁-C₉)-Heteroaryl-(C₁-C₉)-heteroaryl-(R⁴)-sulfinyl-, (C₁-C₉)-Heteroaryl-(R⁴)-sulfonyl-, (C₆-C₁₀)-Aryl-(C₅-C₉)-heteroaryl-(R⁴)-sulfonyl-, (C₁-C₉)-Heteroaryl-(C₁-C₉)-heteroaryl-(R⁴)-sulfonyl-, (C₆-C₁₀)-Arylaminocarbonyl-, (C₆-C₁₀)-Aryl-(C₆-C₁₀)-arylaminocarbonyl-, (C₁-C₉)-Heteroaryl-(C₆-C₁₀)-arylaminocarbonyl-, (C₁-C₉)-Heteroarylaminocarbonyl-, (C₆-C₁₀)-Aryl-(C₁-C₉)-heteroarylaminocarbonyl-, (C₁-C₉)-Heteroaryl-(C₁-C₉)-heteroarylaminocarbonyl-, (C₆-C₁₀)-Arylcarbonyl-, (C₆-C₁₀)-Aryl-(C₆-C₁₀)-arylcarbonyl-, (C₁-C₉)-Heteroaryl-(C₆-C₁₀)-arylcarbonyl-, (C₁-C₉)-Heteroarylcarbonyl-, (C₆-C₁₀)-Aryl-(C₁-C₉)-heteroarylcarbonyl-, (C₁-C₉)-Heteroaryl-(C₁-C₉)-heteroarylcarbonyl-, (C₆-C₁₀)-Aryloxycarbonyl-, (C₆-C₁₀)-Aryl-(C₆-C₁₀)-aryloxycarbonyl-, (C₁-C₉)-Heteroaryl-(C₆-C₁₀)-aryloxycarbonyl-, (C₁-C₉)-Heteroaryloxycarbonyl-, (C₆-G₁₀)-Aryl-(C₁-C₉)-heteroaryloxycarbonyl-, (C₁-C₉)-Heteroaryl-(C₁-C₉)-heteroaryloxycarbonyl-, (R⁴)-Carbonyl-, (R⁴)-Oxycarbonyl-, (R⁴)-Aminocarbonyl-, (C₆-C₁₀)-Aryl-(R⁴)-carbonyl-, (C₆-C₁₀)-Aryl-(R⁴)-oxycarbonyl-, (C₆-C₁₀)-Aryl-(R⁴)-aminocarbonyl-, (C₁-C₉)-Heteroaryl-(R⁴)-carbonyl-, (C₅-C₉)-Heteroaryl-(R⁴)-oxycarbonyl-, (C₁-C₉)-Heteroaryl-(R⁴)-aminocarbonyl-, worin R⁴ die oben angegebenen Bedeutungen aufweist, und worin jede der genannten (C₆-C₁₀)-Aryl- und (C₁-C₉)-Heteroaryl-Gruppen in R² jeweils optional ein- bis fünffach unabhängig substituiert ist durch:
(i) Hydroxy, Halogen, Amino, Trifluormethyl, Carboxy, (C₁-C₆)-Alkoxy-, (C₁-C₆)-Acyloxy-, (C₁-C₆)-Alkylamino-, ((C₁-C₆)-Alkyl)₂-amino-, (C₁-C₆)-Acylamino-, Cyano, Nitro, (C₁-C₆)-Alkyl-, (C₂-C₆)-Alkenyl-, (C₂-C₆)-Alkinyl-, (C₁-C₆)-Acylamino-, Cyano-(C₁-C₆)-alkyl-, Trifluormethyl-(C₁-C₆)-alkyl- oder Nitro-(C₁-C₆)-alkyl-, (C₁-C₃)-Alkyl(difluormethylen)-(C₁-C₃)-alkyl-, (C₁-C₆)-Acylamino-(C₁-C₆)-alkyl-, (C₁-C₆)-Alkoxy-(C₁-C₆)-acylamino-, Amino-(C₁-C₆)-acyl-, Amino-(C₁-C₆)-acyl-(C₁-C₆)-alkyl-, (C₁-C₆)-Alkylamino-(C₁-C₆)-acyl-, ((C₁-C₆)-Alkyl)2-amino-(C₁-C₆)-acyl-, (C₃-C₁₀)-Cycloalkyl-(C₁-C₆)-alkyl-, (C₁-C₆)-Acyloxy-(C₁-C₆)-alkyl-, (C₂-C₆)-Alkoxy-(C₁-C₆)-alkyl-, Piperazinyl-(C₁-C₆)-alkyl, (C₁-C₆)-Acylamino-(C₁-C₆)-alkyl-, (C₆-C₁₀)-Aryl-(C₁-C₆)-alkoxy-(C₁-C₆)-alkyl-, (C₁-C₉)-Heteroaryl-(C₁-C₆)-alkoxy-(C₁-C₆)-alkyl-, (C₁-C₆)-Alkylthio-(C₁C₆)-alkyl-, (C₆-C₁₀)-Arylthio-(C₁-C₆)-alkyl-, (C₁-C₆)-Alkylsulfinyl-(C₁-C₆)-alkyl-, (C₆-C₁₀)-Arylsulfinyl-(C₁-C₆)-alkyl-, (C₁-C₆)-Alkylsulfonyl-(C₁-C₆)-alkyl-,(C₆-C₁₀)-Arylsulfonyl-(C₁-C₆)-alkyl-, Amino-(C₁-C₆)-alkyl-, (C₁-C₆)-Alkylamino-(C₁-C₆)-alkyl-, (C₁-C₆)-Alkyl(difluormethylen)-, (C₁-C₃)-Alkyl(difluormethylen)-(C₁-C₃)-alkyl-, (C₁-C₆)-Alkoxy-(C₁-C₆)-acyl-, (C₁-C₆)-Alkylamino-(C₁-C₆)-acyl-, ((C₁-C₆)-Alkyl)₂-amino-(C₁-C₆)-acyl-, (C₆-C₁₀)-Aryl-, (C₁-C₉)-Heteroaryl-, (C₆-C₁₀)-Aryl-(C₁-C₆)-alkyl-, (C₁-C₉)-Heteroaryl-(C₁-C₆)-alkyl-, (C₆-C₁₀)-Aryl-(C₆-C₁₀)-aryl-, (C₆-C₁₀)-Aryl-(C₆-C₁₀)-aryl-(C₁-C₆)-alkyl-, (C₃-C₁₀)-Cycloalkyl-, (C₃-C₆)-Cycloalkyl-(C₁-C₆)-alkyl-, (C₃-C₁₀)-Heterocycloalkyl-, (C₃-C₁₀)-Heterocycloalkyl-(C₁-C₆)-alkyl-, Hydroxy-(C₂-C₆)-alkyl-, (C₁-C₆)-Acyloxy-(C₂-C₆)-alkyl-, (C₁-C₆)-Alkoxy-(C₂-C₆)-alkyl-, Piperazinyl-(C₁-C₆)-alkyl-, (C₁-C₆)-Acylamino-(C₁-C₆)-alkyl-, (C₆-C₁₀)-Aryl-(C₁-C₆)-alkoxy-(C₁-C₆)-alkyl-, (C₁-C₉)-Heteroaryl-(C₁-C₆)-alkoxy-(C₁-C₆)-alkyl-, (C₁-C₆)-Alkylthio-(C₁-C₆)-alkyl-, (C₆-C₁₀)-Arylthio-(C₁-C₆)-alkyl-, (C₁-C₆)-Alkylsulfinyl-(C₁-C₆)-alkyl-, (C₆-C₁₀)-Arylsulfinyl-(C₁-C₆)-alkyl-, (C₁-C₆)-Alkylsulfonyl-(C₁-C₆)-alkyl-, (C₆-C₁₀)-Arylsulfonyl-(C₁-C₆)-alkyl-, Amino-(C₁-C₆)-alkyl-, (C₁-C₆)-Alkylamino-(C₁-C₆)-alkyl-, ((C₁-C₆)-Alkyl)₂-amino-(C₁-C₆)-alkyl-;
(ii) R⁵OCO(C₁-C₆)-Alkyl-, wobei R⁵ ausgewählt ist aus Wasserstoff, (C₁-C₆)-Alkyl-, (C₆-C₁₀)-Aryl-(C₁-C₆)-alkyl-, (C₁-C₉)-Heteroaryl-(C₁-C₆)-alkyl;
(iii) R⁶(C₀-C₆)-Alkyl-, wobei R⁶ ausgewählt ist aus der Gruppe, enthaltend Piperazino, (C₁-C₆)-Acylpiperazino-, (C₆-C₁₀)-Aryl-piperazino-, (C₅-C₉)-Heteroarylpiperazino-, (C₁-C₆)-Alkylpiperazino-, (C₆-C₁₀)-Aryl-(C₁-C₆)-alkylpiperazino-, (C₁-C₉)-Heteroaryl-(C₁-C₆)-alkylpiperazino-, Morpholino-, (C₁-C₆)-Acylmorpholino-, (C₆-C₁₀)-Arylmorpholino-, (C₁-C₉)-Heteroarylmorpholino-, (C₁-C₆)-Alkylmorpholino-, (C₆-C₁₀)-Aryl-(C₁-C₆)-alkylmorpholino-, (C₁-C₉)-Heteroaryl-(C₁-C₆)-alkylmorpholino-, Thiomorpholino-, (C₁-C₆)-Acylthiomorpholino-, (C₆-C₁₀)-Arylthiomorpholino-, (C₁-C₉)-Heteroarylthiomorpholino-, (C₁-C₆)-Alkylthiomorpholino-, (C₁-C₁₀)-Aryl-(C₁-C₆)-alkylthiomorpholino-, (C₁-C₉)-Heteroaryl-(C₁-C₆)-alkylthiomorpholino-, Piperidino-, (C₁-C₆)-Acylpiperidino-, (C₆-C₁₀)-Arylpiperidino-, (C₁-C₉)-Heteroarylpiperidino-, (C₁-C₆)-Alkylpiperidino-, (C₆-C₁₀)-Aryl-(C₁-C₆)-piperidino-, (C₁-C₉)-Heteroaryl-(C₁-C₆)-alkylpiperidino-, Pyrrolidino-, (C₁-C₆)-Acylpyrrolidino-, (C₆-C₁₀)-Arylpyrrolidino-, (C₁-C₉)-Heteroarylpyrrolidino-, (C₁-C₆)-Alkylpyrrolidino-, (C₆-C₁₀)-Aryl-(C₁-C₆)-alkylpyrrolidino-, (C₁-C₉)-Heteroaryl-(C₁-C₆)-alkylpyrrolidino-, (C₁-C₆)-Alkoxy-(C₁-C₆)-acyl-, (C₁-C₆)-Alkylamino-(C₆-C₁₀)-aryl- und ((C₁-C₆)-Alkyl)₂-amino-(C₁-C₆)-acyl-;
(c) R⁷ oder R⁷Y-, worin R⁷ ausgewählt ist aus der Gruppe, enthaltend Piperazino-, (C₆-C₁₀)-Arylpiperazino-, (C₁-C₉)-Heteroarylpiperazino-, (C₁-C₆)-Alkylpiperazino-, (C₆-C₁₀)-Aryl-(C₁-C₆)-alkylpiperazino-, (C₁-C₉)-Heteroaryl-(C₁-C₆)-alkylpiperazino-, Morpholino-, (C₆-C₁₀)-Arylmorpholino-, (C₁-C₉)-Heteroarylmorpholino-, (C₁-C₆)-Alkylmorpholino-, (C₆-C₁₀)-Aryl-(C₁-C₆)-alkylmorpholino-, (C₁-C₉)-Heteroaryl-(C₁-C₆)-alkylmorpholino-, Thiomorpholino-, (C₆-C₁₀)-Arylhiomorpholino-, (C₁-C₉)-Heteroarylthiomorpholino-, (C₁-C₆)-Alkylthiomorpholino-, (C₆-C₁₀)-Aryl-(C₁-C₆)-alkylthiomorpholino-, (C₁-C₉)-Heteroaryl-(C₁-C₆)-alkylthiomorpholino-, Piperidino-, (C₆-C₁₀)- Arylthiopiperidino-, (C₁-C₉)-Heteroarylthiopiperidino-, (C₁-C₆)-Alkylthiopiperidino-, (C₆-C₁₀)-Aryl-(C₁-C₆)-alkylthiopiperidino-, (C₁-C₉)-Heteroaryl-(C₁-C₆)-alkylthiopiperidino-, Pyrrolidino-, (C₆-C₁₀)-Arylthiopyrrolidino-, (C₁-C₉)-Heteroarylthiopyrrolidino-, (C₁-C₆)-Alkylthiopyrrolidino-, (C₆-C₁₀)-Aryl-(C₁-C₆)-alkylthiopyrrolidino-, (C₁-C₉)-Heteroaryl-(C₁-C₆)-alkylthiopyrrolidino-, und Y, sofern vorhanden, ausgewählt ist aus der Gruppe, enthaltend (C₁-C₆)-Alkyl-, (C₂-C₆)-Alkenyl-, (C₂-C₆)-Alkinyl-, Amino, Sauerstoff, Thio, Sulfinyl, Sulfonyl, Halo-(C₁-C₆)-alkyl- und Hydroxy-(C₂-C₆)-alkyl-;
(d) ZR⁸, worin R⁸ ausgewählt ist aus der Gruppe, enthaltend Piperazino-, (C₆-C₁₀)-Arylpiperazino-, (C₁-C₉)-Heteroarylpiperazino-, (C₁-C₆)-Alkylpiperazino-, (C₆-C₁₀)-Aryl-(C₁-C₆)-alkylpiperazino-, (C₁-C₉)-Heteroaryl-(C₁-C₆)-alkylpiperazino-, Morpholino-, (C₆-C₁₀)-Arylmorpholino-, (C₁-C₉)-Heteroarylmorpholino-, (C₁-C₆)-Alkylmorpholino-, (C₆-C₁₀)-Aryl-(C₁-C₆)-alkylmorpholino-, (C₁-C₉)-Heteroaryl-(C₁-C₆)-alkylmorpholino-, Thiomorpholino-, (C₆-C₁₀)-Arylthiomorpholino-, (C₁-C₉)-Heteroarylthiomorpholino-, (C₁-C₆)-Alkylthiomorpholino-, (C₆-C₁₀)-Aryl-(C₁-C₆)-alkylthiomorpholino-, (C₁-C₉)-Heteroaryl-(C₁-C₆)-alkylthiomorpholino-, Piperidino-, (C₆-C₁₀)-Arylthiopiperidino-, (C₁-C₉)-Heteroarylthiopiperidino-, (C₁-C₆)-Alkylthiopiperidino-, (C₆-C₁₀)-Aryl-(C₁-C₆)-alkylthiopiperidino-, (C₁-C₉)-Heteroaryl-(C₁-C₆)-alkylthiopiperidino-, Pyrrolidino-, (C₆-C₁₀)-Arylthiopyrrolidino-, (C₁-C₉)-Heteroarylthiopyrrolidino-, (C₁-C₆)-Alkylthiopyrrolidino-, (C₆-C₁₀)-Aryl-(C₁-C₆)-alkylthiopyrrolidino-, (C₁-C₉)-Heteroaryl-(C₁-C₆)-alkylthiopyrrolidino-, und Z ausgewählt ist aus der Gruppe, enthaltend (C₁-C₆)-Alkyl-, (C₂-C₆)-Alkylenyl-, (C₂-C₆)-Alkinyl-, Amino, Sauerstoff, Thio, Sulfinyl, Sulfonyl, Halo-(C₁-C₆)-alkyl- und Hydroxy-(C₂-C₆)-alkyl-;
(e) zwei oder mehrere Reste R² bilden, sofern vicinal, gemeinsam einen oder mehrere weitere 4-, 5-, 6- oder 7-gliedrige Ringe, die ausgewählt sind aus der Gruppe, enthaltend Phenyl-, Naphthyl-, Furyl-, Thienyl-, Thiazolyl-, Pyrazolyl-, Isothiazolyl-, Oxazolyl-, Isoxazolyl-, Pyrrolyl-, Triazolyl-, Tetrazolyl-, Imidazolyl-, 1,3,5-Oxadiazolyl-, 1,2,4- Oxadiazolyl-, 1,2,3-Oxadiazolyl-, 1,3,5-Thiadiazolyl-, 1,2,3-Thiadiazolyl-, 1,2,4-Thiadiazolyl-, Pyridyl-, Pyrimidyl-, Pyrazinyl-, Pyridazinyl-, 1,2,4-Triazinyl-, 1,2,3-Triazinyl-, 1,3,5-Triazinyl-, Pyrazolo[3,4-b]pyridinyl-, Cinnolinyl-, Pteridinyl-, Purinyl-, 6,7-Dihydro-5H-[1]pyridinyl-, Benzo[b]thiphenyl-, 5,6,7,8-Tetrahydrochinolin-3-yl, Benzoxazolyl-, Benzothiazolyl-, Benzisothiazolyl-, Benzisoxazolyl-, Benzimidazolyl-, Thianaphthenyl-, Isothianaphthenyl-, Benzofuranyl-, Isobenzofuranyl-, Isoindolyl-, Indolyl-, Indolizinyl-, Indazolyl-, Isochinolyl-, Chinolyl-, Phthalazinyl-, Chinoxalinyl-, Chinazolinyl- und Benzoxazinyl-, wobei dieser Ring (diese Ringe) optional ein- oder mehrfach substituiert ist (sind) durch (C₁-C₆)-Alkyl-, (C₂-C₆)-Alkenyl-, (C₂-C₆)-Alkinyl-, Amino-, Halogen, Hydroxy-, Carboxy-, Thiol-, Nitro-, Cyano-, Sulfonyl- (sulfonic), Halo-(C₁-C₆)-Alkyl- und Hydroxy-(C₂-C₆)-alkyl; und
(f) zwei oder mehrere Reste R² bilden, sofern vicinal, gemeinsam einen oder mehrere weitere 3-, 4-, 5-, 6- oder 7-gliedrige Ringe, die ausgewählt sind aus den folgenden Gruppen:
(i) (C₃-C₁₀)-Cycloalkyl mit 0 - 2 Ungesättigtheitsgraden, ausgewählt aus der Gruppe, enthaltend Cyclopropyl-, Cyclobutyl-, Cyclopentyl-, Cyclohexyl-, Cycloheptyl-, Cyclopropenyl-, Cyclobutenyl-, Cyclopentenyl-, Cyclohexenyl-, Cycloheptenyl-, 1,3-Cyclobutadienyl-, 1,3-Cyclopentadienyl-, 1,3-Cyclohexadienyl-, 1,4-Cyclohexadienyl-, 1,3-Cycloheptadienyl-, 1,4-Cycloheptadienyl-, Bicyclo[3.2.1]octan-, Bicyclo[2.2.1]heptan, deren Norborn-2-en-ungesättigte Form, und dergleichen, wobei der Ring optional substituiert ist durch Hydroxy-, Halogen-, Amino-, Trifluormethyl-, Hydroxy-(C₂-C₆)-alkyl, (C₁-C₆)-Alkoxy-, (C₁-C₆)-Acyloxy-, (C₁-C₆)-Alkylamino-, ((C₁-C₆)-Alkyl)₂amino-, (C₁-C₆)-Acylamino-, Cyano-, Nitro-, Carboxy-, Thiol-, Sulfonyl-,(C₁-C₆)-Alkyl-, (C₂-C₆)-Alkenyl-, (C₂-C₆)-Alkinyl-, (C₁-C₆)-Acylamino-, Cyano-(C₁-C₆)-alkyl-, Trifluormethyl-(C₁-C₆)-alkyl-, (C₁-C₃)-Alkyl(difluormethylen)-(C₁-C₃)-alkyl-, Halo-(C₁-C₆)-alkyl- oder Nitro-(C₁-C₆)-alkyl-; und
(ii) (C₃-C₁₀)-Heterocycloalkyl-, ausgewählt aus der Gruppe, enthaltend Pyrrolidinyl-, Tetrahydrofuranyl-, Dihydrofuranyl-, Tetrahydropyranyl-, Pyranyl-, Thiopyranyl-, Aziridinyl-, Oxiranyl-, Methylendioxyl-, Isoxazolidinyl-, 1,3-Oxazolidin-3-yl-, Isothiazolidinyl-, 1,3-Thiazolidin-3-yl-, 1,2-Pyrazolidin-2-yl-, 1,3-Pyrazolidin-1-yl-, Piperidinyl-, Thiomorpholinyl-, 1,2-Tetrahydrothiazin-2-yl-, 1,3-Tetrahydrothiazin-3-yl-, Tetrahydrothiadiazinyl-, Morpholinyl-, 1,2-Tetrahydrodiazin-2-yl-, 1,3-Tetrahydrodiazin-1-yl-, Tetrahydroazepinyl-, Piperazinyl-, Chromenyl- oder Chromanyl-, wobei der genannte Ring optional substituiert ist durch Hydroxy-, Halogen-, Amino-, Trifluormethyl-, Hydroxy-(C₂-C₆)-alkyl, (C₁-C₆)-Alkoxy-, (C₁-C₆)-Acyloxy-, (C₁-C₆)-Alkylamino-, ((C₁-C₆)-Alkyl)₂amino-, (C₁-C₆)-Acylamino-, Cyano-, Nitro-, Carboxy-, Thiol-, Sulfonyl-, (C₁-C₆)-Alkyl-, (C₂-C₆)-Alkenyl-, (C₂-C₆)-Alkinyl-, (C₁-C₆)-Acylamino-, Cyano-(C₁-C₆)-alkyl-, Trifluormethyl-(C₁-C₆)-alkyl-, (C₁-C₃)-Alkyl-(difluormethylen)-(C₁-C₃)-alkyl-, Halo-(C₁-C₆)-alkyl- oder Nitro-(C₁-C₆)-alkyl-;
wobei jede der (C₁-C₆)-Alkyl-, (C₂-C₆)-Alkenyl-, (C₂-C₆)-Alkinyl-, (C₃-C₁₀)-Cycloalkyl- oder (C₃-C₁₀)-Heterocycloalkyl-Gruppen, die jeweils einen der 1 - 4 optionalen Substituenten von R² darstellen oder jeweils einen Teil der 1 - 4 optionalen Substituenten von R² umfassen, ihrerseits jeweils optional substituiert sind durch Deuterium, Hydroxy-, Amino-, Trifluormethyl-, Cyano-, Nitro-, Carboxy-, (C₁-C₄)-Alkoxy-, (C₁-C₆)-Acyloxy-, (C₁-C₆)-Alkylamino-, ((C₁-C₆)-Alkyl)₂-amino-, (C₁-C₆)-Alkyl-, (C₂-C₆)-Alkenyl-, (C₂-C₆)-Alkinyl-, (C₁-C₆)-Acylamino-, (C₃-C₁₀)-Cycloalkyl-, (C₃-C₁₀)-Heterocycloalkyl-, Cyano-(C₁-C₆)-alkyl-, Trifluormethyl-(C₁-C₆)-alkyl-, Nitro-(C₁-C₆)-alkyl- und (C₁-C₆)-Acylamino; und
R³ einen oder mehrere optionale Reste an einem Ring-Kohlenstoffatom, einschließlich X, wenn X für -CH₂- steht, darstellt, wobei R³ ausgewählt ist aus (C₁-C₆)-Alkyl- und Trihalo-(C₁-C₆)-alkyl-, bevorzugt Trifluormethyl-, Deuterium oder Fluor.

2. Verbindung nach Anspruch 1, deren Strukturkomponente 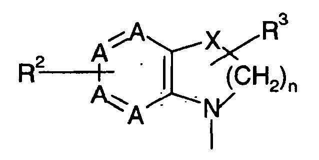 1,2,3,4-Tetrahydrochinolin, 1,2,3,4-Tetrahydrochinoxalin, 3,4-Dihydro-1 H-chinoxalin-2-on, 3,4-Dihydro-2H-benzo[1,4]oxazin, 2,3-Dihydro-1 H-indol oder 3,4-Dihydro-2H-benzo[1,4]thiazin darstellt.

3. Verbindung nach Anspruch 2, enthaltend eine Struktur, die ausgewählt ist aus der Gruppe, enthaltend 6-Methoxy-1,2,3,4-tetrahydrochinolin; 4-Methyl-1,2,3,4-tetrahydrochinolin; 7-(Trifluormethyl)-1,2,3,4-tetrahydrochinolin; 8-Methyl-1,2,3,4-tetrahydrochinolin; 6-Hydroxy-1,2,3,4-tetrahydrochinolin; 8-Chlor-1,2,3,4-tetrahydrochinolin; 7-Chlor-1,2,3,4-tetrahydrochinolin; 6-Benzyloxy-7-methoxy-1,2,3,4-tetrahydrochinolin; 6,7-Dimethyl-1,2,3,4-tetrahydrochinoxalin; 1,2,3,4-Tetrahydrochinoxalin; 1-Phenylsulfonyl-1,2,3,4-tetrahydrochinoxalin; 6-Methyl-1,2,3,4-tetrahydrochinoxalin; 3,4-Dihydro-2H-benzo[1,4]oxazin; 5-Fluor-, 2,3-dihydro-1H-indol und 3,3-Dimethyl-2,3-dihydro-1H-indol.

4. Verbindung nach Anspruch 1, worin R¹ eine (C₁-C₉)-Heteroarylgruppe darstellt, die ausgewählt ist aus der Gruppe Pyridyl-, Indazolyl-, Indolyl-, 1,3-Dihydro-Benzoimidazol-2-on, Thienyl-, Oxazoyl-, 2H-Pyrazolyl-, 1 H-Pyrazolyl-, Isooxazoyl-, Thiazolyl- und Isothiazolyl-; und optional ein- oder mehrfach unabhängig substituiert ist durch Hydroxy-, Halogen-, Amino-, (C₁-C₆)-Alkyl-, (C₁-C₆)-Alkoxy-, Trihalomethyl-, (C₁-C₆)-Alkinyl-, (C₁-C₆)-Alkylamino-, ((C₁-C₆)₂)-Dialkylamino-, Carboxy-, (C₁-C₆)-Alkoxycarbonyl-, (C₁-C₆)-Acyloxy- und (C₁-C₆)-Acylamino-.

5. Verbindung nach Anspruch 1, worin R¹ für Phenyl steht, das optional ein- bis fünffach unabhängig substituiert ist durch Hydroxy-, Halogen-, Amino-, (C₁-C₆)-Alkyl-, (C₁-C₆)-Alkoxy-, Trihalo-(C₁-C₆)-alkyl-, bevorzugt Trifluormethyl, (C₁-C₆)-Alkinyl-, (C₁-C₆)-Alkylamino-, ((C₁-C₆)₂)-Dialkylamino-, Carboxy-, (C₁-C₆)-Alkoxycarbonyl-, (C₁-C₆)-Acyloxy- und (C₁-C₆)-Acylamino-.

6. Verbindung nach Anspruch 1, worin R¹ ausgewählt ist aus der Gruppe, enthaltend 3,4,5-Trimethoxyphenyl-, 2,3-Dimethyl-1 H-indol-5-yl, 3,4-Dihydro-2H-chinolin-1-yl und 6-Morpholin-4-yl-pyridin-3-yl.

7. Verbindung nach Anspruch 1, worin einer oder mehrere Substituenten R² ausgewählt sind aus den folgenden Gruppen:
(a) (C₁-C₆)-Alkyl-, (C₁-C₆)-Alkinyl-, (C₁-C₆)-Alkoxy-, Trihalo-(C₁-C₆)-alkyl-, bevorzugt Trifluormethyl-, (C₁-C₆)-Alkylamino-, ((C₁-C₆)₂)-Dialkylamino-, Amino-, Cyano- und Halogen-, und
(b) Benzyloxy-, Phenylsulfonyl-, Phenylaminocarbonyl-, (C₁-C₉)-Heteroarylsulfonyl- und (C₁-C₉)-Heteroarylaminocarbonyl-, optional ein- oder mehrfach substituiert durch (C₁-C₆)-Alkyl-, (C₂-C₆)-Alkinyl-, Trihalo-(C₁-C₆)-alkyl, bevorzugt Trifluormethyl-, (C₁-C₆)-Alkoxy-, (C₁-C₆)-Alkylamino-, ((C₁-C₆)₂)-Alkylamino- und Halogen.

8. Verbindung nach Anspruch 1, worin einer oder mehrere der Substituenten R³ ausgewählt ist aus (C₁-C₆)-Alkyl-, Trihalo-(C₁-C₆)-alkyl-, Deuterium und Fluor.

9. Verbindung nach Anspruch 8, worin R³ für Trifluormethyl steht.

10. Verbindung nach Anspruch 1, ausgewählt aus
(a) 1-[(2-Anilino)-4-pyrimidinyl]-6-methyl-1,2,3,4-tetrahydrochinolin;
(b) 1-[2-[(4-Bromphenyl)amino]-4-pyrimidinyl]-6-methyl-1,2,3,4-tetrahydrochinolin;
(c) 1-[2-[(4-Methoxyphenyl)amino]-4-pyrimidinyl]-6-methyl-1,2,3,4-tetrahydrochinolin;
(d) 1-[2-[(1 H-Indazol-5-yl)]-4-pyrimidinyl]-6-methyl-1,2,3,4-tetrahydrochinolin;
(e) 1-[2-[(4-Phenoxyphenyl)amino]-4-pyrimidinyl]-6-methyl-1,2,3,4-tetrahydrochinolin;
(f) 1-[2-[(3,4-Dimethoxyphenyl)amino]-4-pyrimidinyl]-6-methyl-1,2,3,4-tetrahydrochinolin;
(g) 1-[2-[(3,4,5-Trimethoxyphenyl)amino]-4-pyrimidinyl]-6-methyl-1,2,3,4-tetrahydrochinolin;
(h) 1-[2-[(4,N-Phenylaminophenyl)amino]-4-pyrimidinyl]-6-methyl-1,2,3,4-tetrahydrochinolin;
(i) [4-(6-Methyl-3,4-dihydro-2H-chinolin-1-yl)-pyrimidin-2-yl]-(6-morpholin-4-yl-pyridin-3-yl)-amin;
(j) 5-[4-(6-Methyl-3,4-dihydro-2H-chinolin-1-yl)-pyrimidin-2-ylamino]-1,3-dihydro-benzoimidazol-2-on;
(k) (2,3-Dimethyl-1 H-indol-5-yl)-[4-(6-methyl-3,4-dihydro-2H-chinolin-1-yl)-pyrimidin-2-yl]-amin;
(l) [4-(6-Methyl-3,4-dihydro-2H-chinolin-1-yl)-pyrimidin-2-yl]-(2-methyl-2H-pyrazol-3-yl)-amin;
(m) (6-Methoxy-pyridin-3-yl)-[4-(6-methyl-3,4-dihydro-2H-chinolin-1-yl)-pyrimidin-2-yl]-amin;
(n) (4-Fluor-3-methyl-phenyl)-[4-(6-methyl-3,4-dihydro-2H-chinolin-1-yl)-pyrimidin-2-yl]-amin;
(o) (5-Cyclopropyl-2H-pyrazol-3-yl)-[4-(6-methyl-3,4-dihydro-2H-chinolin-1-yl)-pyrimidin-2-yl]-amin;
(p) 4-Benzyl-N-3-[4-(6-methyl-3,4-dihydro-2H-chinolin-1-yl)-pyrimidin-2-yl]-1 H-pyrazol-3,5-diamin;
(q) [4-(6-Methyl-3,4-dihydro-2H-chinolin-1-yl)-pyrimidin-2-yl]-(4-methyl-thiazol-2-yl)-amin und
(r) [4-(6-Methyl-3,4-dihydro-2H-chinolin-1-yl)-pyrimidin-2-yl]-(5-methyl-1 H-pyrazol-3-yl)-amin.

11. Verbindung nach Anspruch 1, ausgewählt aus
(a) [4-(3,4-Dihydro-2H-chinolin-1-yl)-pyrimidin-2-yl]-(6-pyrrolidin-1-yl-pyridin-3-yl)-amin;
(b) (1-Cyclopentyl-1H-indol-6-yl)-[4-(6-methyl-3,4-dihydro-2H-chinolin-1-yl)-pyrimidin-2-yl]-amin;
(c) [4-(6-Methyl-3,4-dihydro-2H-chinolin-1-yl)-pyrimidin-2-yl]-oxazol-4-yl-amin;
(d) (3,4-Dichlor-phenyl)-[4-(6-methyl-3,4-dihydro-2H-chinolin-1-yl)-pyrimidin-2-yl]-amin; und
(e) [4-(3,4-Dihydro-1H-chinolin-1-yl)-pyrimidin-2-yl]-isothiazol-3-yl-amin.

12. Verbindung nach Anspruch 1, ausgewählt aus
(a) 2-({5-[4-(2,3-Dihydro-benzo[1,4]oxazin-4-yl)-pyrimidin-2-ylamino]-pyridin-2-yl}-methyl-amino)-ethanol;
(b) N-{5-[4-(3-Oxo-3,4-dihydro-2H-chinoxalin-1-yl)-pyrimidin-2-ylamino]-pyridin-2-yl}-acetamid;
(c) 3-Chlor-N-[4-(4-methyl-3-oxo-3,4-dihydro-2H-chinoxalin-1-yl)-pyrimidin-2-yl]-benzamid;
(d) [4-(2,3-Dihydro-benzo[1,4]thiazin-4-yl)-pyrimidin-2-yl]-oxazol-4-yl-amin;
(e) N-[4-(5-Fluor-2,3-dihydro-indol-1-yl)-pyrimidin-2-yl]-3-methoxy-benzolsulfonamid;
(f) [4-(5,6-Dihydro-pyrrolo[2,3-d]pyrimidin-7-yl)-pyrimidin-2-yl]-(2-trifluormethyl-phenyl)-amin;
(g) 6-Methoxy-1-[2-(pyridazin-3-ylamino)-pyrimidin-4-yl]-2,3-dihydro-1H-chinolin-4-on;
(h) 2-{5-[4-(3,4-Dihydro-2H-chinoxalin-1-yl)-pyrimidin-2-ylamino]-indol-1-yl}-ethanol;
(i) (2H-Pyrazol-3-yl)-[4-(7-trifluormethyl-3,4-dihydro-2H-chinolin-1-yl)-pyrimidin-2-yl]-amin;
(j) 1-[4-(3,4-Dihydro-2H-[1,5]naphthyridin-1-yl)-pyrimidin-2-yl]-3-ethyl-harnstoff;
(k) 1-[4-(2,3-Dihydro-benzo[1,4]oxazin-4-yl)-pyrimidin-2-yl]-3-(2-ethoxy-ethyl)-harnstoff;
(l) [4-(3,3-Dimethyl-2,3-dihydro-indol-1-yl)-pyrimidin-2-yl]-carbamidsäure-tert.-butylester;
(m) 3-Cyano-N-[4-(7-methoxy-2,3,4,5-tetrahydro-benzo[b]azepin-1-yl)-pyrimidin-2-yl]-benzamid;
(n) Isoxazol-4-yl-[4-(2,3,4,5-tetrahydro-benzo[b][1,4]diazepin-1-yl)-pyrimidin-2-yl]-amin;
(o) (3,4-Dichlor-phenyl)-[4-(3,4-dihydro-2H-benzo[b][1,4]thiazepin-5-yl)-pyrimidin-2-yl]-amin;
(p) (6-Aziridin-1-yl-pyridin-3-yl)-[4-(5-methansulfonyl-2,3-dihydro-indol-1-yl)-pyrimidin-2-yl]-amin;
(q) N²-Cyclopropyl-N⁵-[4-(6-fluor-3,4-dihydro-2H-chinolin-1-yl)-pyrimidin-2-yl]-pyridin-2,5-diamin; und
(r) Benzo[1,3]dioxol-5-carbonsäure-[4-(6-fluor-3,4-dihydro-2H-chinolin-1-yl)-pyrimidin-2-yl]-amid.

13. Verbindung nach Anspruch 1, worin X für Methylen steht.

14. Verbindung nach Anspruch 1, worin die Struktur 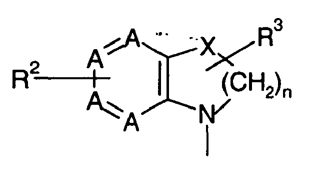 eine Gruppe umfasst, die ausgewählt ist aus 2,3-Dihydro-1H-pyrrolo[2,3-b]-pyridin; 2,3-Dihydro-1H-pyrrolo[2,3-c]-pyridin; 2,3-Dihydro-1H-pyrrolo[3,2-c]-pyridin; 2,3-Dihydro-1H-pyrrolo[3,2-b]-pyridin; 6,7-Dihydro-5H-pyrrolo[3,2-d]-pyrimidin; 6,7-Dihydro-5H-pyrrolo[3,2-d][1,2,3]triazin; 6,7-Dihydro-5H-pyrrolo[2,3-d][1,2,3]-triazin; 1,4,5,7-Tetraaza-indan; 1,4,6,7-Tetraaza-indan; 6,7-Dihydro-5H-pyrrolo-[2,3-c]pyridazin; 2,3-Dihydro-1H-pyrrolo[2,3-d]pyridazin; 6,7-Dihydro-5H-pyrrolo-[3,2-c]-pyridazin; 6,7-Dihydro-5H-pyrrolo[2,3-b]pyrazin; 6,7-Dihydro-5H-pyrimido[4,5-b][1,4]oxazin; 5,6,7,8-Tetrahydro-pteridin; 1,2,3,4-Tetrahydro-pyrido[2,3-b]pyrazin; 1,2,3,4-Tetrahydro-pyrido[3,4-b]pyrazin; 1,2,3,4-Tetrahydro-pyrido-[3,4-b]pyrazin; 1,2,3,4-Tetrahydro-pyrido[2,3-b]pyrazin; 5,6,7,8-Tetrahydro-pyrazino[2,3-c]pyridazin; 5,6,7,8-Tetrahydro-pteridin; 1,2,3,4-Tetrahydro-pyrazino-[2,3-d]pyridazin; 5,6,7,8-Tetrahydro-pyrazino[2,3-c]pyridazin; 1,2,3,4-Tetrahydro-pyrazino[2,3-b]pyrazin; 5,6,7,8-Tetrahydro-pyrazino[2,3-e][1,2,4]triazin; 5,6,7,8-Tetrahydro-pyrazino[2,3-e][1,2,4]triazin; 5,6,7,8-Tetrahydro-pyrazino[2,3-d][1,2,3]triazin; 5,6,7,8-Tetrahydro-pyrazino[2,3-d][1,2,3]triazin; 2,3-Dihydro-1H-4-oxa-1,5-diaza-naphthalen; 2,3-Dihydro-1H-4-oxa-1,6-diaza-naphthalen; 3,4-Dihydro-2H-1-oxa-4,6-diaza-naphthalen; 3,4-Dihydro-2H-1-oxa-4,5-diaza-naphthalen; 7,8-Dihydro-6H-5-oxa-1,2,8-triaza-naphthalen; 3,4-Dihydro-2H-1-oxa-4,6,7-triaza-naphthalen; 6,7-Dihydro-5H-8-oxa-1,2,5-triaza-naphthalen; 3,4-Dihydro-2H-1-oxa-4,5,8-triaza-naphthalen; 7,8-Dihydro-6H-pyrimido[5,4-b][1,4]oxazin; 6,7-Dihydro-5H-pyrimido[4,5-b][1,4]oxazin; 6,7-Dihydro-5H-8-oxa-1,2,3,5-tetraaza-naphthalen; 6,7-Dihydro-5H-8-oxa-1,2,4,5-tetraaza-naphthalen; 7,8-Dihydro-6H-5-oxa-1,2,3,8-tetraaza-naphthalen; 6,7-Dihydro-5H-8-oxa-1,2,4,5-tetraaza-naphthalen; 2,3-Dihydro-1H-pyrido[2,3-b][1,4]thiazin; 2,3-Dihydro-1H-4-thia-1,6-diaza-naphthalen; 3,4-Dihydro-2H-1-thia-4,6-diaza-naphthalen; 3,4-Dihydro-2H-pyrido[3,2-b][1,4]thiazin; 7,8-Dihydro-6H-5-thia-1,2,8-triaza-naphthalen; 3,4-Dihydro-2H-1-thia-4,6,7-triaza-naphthalen; 6,7-Dihydro-5H-8-thia-1,2,5-triaza-naphthalen; 6,7-Dihydro-5H-pyrimido[4,5-b][1,4]thiazin; 7,8-Dihydro-6H-pyrimido[5,4-b][1,4]thiazin; 3,4-Dihydro-2H-1-thia-4,5,8-triaza-naphthalen; 6,7-Dihydro-5H-8-thia-1,2,4,5-tetraaza-naphthalen; 7,8-Dihydro-6H-5-thia-1,2,4,8-tetraaza-naphthalen; 7,8-Dihydro-6H-5-thia-1,2,3,8-tetraaza-naphthalen; 6,7-Dihydro-5H-8-thia-1,2,3,5-tetraaza-naphthalen; 5,6,7,8-Tetrahydro-pyrido[3,2-d]pyrimidin; 1,2,3,4-Tetrahydro-pyrido[2,3-d]pyridazin; 5,6,7,8-Tetrahydro-pyrido[2,3-b]pyrazin; 5,6,7,8- Tetrahydro-pyrido[3,2-e][1,2,4]triazin; 5,6,7,8-Tetrahydro-pyrido[2,3-e][1,2,4]triazin; 5,6,7,8-Tetrahydro-pyrido[3,2-d][1,2,3]triazin; und 5,6,7,8-Tetrahydro-pyrido[2,3-d][1,2,3]triazin.

15. Pharmazeutische Komposition, enthaltend eine wirksame Menge einer Verbindung nach Anspruch 1 und einen pharmazeutischen Träger.

16. Pharmazeutische Komposition nach Anspruch 15 zur Behandlung oder Prävention eines Zustands bei Säugetieren, bei dem durch Modulation eines T-Zell-vermittelten Zellprozesses ein therapeutischer Nutzen erzielt wird.

17. Pharmazeutische Komposition nach Anspruch 15 zur Behandlung oder Prävention der Transplantat-Abstoßung bei Säugetieren.

18. Pharmazeutische Komposition nach Anspruch 15 zur Behandlung oder Prävention von Autoimmunkrankheiten bei Säugetieren.

19. Pharmazeutische Komposition nach Anspruch 15 zur Behandlung oder Prävention von Entzündungskrankheiten bei Säugetieren.

20. Pharmazeutische Komposition nach Anspruch 15 zur Behandlung oder Prävention von Allergien bei Säugetieren.

21. Pharmazeutische Komposition nach Anspruch 15 zur Behandlung von T-Zell-Leukämie oder T-Zell-Lymphom bei Säugetieren.

22. Pharmazeutische Komposition nach Anspruch 15 zur Behandlung einer Krankheit bei Säugetieren, bei der die Behandlung durch Hemmung der Aktivierung von T-Zellen oder durch Hemmung der Folgen einer solchen Aktivierung durchgeführt werden kann.

23. Pharmazeutische Komposition nach Anspruch 15, weiter enthaltend einen zusätzlichen Wirkstoff, der das Immunsystem bei Säugetieren moduliert oder einen entzündungshemmenden Wirkstoff darstellt.

24. Pharmazeutische Komposition nach Anspruch 15, die geeignet ist zur Behandlung einer Störung oder eines Zustands bei Säugetieren, die ausgewählt aus Asthma, Heuschnupfen, Nesselsucht, Säuglingsekzem, atopischer Dermatitis und anderen allergischen Krankheiten, bei denen Antikörper-vermittelte (intermediäre) Hypersensibilitätsreaktionen eine Rolle spielen, Transplantat-Abstoßung, Psoriasis, Colitis ulcerosa, Morbus Crohn, Lupus, Multiple Sklerose, rheumatische Arthritis, Diabetes Typ I, Autoimmunthyreopathien, T-Zell-Malignitäten, einschließlich T-Zell-Leukämie und T-Zell-Lymphom, und Alzheimer.

25. Pharmazeutische Komposition nach Anspruch 15, die geeignet ist zur Hemmung von Tyrosinkinase Lck bei Säugetieren.

26. Pharmazeutische Komposition nach Anspruch 15, worin die Verbindung als pharmazeutisch akzeptables Salz, Solvat oder Hydrat vorliegt.

27. Verbindung, Salz, Solvat oder Hydrat nach einem der Ansprüche 1 - 14 zur Verwendung in der Medizin.

28. Verwendung einer Verbindung, eines Salzes, Solvats oder Hydrats nach einem der Ansprüche 1 - 14 zur Herstellung eines Arzneimittels zur Behandlung eines Zustands bei Säugetieren, bei dem durch Modulation eines T-Zell-vermittelten Zellprozesses ein therapeutischer Nutzen erzielt wird, zur Behandlung der Abstoßung von Transplantaten bei Säugetieren, von Autoimmunkrankheiten bei Säugetieren, von Entzündungskrankheiten bei Säugetieren, von Allergien bei Säugetieren, von T-Zell-Leukämie oder T-Zell-Lymphom bei Säugetieren, einer Krankheit bei Säugetieren, bei der die Behandlung durch Hemmung der Aktivierung von T-Zellen oder durch Hemmung der Folgen einer solchen Aktivierung durchgeführt werden kann, von Asthma, Heuschnupfen, Nesselsucht, Säuglingsekzem, atopischer Dermatitis und anderen allergischen Krankheiten, bei denen Antikörper-vermittelte (intermediäre) Hypersensibilitätsreaktionen eine Rolle spielen, Transplantat-Abstoßung, Psoriasis, Colitis ulcerosa, Morbus Crohn, Lupus, Multiple Sklerose, rheumatische Arthritis, Diabetes Typ I, Autoimmunthyreopathien, T-Zell-Malignitäten, einschließlich T-Zell-Leukämie und T-Zell-Lymphom, von Alzheimer.

## Revendications

1. Composé répondant à la formule : 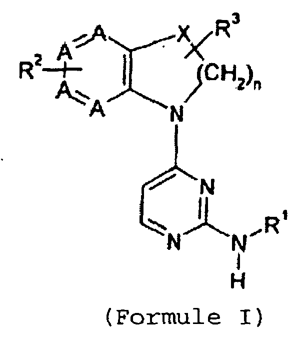
ou un de ses sels, produits de solvatation ou hydrates pharmaceutiquement acceptables ; formule dans laquelle :
A, chaque fois qu'il apparaît, est choisi indépendamment entre un groupe CH et N ;
X est choisi dans le groupe consistant en des groupes -CH₂-, -O-, -NH-, (alkyle en C₁ à C₆) amino-, (alkyle en C₁ à C₆) -aminocarbonylamino-, (alkyle en C₁ à C₆) -carbonylamino-, (alkyle en C₁ à C₆) -sulfonylamino, phénylsulfonylamino-, carbonyle, -NH-C (O) -, -N- (alkyle en C₁ à C₆) -C (O) -, -S(O)_{y}-, dans lequel y est égal à 0, 1 ou 2 ; et
n dans le groupe -(CH₂)ₙ- est égal à 1, 2 ou 3 ;
R¹ est choisi dans le groupe consistant en les groupes aryl- en C₆ à C₁₀, hétéroaryl- en C₁ à C₉, (aryle en C₆ à C₁₀) - (aryle en C₆ à C₁₀) -, (aryle en C₆ à C₁₀) - (hétéroaryle en C₁ à C₉) -, (hétéroaryle en C₁ à C₉) - (hétéroaryle en C₁ à C₉) -, (hétéroaryle en C₁ à C₉) - (aryle en C₆ à C₁₀) -, (aryle en C₆ à C₁₀) -sulfinyle, (aryle en C₆ à C₁₀) - (aryle en C₆ à C₁₀) -sulfinyl-, (hétéroaryle en C₁ à C₉) - (aryle en C₆ à C₁₀) - sulfinyl-, (aryle en C₆ à C₁₀) -sulfonyl-, (aryle en C₆ à C₁₀) - (aryle en C₆ à C₁₀) -sulfonyl-, (hétéroaryle en C₁ à C₉) - (aryle en C₆ à C₁₀) -sulfonyl-, (hétéroaryle en C₁ à C₉) sulfinyl-, (hétéroaryle en C₁ à C₉) - (hétéroaryle en C₁ à C₉) sulfinyl-, (aryle en C₆ à C₁₀) - (hétéroaryle en C₁ à C₉) sulfinyl-, (hétéroaryle en C₁ à C₉) -sulfonyl-, (hétéroaryle en C₁ à C₉) - (hétéroaryle en C₁ à C₉) -sulfonyl-, (aryle en C₆ à C₁₀) - (hétéroaryle en C₁ à C₉) -sulfonyl-, (R⁴) - sulfinyl-, (R⁴) -sulfonyl-, (aryle en C₆ à C₁₀) - (R⁴)-sulfinyl-, (aryle en C₆ à C₁₀) - (aryle en C₆ à C₁₀) - (R⁴)-sulfinyl-, (hétéroaryle en C₁ à C₉) - (aryle en C₆ à C₁₀) - (R⁴) - sulfinyl-, (aryle en C₆ à C₁₀)- (R⁴)-sulfonyl-, (aryle en C₆ à C₁₀) - (aryle en C₆ à C₁₀) (R⁴) -sulfonyl-, (hétéroaryle en C₁ à C₉) - (aryle en C₆ à C₁₀) (R⁴) -sulfonyl-, (hétéroaryle en C₁ à C₉) - (R⁴) -sulfinyl-, (aryle en C₆ à C₁₀) - (hétéroaryle en C₁ à C₉) - (R⁴) -sulfinyl-, (hétéroaryle en C₁ à C₉) - (hétéroaryle en C₁ à C₉) (R⁴) -sulfinyl-, (hétéroaryle en C₁ à C₉) (R⁴) -sulfonyl-, (aryle en C₆ à C₁₀) - (hétéroaryle en C₁ à C₉) - (R⁴) -sulfonyl-, (hétéroaryle en C₁ à C₉) - (hétéroaryle en C₁ à C₉) - (R⁴) - sulfonyl-, (aryle en C₆ à C₁₀) -aminocarbonyl-, (aryle en C₆ à C₁₀) - (aryle en C₆ à C₁₀) -aminocarbonyl-, (hétéroaryle en C₁ à C₉) - (aryle en C₆ à C₁₀) -aminocarbonyl-, (hétéroaryle en C₁ à C₉) -aminocarbonyl-, (aryle en C₆ à C₁₀) - (hétéroaryle en C₁ à C₉) -aminocarbonyl-, (hétéroaryle en C₁ à C₉) - (hétéroaryle en C₁ à C₉) -aminocarbonyl-, (aryle en C₆ à C₁₀) -carbonyl-, (aryle en C₆ à C₁₀) - (aryle en C₆ à C₁₀) -carbonyl-, (hétéroaryle en C₁ à C₉) - (aryle en C₆ à C₁₀)-carbonyl-, (hétéroaryle en C₁ à C₉) -carbonyl-, (aryle en C₆ à C₁₀) - (hétéroaryle en C₁ à C₉) -carbonyl-, (hétéroaryle en C₁ à C₉) - (hétéroaryle en C₁ à C₉) -carbonyl-, (aryle en C₆ à C₁₀) - oxycarbonyl - , (aryle en C₆ à C₁₀)-(aryle en C₆ à C₁₀)-oxycarbonyl-, (hétéroaryle en C₁ à C₉) - (aryle en C₆ à C₁₀) - oxycarbonyl-, (hétéroaryle en C₁ à C₉) -oxycarbonyl-, (aryle en C₆ à C₁₀) - (hétéroaryle en C₁ à C₉) -oxycarbonyl-, (hétéroaryle en C₁ à C₉) - (hétéroaryle en C₁ à C₉) - oxycarbonyl - , (R⁴) carbonyl - , (R⁴) oxycarbonyl - , (R⁴) aminocarbonyl - , (aryle en C₆ à C₁₀) - (R⁴) carbonyl - , (aryle en C₆ à C₁₀) (R⁴)oxycarbonyl-, (aryle en C₆ à C₁₀) - (R⁴) aminocarbonyl-, (hétéroaryle en C₁ à C₉) - (R⁴) -carbonyl-, (hétéroaryle en C₁ à C₉) (R⁴) oxycarbonyl- et (hétéroaryle en C₁ à C₉)(R⁴)-aminocarbonyl- ;
dans lesquels R⁴ est choisi dans les groupes consistant en :
(a) un groupe alkyl- en C₁ à C₆, alcényl- en C₂ à C₆ ou alcynyl- en C₂ à C₆, ces groupes alkyl-, alcényl- et alcynyl- étant facultativement substitués avec des substituants hydroxy, halogéno, amino, trifluorométhyle, hydroxyalkyl- en C₂ à C₆, alkoxy- en C₁ à C₆, (acyle en C₁ à C₆)-oxy-, (alkyle en C₁ à C₆) -amino-, (alkyle en C₁ à C₆)₂-amino-, (acyle en C₁ à C₆)-amino-, cyano, nitro, alkyl- en C₁ à C₆, alcényl- en C₂ à C₆, alcynyl- en C₂ à C₆, (acyle en C₁ à C₆) -amino-, cyano (alkyle en C₁ à C₆) -, trifluorométhyl (alkyle en C₁ à C₆) -, (alkyle en C₁ à C₃) - (difluorométhylène)-(alkyle en C₁ à C₃) - ou nitro(alkyle en C₁ à C₆) - ;
(b) un groupe cycloalkyl- en C₃ à C₁₀, ledit groupe cycloalkyl- étant facultativement substitué avec des substituants hydroxy, halogéno, amino, trifluorométhyle, hydroxyalkyl- en C₂ à C₆, alkoxy- en C₁ à C₆, (acyle en C₁ à C₆) -oxy-, (alkyle en C₁ à C₆) -amino-, (alkyle en C₁ à C₆)₂-amino-, (acyle en C₁ à C₆) -amino-, cyano, nitro, alkyl- en C₁ à C₆, alcényl- en C₂ à C₆, alcynyl- en C₂ à C₆, (acyle en C₁ à C₆) -amino-, cyano(alkyle en C₁ à C₆) -, trifluorométhyl (alkyle en C₁ à C₆) -, (alkyle en C₁ à C₃) - (difluorométhylène) - (alkyle en C₁ à C₃) - ou nitro(alkyle en C₁ à C₆) - ; ou bien
(c) un groupe hétérocycloalkyl- en C₃ à C₁₀, ledit groupe hétérocycloalkyl- étant facultativement substitué avec des substituants hydroxy, halogéno, amino, trifluorométhyle, hydroxyalkyl- en C₂ à C₆, alkoxy- en C₁ à C₆, (acyle en C₁ à C₆) -oxy-, (alkyle en C₁ à C₆) amino-, (alkyle en C₁ à C₆)₂amino-, (acyle en C₁ à C₆) amino-, cyano, nitro, alkyl- en C₁ à C₆, alcényl- en C₂ à C₆, alcynyl- en C₂ à C₆, (acyle en C₁ à C₆) amino-, cyano(alkyle en C₁ à C₆) -, trifluorométhyl- (alkyle en C₁ à C₆) -, (alkyle en C₁ à C₃) - difluorométhylène-(alkyle en C₁ à C₃)- ou nitro(alkyle en C₁ à C₆) - ; et n'importe lesquels desdits groupes aryl- en C₆ à C₁₀ ou hétéroaryl- en C₁ à C₉ de R¹ pouvant être facultativement substitués avec 1 à 5 groupes choisis entre :
(a) deutérium, hydroxy, halogéno, amino, trifluorométhyle, carboxy, alkoxy- en C₁ à C₆, (acyle en C₁ à C₆) -oxy-, (alkyle en C₁ à C₆) -amino-, (alkyle en C₁ à C₆)₂-amino-, (acyle en C₁ à C₆)-amino-, cyano, nitro, alkyl- en C₁ à C₆, alcényl- en C₂ à C₆, alcynyl- en C₂ à C₆, (acyle en C₁ à C₆) -amino-, cyano (alkyle en C₁ à C₆)-, trifluorométhyl-(alkyle en C₁ à C₆) - ou nitro(alkyle en C₁ à C₆) -, (alkyle en C₁ à C₃) -difluorométhylène- (alkyle en C₁ à C₃) -, (acyle en C₁ à C₆) -amino- (alkyle en C₁ à C₆) -, (alkoxy en C₁ à C₆) - (acyle en C₁ à C₆) amino- , amino- (acyle en C₁ à C₆) - , amino-(acyle en C₁ à C₆) - (alkyle en C₁ à C₆) -, (alkyle en C₁ à C₆) - amino- (acyle en C₁ à C₆) -, (alkyle en C₁ à C₆)₂-amino-acyle en C₁ à C₆)-, (cycloalkyle en C₃ à C₁₀) - (alkyle en C₁ à C₆) -, (acyle en C₁ à C₆) -oxy- (alkyle en C₁ à C₆) -, (alkoxy en C₂ à C₆) - (alkyle en C₁ à C₆) -, pipérazinyl- (alkyle en C₁ à C₆) -, (acyle en C₁ à C₆) -amino- (alkyle en C₁ à C₆) -, (aryle en C₆ à C₁₀) - (alkoxy en C₁ à C₆) - (alkyle en C₁ à C₆) -, (hétéroaryle en C₅ à C₉) - (alkoxy en C₁ à C₆) - (alkyle en C₁ à C₆) -, (alkyle en C₁ à C₆) -thio- (alkyle en C₁ à C₆) -, (aryle en C₆ à C₁₀) - thio (alkyle en C₁ à C₆) -, (alkyle en C₁ à C₆) -sulfinyl-(alkyle en C₁ à C₆) -, (aryle en C₆ à C₁₀) -sulfinyl- (alkyle en C₁ à C₆) -, (alkyle en C₁ à C₆) -sulfonyl- (alkyle en C₁ à C₆) -, (aryle en C₆ à C₁₀) -sulfonyl- (alkyle en C₁ à C₆) -, amino (alkyle en C₁ à C₆) -, (alkyle en C₁ à C₆) -amino- (alkyle en C₁ à C₆) -, (alkyle en C₁ à C₆) -difluorométhylène-, (alkyle en C₁ à C₃) -difluorométhylène- (alkyle en C₁ à C₃) -, (alkoxy en C₁ à C₆) - (acyle en C₁ à C₆) -, (alkyle en C₁ à C₆) - amino- (acyle en C₁ à C₆) -, (alkyle en C₁ à C₆)₂amino- (acyle en C₁ à C₆) -, aryl- en C₆ à C₁₀, hétéroaryl- en C₁ à C₉, (aryle en C₆ à C₁₀) - (alkyle en C₁ à C₆) - , (hétéroaryle en C₁ à C₉) - (alkyle en C₁ à C₆) -, (aryle en C₆ à C₁₀) - (aryle en C₆ à C₁₀)-, (aryle en C₆ à C₁₀) - (aryle en C₆ à C₁₀) - (alkyle en C₁ à C₆) -, cycloalkyl- en C₃ à C₁₀, (cycloalkyle en C₃ à C₆) - (alkyle en C₁ à C₆) -, hétérocycloalkyl- en C₃ à C₁₀, (hétérocycloalkyle en C₃ à C₁₀) - (alkyle en C₁ à C₆) -, hydroxyalkyl- en C₂ à C₆, (acyle en C₁ à C₆) -oxy- (alkyle en C₂ à C₆) -, (alkoxy en C₁ à C₆) - (alkyle en C₂ à C₆) -, pipérazinyl- (alkyle en C₁ à C₆) -, (acyle en C₁ à C₆) -amino-(alkyle en C₁ à C₆) -, (aryle en C₆ à C₁₀) - (alkoxy en C₁ à C₆) - (alkyle en C₁ à C₆) -, (hétéroaryle en C₁ à C₉) - (alkoxy en C₁ à C₆) - (alkyle en C₁ à C₆) -, (alkyle en C₁ à C₆) -thio-(alkyle en C₁ à C₆) -, (aryle en C₆ à C₁₀) -thio- (alkyle en C₁ à C₆) -, (alkyle en C₁ à C₆) -sulfinyl- (alkyle en C₁ à C₆) -, (aryle en C₆ à C₁₀) -sulfinyl- (alkyle en C₁ à C₆) -, (alkyle en C₁ à C₆) -sulfonyl- (alkyle en C₁ à C₆) -, (aryle en C₆ à C₁₀) -sulfonyl- (alkyle en C₁ à C₆) -, amino- (alkyle en C₁ à C₆) -, (alkyle en C₁ à C₆) amino- (alkyle en C₁ à C₆) -, (alkyle en C₁ à C₆) ₂amino- (alkyle en C₁ à C₆) - ;
(b) R⁵OCO (alkyle en C₁ à C₆) -, dans lequel R⁵ est choisi dans le groupe consistant en l'hydrogène, des groupes alkyl- en C₁ à C₆, (aryle en C₆ à C₁₀) - (alkyle en C₁ à C₆) - et (hétéroaryle en C₁ à C₉) - (alkyle en C₁ à C₆) - ; ou bien
(c) R⁶ (alkyle en C₀ à C₆) -, dans lequel R⁶ est choisi dans le groupe consistant en des groupes pipérazino, (acyle en C₁ à C₆) -pipérazino-, (aryle en C₆ à C₁₀) -pipérazino-, (hétéroaryle en C₅ à C₉) -pipérazino-, (alkyle en C₁ à C₆) - pipérazino-, (aryle en C₆ à C₁₀) - (alkyle en C₁ à C₆) - pipérazino-, (hétéroaryle en C₁ à C₉) - (alkyle en C₁ à C₆) - pipérazino-, morpholino-, (acyle en C₁ à C₆) -morpholino-, (aryle en C₆ à C₁₀) -morpholino-, (hétéroaryle en C₁ à C₉) - morpholino-, (alkyle en C₁ à C₆) -morpholino-, (aryle en C₆ à C₁₀) - (alkyle en C₁ à C₆) -morpholino-, (hétéroaryle en C₁ à C₉) - (alkyle en C₁ à C₆)-morpholino-, thiomorpholino-, (acyle en C₁ à C₆) -thiomorpholino-, (aryle en C₆ à C₁₀) - thiomorpholino-, (hétéroaryle en C₁ à C₉)-thiomorpholino-, (alkyle en C₁ à C₆) -thiomorpholino-, (aryle en C₆ à C₁₀) - (alkyle en C₁ à C₆)-thiomorpholino-, (hétéroaryle en C₁ à C₉)-(alkyle en C₁ à C₆)-thiomorpholino-, pipéridino-, (acyle en C₁ à C₆) -pipéridino-, (aryle en C₆ à C₁₀) -pipéridino-, (hétéroaryle en C₁ à C₉) -pipéridino-, (alkyle en C₁ à C₆) - pipéridino-, (aryle en C₆ à C₁₀) - (C₁ à C₆) -pipéridino-, (hétéroaryle en C₁ à C₉) - (alkyle en C₁ à C₆) -pipéridino-, pyrrolidino-, (acyle en C₁ à C₆) -pyrrolidino-, (aryle en C₆ à C₁₀) -pyrrolidino-, (hétéroaryle en C₁ à C₉) -pyrrolidino-, (alkyle en C₁ à C₆) -pyrrolidino-, (aryle en C₆ à C₁₀) - (C₁ à C₆) -pyrrolidino-, (hétéroaryle en C₁ à C₉) - (alkyle en C₁ à C₆) -pyrrolidino-, (alkoxy en C₁ à C₆) - (acyle en C₁ à C₆) -, (alkyle en C₁ à C₆) -amino- (aryle en C₆ à C₁₀) - et (alkyle en C₁ à C₆) ₂amino- (acyle en C₁ à C₆) - ;
R² représente un à quatre substituants facultatifs, chacun étant choisi indépendamment parmi les membres des groupes (a) à (f) :
(a) deutérium, halogéno, hydroxy, carboxy, amino, trifluorométhyle, alkyl- en C₁ à C₆, alcényl- en C₂ à C₆, alcynyl- en C₂ à C₆, (alkyle en C₁ à C₆) -amino-, (alkyle en C₁ à C₆)₂-amino-, cyanalkyl-, cycloalkyl- en C₃ à C₁₀, hétérocycloalkyl- en C₃ à C₁₀, cycloalkoxy- en C₃ à C₁₀, alkylthio- en C₁ à C₆, (alkyle en C₁ à C₆)-sulfinyl-, (alkyle en C₁ à C₆) -sulfonyl-, amino-CO-NH-, (alkoxy en C₁ à C₆) -CO-NH-, (alkyle en C₁ à C₆) -CO-NH-, (alkyle en C₁ à C₆)-CO-NH-(alkyle en C₁ à C₆) -, (alkyle en C₁ à C₆)-CO-NH-(alkoxy en C₁ à C₆) -, (alkoxy en C₁ à C₆) -carbonyl- (alkoxy en C₁ à C₆) -, (alkoxy en C₁ à C₆) -CO-NH- (alkoxy en C₁ à C₆) -, (alkyle en C₁ à C₆) -amino-CO-NH-, (alkyle en C₁ à C₆) -amino-CO-NH- (alkyle en C₁ à C₆) -, (alkyle en C₁ à C₆)₂amino-CO-NH-(alkyle en C₁ à C₆) -, (alkyle en C₁ à C₆)₂amino-CO-NH-, carboxy-, carboxy-(alkyle en C₁ à C₆)-, carboxy(alkoxy en C₁ à C₆) -, benzyloxycarbonyl- (alkoxy en C₁ à C₆) -, (alkyle en C₁ à C₆) -amino-CO-, (acyle en C₁ à C₆) -amino-, alkoxy- en C₁ à C₆-, acyl- en C₁ à C₆, (acyle en C₁ à C₆) -oxy-, (acyle en C₁ à C₆) - (alkyle en C₁ à C₆) -amino-, (alkoxy en C₁ à C₆) - acyl-, (alkyle en C₁ à C₆) -aminoacyl-, (alkyle en C₁ à C₆)₂aminoacyl - , amino- (acyle en C₁ à C₆)-, amino- (alkyle en C₁ à C₆) -, (alkoxy en C₁ à C₆) -carbonylamino-, (alkoxy en C₁ à C₆)-carbonyl-(alkyle en C₁ à C₆) -, (aryle en C₆ à C₁₀) - (alkoxy en C₁ à C₆)-carbonylamino-, trihalogénométhyl-, trihalogénométhyl- (alkyle en C₁ à C₆) -, (alkyle en C₁ à C₆) - dihalogénométhylène-, (alkyle en C₁ à C₃) - (dihalogénométhylène)-(alkyle en C₁ à C₃)-, cycloalkyl- en C₃ à C₆, (cycloalkyle en C₃ à C₆) - (alkyle en C₁ à C₆) -, hydroxyalkyl- en C₁ à C₆, (acyle en C₁ à C₆)-oxy-(alkyle en C₁ à C₆) -, (alkoxy en C₁ à C₆) - (alkyle en C₁ à C₆) -, (acyle en C₁ à C₆) -amino- (alkyle en C₁ à C₆) -, (alkyle en C₁ à C₆) - thio-(alkyle en C₁ à C₆) -, (alkoxy en C₁ à C₆) -carbonyl-, (alkyle en C₁ à C₆) -sulfinyl- (alkyle en C₁ à C₆) -, (alkyle en C₁ à C₆) -sulfonyl- (alkyle en C₁ à C₆) -, (alkyle en C₁ à C₆) -sulfonyl-, (alkyle en C₁ à C₆) -sulfonylamino-, (alkyle en C₁ à C₆) sulfonylamino- (alkyle en C₁ à C₆) -, amino-(alkyle en C₁ à C₆) -, (alkyle en C₁ à C₆) -amino- (alkyle en C₁ à C₆) -, (alkyle en C₁ à C₆) ₂amino- (alkyle en C₁ à C₆) -, (alkyle en C₁ à C₆)-CO- (alkyle en C₁ à C₆) - ;
(b) aryl- en C₆ à C₁₀, hétéroaryl- en C₁ à C₉, (aryle en C₆ à C₁₀) - (aryle en C₆ à C₁₀) -, (aryle en C₆ à C₁₀)-(hétéroaryle en C₁ à C₉) -, (hétéroaryle en C₁ à C₉) - (hétéroaryle en C₁ à C₉) -, (hétéroaryle en C₁ à C₉) - (aryle en C₆ à C₁₀) -, (aryle en C₆ à C₁₀) -sulfinyl-, (aryle en C₆ à C₁₀) - (aryle en C₆ à C₁₀) -sulfinyl-, (hétéroaryle en C₁ à C₉) - (aryle en C₆ à C₁₀) -sulfinyl-, (aryle en C₆ à C₁₀) -sulfonyl-, (aryle en C₆ à C₁₀) - (aryle en C₆ à C₁₀) -sulfonyl-, (hétéroaryle en C₁ à C₉) - (aryle en C₆ à C₁₀) -sulfonyl-, (hétéroaryle en C₁ à C₉)-sulfinyl-, (hétéroaryle en C₁ à C₉) - (hétéroaryle en C₁ à C₉) -sulfinyl-, (aryle en C₆ à C₁₀)-(hétéroaryle en C₁ à C₉) - sulfinyl-, (hétéroaryle en C₁ à C₉) - sulfonyl-, (hétéroaryle en C₁ à C₉) - (hétéroaryle en C₁ à C₉) -sulfonyl-, (aryle en C₆ à C₁₀) - (hétéroaryle en C₁ à C₉-sulfonyl-, (R⁴) sulfinyl-, (R⁴) sulfonyl-, (aryle en C₆ à C₁₀) - (R⁴) -sulfinyl-, (aryle en C₆ à C₁₀) - (aryle en C₆ à C₁₀) - (R⁴) -sulfinyl-, (hétéroaryle en C₁ à C₉) - (aryle en C₆ à C₁₀) - (R⁴) -sulfinyl-, (aryle en C₆ à C₁₀) (R⁴) -sulfonyl-, (aryle en C₆ à C₁₀) - (aryle en C₆ à C₁₀) (R⁴) -sulfonyl-, (hétéroaryle en C₁ à C₉) - (aryle en C₆ à C₁₀) (R⁴) -sulfonyl-, (hétéroaryle en C₁ à C₉) - (R⁴) -sulfinyl-, (aryle en C₆ à C₁₀) - (hétéroaryle en C₁ à C₉) (R⁴) -sulfinyl-, (hétéroaryle en C₁ à C₉) - (hétéroaryle en C₁ à C₉) - (R⁴) -sulfinyl-, (hétéroaryle en C₁ à C₉) (R⁴) -sulfonyl-, (aryle en C₆ à C₁₀) - (hétéroaryle en C₅ à C₉) - (R⁴) -sulfonyl-, (hétéroaryle en C₁ à C₉) - (hétéroaryle en C₁ à C₉) - (R⁴) -sulfonyl-, (aryle en C₆ à C₁₀) aminocarbonyl-, (aryle en C₆ à C₁₀) (aryle en C₆ à C₁₀) -aminocarbonyl-, (hétéroaryle en C₁ à C₉) - (aryle en C₆ à C₁₀) -aminocarbonyl-, (hétéroaryle en C₁ à C₉)-aminocarbonyl-, (aryle en C₆ à C₁₀) - (hétéroaryle en C₁ à C₉) -aminocarbonyl-, (hétéroaryle en C₁ à C₉) - (hétéroaryle en C₁ à C₉) -aminocarbonyl-, (aryle en C₆ à C₁₀) -carbonyl-, (aryle en C₆ à C₁₀) - (aryle en C₆ à C₁₀) -carbonyl-, (hétéroaryle en C₁ à C₉) - (aryle en C₆ à C₁₀) - carbonyl-, (hétéroaryle en C₁ à C₉)-carbonyl-, (aryle en C₆ à C₁₀) - (hétéroaryle en C₁ à C₉) -carbonyl-, (hétéroaryle en C₁ à C₉) - (hétéroaryle en C₁ à C₉) -carbonyl-, (aryle en C₆ à C₁₀) -oxycarbonyl-, (aryle en C₆ à C₁₀) - (aryle en C₆ à C₁₀) - oxycarbonyl-, (hétéroaryle en C₁ à C₉) - (aryle en C₆ à C₁₀) - oxycarbonyl-, (hétéroaryle en C₁ à C₉)-oxycarbonyle, (aryle en C₆ à C₁₀) - (hétéroaryle en C₁ à C₉) -oxycarbonyl-, (hétéroaryle en C₁ à C₉) - (hétéroaryle en C₁ à C₉) - oxycarbonyl - , (R⁴) carbonyl-, (R⁴) oxycarbonyl-, (R⁴) aminocarbonyl-, (aryle en C₆ à C₁₀) (R⁴) carbonyl-, (aryle en C₆ à C₁₀) (R⁴) oxycarbonyl-, (aryle en C₆ à C₁₀) (R⁴) -aminocarbonyl-, (hétéroaryle en C₁ à C₉) - (R⁴) carbonyl-, (hétéroaryle en C₅ à C₉) (R⁴) -oxycarbonyl-, (hétéroaryle en C₁ à C₉) (R⁴) - aminocarbonyl-, dans lesquels R⁴ répond à la définition précitée, n'importe lequel desdits groupes aryle en C₆ à C₁₀ ou hétéroaryl- en C₁ à C₉ de R² pouvant être facultativement substitué avec un à cinq groupes choisis indépendamment entre :
(i) hydroxy, halogéno, amino, trifluorométhyle, carboxy, alkoxy- en C₁ à C₆, (acyle en C₁ à C₆)oxy-, (alkyle en C₁ à C₆) -amino-, (alkyle en C₁ à C₆)₂amino-, (acyle en C₁ à C₆)-amino-, cyano, nitro, alkyl- en C₁ à C₆, alcényl- en C₂ à C₆, alcynyl- en C₂ à C₆, (acyle en C₁ à C₆) -amino-, cyano- (alkyle en C₁ à C₆) -, trifluorométhyl- (alkyle en C₁ à C₆) - ou nitro- (alkyle en C₁ à C₆) -, (alkyle en C₁ à C₃) - difluorométhylène- (alkyle en C₁ à C₃) -, (acyle en C₁ à C₆) - amino-(alkyle en C₁ à C₆) -, (alkoxy en C₁ à C₆) - (acyle en C₁ à C₆) -amino-, amino-(acyle en C₁ à C₆) -, amino(acyle en C₁ à C₆) - (alkyle en C₁ à C₆) -, (alkyle en C₁ à C₆) -amino- (acyle en C₁ à C₆) -, (alkyle en C₁ à C₆) ₂amino- (acyle en C₁ à C₆) -, (cycloalkyle en C₃ à C₁₀) - (alkyle en C₁ à C₆) -, (acyle en C₁ à C₆) -oxy- (alkyle en C₁ à C₆) -, (alkoxy en C₂ à C₆) - (alkyle en C₁ à C₆) -, pipérazinyl- (alkyle en C₁ à C₆) -, (acyle en C₁ à C₆) -amino- (alkyle en C₁ à C₆) -, (aryle en C₆ à C₁₀) - (alkoxy en C₁ à C₆) - (alkyle en C₁ à C₆) -, (hétéroaryle en C₁ à C₉) - (alkoxy en C₁ à C₆) - (alkyle en C₁ à C₆) -, (alkyle en C₁ à C₆) -thio- (alkyle en C₁ à C₆) -, (aryle en C₆ à C₁₀) -thio-(alkyle en C₁ à C₆) -, (alkyle en C₁ à C₆) -sulfinyl- (alkyle en C₁ à C₆) -, (aryle en C₆ à C₁₀) -sulfinyl- (alkyle en C₁ à C₆) -, (alkyle en C₁ à C₆) sulfonyl- (alkyle en C₁ à C₆) -, (aryle en C₆ à C₁₀) -sulfonyl- (alkyle en C₁ à C₆) -, amino-(alkyle en C₁ à C₆) -, (alkyle en C₁ à C₆) -amino- (alkyle en C₁ à C₆) -, (alkyle en C₁ à C₆) -difluorométhylène-, (alkyle en C₁ à C₃) difluorométhylène- (alkyle en C₁ à C₃) -, (alkoxy en C₁ à C₆) - (acyle en C₁ à C₆) -, (alkyle en C₁ à C₆) amino-(acyle en C₁ à C₆) -, (alkyle en C₁ à C₆) ₂amino (acyle en C₁ à C₆) -, aryl- en C₆ à C₁₀, hétéroaryl- en C₁ à C₉, (aryle en C₆ à C₁₀) - (alkyle en C₁ à C₆) -, (hétéroaryle en C₁ à C₉) - (alkyle en C₁ à C₆)-, (aryle en C₆ à C₁₀) - (aryle en C₆ à C₁₀) -, (aryle en C₆ à C₁₀) - (aryle en C₆ à C₁₀) - (alkyle en C₁ à C₆) -, cycloalkyl- en C₃ à C₁₀, (cycloalkyle en C₃ à C₆) - (alkyle en C₁ à C₆) -, hétérocycloalkyl- en C₃ à C₁₀, (hétérocycloalkyle en C₃ à C₁₀) - (alkyle en C₁ à C₆) -, hydroxy (alkyle en C₂ à C₆) -, (acyle en C₁ à C₆) -oxy- (alkyle en C₂ à C₆) -, (alkoxy en C₁ à C₆) - (alkyle en C₂ à C₆) -, pipérazinyl- (alkyle en C₁ à C₆) -, (acyle en C₁ à C₆)-amino- (alkyle en C₁ à C₆) -, (aryle en C₆ à C₁₀) - (alkoxy en C₁ à C₆) - (alkyle en C₁ à C₆) -, (hétéroaryle en C₁ à C₉) - (alkoxy en C₁ à C₆) - (alkyle en C₁ à C₆) -, (alkyle en C₁ à C₆) -thio- (alkyle en C₁ à C₆) -, (aryle en C₆ à C₁₀) -thio- (alkyle en C₁ à C₆) -, (alkyle en C₁ à C₆) sulfinyl- (alkyle en C₁ à C₆) -, (aryle en C₆ à C₁₀) - sulfinyl- (alkyle en C₁ à C₆) -, (alkyle en C₁ à C₆) sulfonyl-(alkyle en C₁ à C₆) -, (aryle en C₆ à C₁₀) -sulfonyl- (alkyle en C₁ à C₆) -, amino- (alkyle en C₁ à C₆) -, (alkyle en C₁ à C₆) amino- (alkyle en C₁ à C₆) -, (alkyle en C₁ à C₆) ₂amino-(alkyle en C₁ à C₆) - ;
(ii) R⁵OCO- (alkyle en C₁ à C₆) - dans lequel R⁵ est choisi dans le groupe consistant en l'hydrogène, des groupes alkyle en C₁ à C₆, (aryle en C₆ à C₁₀) - (alkyle en C₁ à C₆) - et (hétéroaryle en C₁ à C₉) - (alkyle en C₁ à C₆) - ;
(iii) R⁶- (alkyle en C₂ à C₆) - dans lequel R⁶ est choisi dans le groupe consistant en des groupes pipérazino, (acyle en C₁ à C₆)-pipérazino-, (aryle en C₆ à C₁₀) -pipérazino-, (hétéroaryle en C₅ à C₉) -pipérazino-, (alkyle en C₁ à C₆) - pipérazino-, (aryle en C₆ à C₁₀) - (alkyle en C₁ à C₆) pipérazino-, (hétéroaryle en C₁ à C₉) - (alkyle en C₁ à C₆) -pipérazino-, morpholino-, (acyle en C₁ à C₆) - morpholino-, (aryle en C₆ à C₁₀)-morpholino-, (hétéroaryle en C₁ à C₉) -morpholino-, (alkyle en C₁ à C₆)morpholino-, (aryle en C₆ à C₁₀) - (alkyle en C₁ à C₆) -morpholino-, (hétéroaryle en C₁ à C₉) - (alkyle en C₁ à C₆) morpholino-, thiomorpholino-, (acyle en C₁ à C₆)-thiomorpholino-, (aryle en C₆ à C₁₀) -thiomorpholino-, (hétéroaryle en C₁ à C₉) - thiomorpholino-, (alkyle en C₁ à C₆)thiomorpholino-, (aryle en C₆ à C₁₀) - (alkyle en C₁ à C₆) -thiomorpholino-, (hétéroaryle en C₁ à C₉) - (alkyle en C₁ à C₆) - thiomorpholino-, pipéridino-, (acyle en C₁ à C₆)-pipéridino-, (aryle en C₆ à C₁₀)-pipéridino-, (hétéroaryle en C₁ à C₉) -pipéridino-, (alkyle en C₁ à C₆) -pipéridino-, (aryle en C₆ à C₁₀) - (alkyle en C₁ à C₆) -pipéridino-, (hétéroaryle en C₁ à C₉) - (alkyle en C₁ à C₆) -pipéridino-, pyrrolidino-, (acyle en C₁ à C₆) -pyrrolidino-, (aryle en C₆ à C₁₀)pyrrolidino-, (hétéroaryle en C₁ à C₉) -pyrrolidino-, (alkyle en C₁ à C₆) -pyrrolidino-, (aryle en C₆ à C₁₀) - (alkyle en C₁ à C₆) -pyrrolidino-, (hétéroaryle en C₁ à C₉) - (alkyle en C₁ à C₆) -pyrrolidino-, (alkoxy en C₁ à C₆) - (acyle en C₁ à C₆) -, (alkyle en C₁ à C₆) -amino- (aryle en C₆ à C₁₀) - et (alkyle en C₁ à C₆) ₂amino (acyle en C₁ à C₆) - ;
(c) R⁷, ou R⁷Y-, dans lequel R⁷ est choisi dans le groupe consistant en des groupes pipérazino-, (aryle en C₆ à C₁₀) -pipérazino-, (hétéroaryle en C₁ à C₉) -pipérazino-, (alkyle en C₁ à C₆) -pipérazino-, (aryle en C₆ à C₁₀) - (alkyle en C₁ à C₆) -pipérazino-, (hétéroaryle en C₁ à C₉) - (alkyle en C₁ à C₆) -pipérazino-, morpholino-, (aryle en C₆ à C₁₀) - morpholino-, (hétéroaryle en C₁ à C₉)-morpholino-, (alkyle en C₁ à C₆) -morpholino-, (aryle en C₆ à C₁₀) - (alkyle en C₁ à C₆) -morpholino-, (hétéroaryle en C₁ à C₉) - (alkyle en C₁ à C₆) -morpholino-, thiomorpholino-, (aryle en C₆ à C₁₀) - thiomorpholino-, (hétéroaryle en C₁ à C₉)-thiomorpholino-, (alkyle en C₁ à C₆) -thiomorpholino-, (aryle en C₆ à C₁₀)-(alkyle en C₁ à C₆) -thiomorpholino-, (hétéroaryle en C₁ à C₉) - (alkyle en C₁ à C₆) thiomorpholino-, pipéridino-, (aryle en C₆ à C₁₀) -thiopipéridino-, (hétéroaryle en C₁ à C₉) - thiopipéridino-, (alkyle en C₁ à C₆)-thiopipéridino-, (aryle en C₆ à C₁₀) - (alkyle en C₁ à C₆) -thiopipéridino-, (hétéroaryle en C₁ à C₉) - (alkyle en C₁ à C₆) - thiopipéridino-, pyrrolidino-, (aryle en C₆ à C₁₀)-thiopyrrolidino-, (hétéroaryle en C₁ à C₉)-thiopyrrolidino-, (alkyle en C₁ à C₆)-thiopyrrolidino-, (aryle en C₆ à C₁₀) - (alkyle en C₁ à C₆) -thiopyrrolidino-, (hétéroaryle en C₁ à C₉) - (alkyle en C₁ à C₆) - thiopyrrolidino-, et Y, s'il est présent, est choisi dans le groupe consistant en des groupes alkyl- en C₁ à C₆, alcényl- en C₂ à C₆, alcynyl- en C₂ à C₆, amino-, oxygène, thio, sulfinyle, sulfonyle, halogénalkyl- en C₁ à C₆ et hydroxyalkyl- en C₂ à C₆ ;
(d) ZR⁸-, dans lequel R⁸ est choisi dans le groupe consistant en des groupes pipérazino-, (aryle en C₆ à C₁₀)-pipérazino-, (hétéroaryle en C₁ à C₉) -pipérazino-, (alkyle en C₁ à C₆) -pipérazino-, (aryle en C₆ à C₁₀) - (alkyle en C₁ à C₆) -pipérazino-, (hétéroaryle en C₁ à C₉) - (alkyle en C₁ à C₆) -pipérazino-, morpholino-, (aryle en C₆ à C₁₀) - morpholino-, (hétéroaryle en C₁ à C₉)-morpholino-, (alkyle en C₁ à C₆) -morpholino-, (aryle en C₆ à C₁₀) - (alkyle en C₁ à C₆) -morpholino-, (hétéroaryle en C₁ à C₉) - (alkyle en C₁ à C₆) -morpholino-, thiomorpholino-, (aryle en C₆ à C₁₀) - thiomorpholino-, (hétéroaryle en C₁ à C₉)-thiomorpholino-, (alkyle en C₁ à C₆) -thiomorpholino-, (aryle en C₆ à C₁₀)-(alkyle en C₁ à C₆) -thiomorpholino-, (hétéroaryle en C₁ à C₉)-(alkyle en C₁ à C₆)thiomorpholino-, pipéridino-, (aryle en C₆ à C₁₀) -thiopipéridino-, (hétéroaryle en C₁ à C₉) - thiopipéridino-, (alkyle en C₁ à C₆)-thiopipéridino-, (aryle en C₆ à C₁₀) - (alkyle en C₁ à C₆) -thiopipéridino-, (hétéroaryle en C₁ à C₉) - (alkyle en C₁ à C₆)-thiopipéridino-, pyrrolidino-, (aryle en C₆ à C₁₀)-thiopyrrolidino-, (hétéroaryle en C₁ à C₉)-thiopyrrolidino-, (alkyle en C₁ à C₆) -thiopyrrolidino-, (aryle en C₆ à C₁₀)-(alkyle en C₁ à C₆)-thiopyrrolidino-, (hétéroaryle en C₁ à C₉) - (alkyle en C₁ à C₆) -thiopyrrolidino-, et Z est choisi dans le groupe consistant en des groupes alkyl- en C₁ à C₆, alcényl- en C₂ à C₆, alcynyl- en C₂ à C₆, amino-, oxygène, thio, sulfinyle, sulfonyle, halogénalkyl- en C₁ à C₆ et hydroxyalkyl- en C₂ à C₆ ;
(e) deux ou plus de deux groupes R², lorsqu'ils sont vicinaux, pris conjointement pour former un ou plusieurs noyaux de 4, 5, 6 ou 7 atomes choisis dans le groupe consistant en des noyaux phényl-, naphtyl-, furyl-, thiényl-, thiazolyl-, pyrazolyl-, isothiazolyl-, oxazolyl-, isoxazolyl-, pyrrolyl-, triazolyl-, tétrazolyl-, imidazolyl-, 1,3,5-oxadiazolyl-, 1,2,4-oxadiazolyl-, 1,2,3-oxadiazolyl-, 1,3,5-thiadiazolyl-, 1,2,3-thiadiazolyl-, 1,2,4-thiadiazolyl-, pyridyl-, pyrimidyl-, pyrazinyl-, pyridazinyl-, 1,2,4-triazinyl-, 1,2,3-triazinyl-, 1,3,5-triazinyl-, pyrazolo[3,4-b]pyridinyl-, cinnolinyl-, ptéridinyl-, purinyl-, 6,7-dihydro-5H-[1]pyridinyl-, benzo[b]thiophényl-, 5,6,7,8-tétrahydro-quinoléine-3-yle, benzoxazolyl-, benzothiazolyl-, benzisothiazolyl-, benzisoxazolyl-, benzimidazolyl-, thianaphtényl-, isothianaphtényl-, benzofurannyl-, isobenzofurannyl-, isoindolyl-, indolyl-, indolizinyl-, indazolyl-, isoquinolyl-, quinolyl-, phtalazinyl-, quinoxalinyl-, quinazolinyl-, benzoxazinyl-, ledit ou lesdits noyaux étant facultativement substitué(s) avec un ou plusieurs substituants alkyl- en C₁ à C₆, alcényl- en C₂ à C₆, alcynyl- en C₂ à C_{6,} amino-, halogéno- , hydroxy-, carboxy-, thiol-, nitro-, cyano-, sulfonyl-, halogénalkyl- en C₁ à C₆ ou hydroxyalkyl- en C₂ à C₆ ; et
(f) deux ou plus de deux groupes R² , lorsqu'ils sont vicinaux, pris conjointement pour former un ou plusieurs noyaux supplémentaires de 3, 4, 5, 6 ou 7 atomes constitutifs, choisis dans les groupes consistant en les suivants :
(i) un groupe cycloalkyle en C₃ à C₁₀, contenant 0 à 2 niveaux d'insaturation, choisi dans le groupe consistant en des groupes cyclopropyl-, cyclobutyl-, cyclopentyl-, cyclohexyl-, cycloheptyl-, cyclopropényl-, cyclobutényl-, cyclopentényl-, cyclohexényl-, cycloheptényl-, 1,3-cyclobutadiényl-, 1,3-cyclopentadiényl-, 1,3-cyclohexadiényl-, 1,4-cyclohexadiényl-, 1,3-cycloheptadiényl-, 1,4-cycloheptadiényl-, bicyclo[3.2.1]octane-, bicyclo[2.2.1]heptane, sa forme insaturée norborn-2-ène, etc., ledit noyau étant facultativement substitué avec des substituants hydroxy-, halogéno-, amino-, trifluorométhyl-, hydroxyalkyl- en C₂ à C₆, alkoxy- en C₁ à C₆, (acyle en C₁ à C₆) -oxy-, (alkyle en C₁ à C₆) -amino-, (alkyle en C₁ à C₆)₂amino-, (acyle en C₁ à C₆)-amino-, cyano-, nitro-, carboxy-, thiol-, sulfonyl-, alkyl- en C₁ à C₆, alcényl- en C₂ à C₆, alcynyl- en C₂ à C₆, (acyle en C₁ à C₆)-amino-, cyano-(alkyle en C₁ à C₆)-, trifluorométhyl- (alkyle en C₁ à C₆) -, (alkyle en C₁ à C₃) - difluorométhylène- (alkyle en C₁ à C₃) -, halogénalkyl- en C₁ à C₆ ou nitro-(alkyle en C₁ à C₆) - ; et
(ii) un groupe hétérocycloalkyl- en C₃ à C₁₀ choisi dans le groupe consistant en des groupes pyrrolidinyl-, tétrahydrofurannyl-, dihydrofurannyl-, tétrahydropyranyl-, pyranyl-, thiopyranyl-1, aziridinyl-, oxiranyl-, méthylènedioxyl-, isoxazolidinyl-, 1,3-oxazolidine-3-yl-, isothiazolidinyl-, 1,3-thiazolidine-3-yl-, 1,2-pyrazolidine-2-yl-, 1,3-pyrazolidine-1-yl-, pipéridinyl-, thiomorpholinyl-, 1,2-tétrahydrothiazine-2-yl-, 1,3-tétrahydrothiazine-3-yl-, tétrahydrothiadiazinyl-, morpholinyl-, 1,2-tétrahydro-diazine-2-yl-, 1,3-tétrahydrodiazine-1-yl-, tétrahydro-azépinyl-, pipérazinyl-, chroményl-, chromanyl-, ledit noyau étant facultativement substitué avec des substituants hydroxy-, halogéno-, amino-, trifluorométhyl-, hydroxyalkylen C₂ à C₆, alkoxy- en C₁ à C₆, (acyle en C₁ à C₆) oxy-, (alkyle en C₁ à C₆) -amino-, (alkyle en C₁ à C₆)₂amino-, (acyle en C₁ à C₆)-amino-, cyano-, nitro-, carboxy-, thiol-, sulfonyl-, alkyl- en C₁ à C₆, alcényl- en C₂ à C₆, alcynylen C₂ à C₆, (acyle en C₁ à C₆) amino-, cyano- (alkyle en C₁ à C₆) -, trifluorométhyl- (alkyle en C₁ à C₆) -, (alkyle en C₁ à C₃)-difluorométhylène-(alkyle en C₁ à C₃) -, halogénalkyl- en C₁ à C₆ ou nitro- (alkyle en C₁ à C₆) - ;
n'importe quel groupe alkyl- en C₁ à C₆, alcényl- en C₂ à C₆, alcynyl- en C₂ à C₆, cycloalkyle en C₃ à C₁₀ ou hétérocycloalkyl- en C₃ à C₁₀ qui constitue les, ou qui constitue une partie des, un à quatre substituants R² facultatifs sont eux-mêmes facultativement substitués avec des substituants deutérium-, hydroxy-, amino-, trifluorométhyl-, cyano-, nitro-, carboxy-, alkoxy- en C₁ à C₄, (acyle en C₁ à C₆) -oxy-, (alkyle en C₁ à C₆) -amino-, (alkyle en C₁ à C₆)₂amino-, alkyl- en C₁ à C₆, alcényl- en C₂ à C₆, alcynyl- en C₂ à C₆, (acyle en C₁ à C₆) -amino-, cycloalkyl- en C₃ à C₁₀, hétérocycloalkyl- en C₃ à C₁₀, cyano-(alkyle en C₁ à C₆) -, trifluorométhyl- (alkyle en C₁ à C₆) -, nitro- (alkyle en C₁ à C₆) -, et (acyle en C₁ à C₆) - amino-, et
R³ représente un ou plusieurs substituants facultatifs sur un atome de carbone du noyau, y compris au niveau de X lorsque X représente un groupe -CH₂-, choisis dans le groupe consistant en des substituants alkyl- en C₁ à C₆, trihalogéno- (alkyle en C₁ à C₆) -, consistant de préférence en un groupe trifluorométhyl-, deutérium et fluor.

2. Composé suivant la revendication 1, dans lequel son constituant structural qui est représenté par la formule : 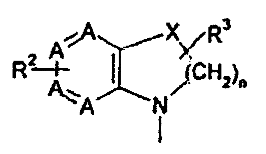 est fourni par la 1,2,3,4-tétrahydroquinoléine, la 1,2,3,4-tétrahydroquinoxaline, la 3,4-dihydro-1H-quinoxaline-2-one, la 3,4-dihydro-2H-benzo[1,4]oxazine, le 2,3-dihydro-1H-indole ou la 3,4-dihydro-2H-benzo[1,4]-thiazine.

3. Composé suivant la revendication 2, qui comprend une structure choisie dans le groupe consistant en les structures suivantes :
6-méthoxy-1,2,3,4-tétrahydroquinoléine ; 4-méthyl-1,2,3,4-tétrahydroquinoléine ; 7-(trifluorométhyl)-1,2,3,4-tétrahydroquinoléine ; 8-méthyl-1,2,3,4-tétrahydroquinoléine ; 6-hydroxy-1,2,3,4-tétrahydroquinoléine ; 8-chloro-1,2,3,4-tétrahydroquinoléine ; 7-chloro-1,2,3,4-tétrahydroquinoléine ; 6-benzyloxy-7-méthoxy-1,2,3,4-tétrahydroquinoléine ; 6,7-diméthyl-1,2,3,4-tétrahydroquinoxaline ; 1,2,3,4-tétrahydro-quinoxaline ; 1-phénylsulfonyl-1,2,3,4-tétrahydroquinoxaline ; 6-méthyl-1,2,3,4-tétrahydroquinoléine ; 3,4-dihydro-2H-benzo-[1,4]oxazine ; 5-fluoro-2,3-dihydro-1H-indole et 3,3-diméthyl-2,3-dihydro-1H-indole.

4. Composé suivant la revendication 1, dans lequel R¹ représente un groupe hétéroaryl- en C₁ à C₉ choisi dans le groupe consistant en les groupes pyridyl-, indazolyl-, indolyl-, 1,3-dihydro-benzo-imidazole-2-one, thiényl-, oxazoyl-, 2H-pyrazolyl-, 1H-pyrazolyl-, iso-oxazoyl-, thiazolyl- et isothiazoyl-, et est facultativement substitué avec un ou plusieurs groupes choisis chacun indépendamment entre des groupes hydroxy-, halogéno-, amino-, alkyl- en C₁ à C₆, alkoxy- en C₁ à C₆, trihalogénométhyl-, alcynyl- en C₁ à C₆, (alkyle en C₁ à C₆) -amino-, di-(alkyle en C₁ à C₆)₂amino-, carboxy-, (alkoxy en C₁ à C₆) -carbonyl-, (acyle en C₁ à C₆) -oxy- et (acyle en C₁ à C₆) -amino- .

5. Composé suivant la revendication 1, dans lequel R¹ représente un groupe phényle, facultativement substitué avec un à cinq substituants, qui sont choisis chacun indépendamment entre des substituants hydroxy-, halogéno-, amino-, alkyl- en C₁ à C₆, alkoxy- en C₁ à C₆, trihalogéno(alkyle en C₁ à C₆)-, qui consiste de préférence en un groupe trifluorométhyl-, alcynyl- en C₁ à C₆, (alkyle en C₁ à C₆) -amino-, di- (alkyle en C₁ à C₆)-₂amino-, carboxy-, (alkoxy en C₁ à C₆) -carbonyl-, (acyle en C₁ à C₆) -oxy-, et (acyle en C₁ à C₆) -amino-.

6. Composé suivant la revendication 1, dans lequel R¹ est choisi dans le groupe consistant en les groupes 3,4,5-triméthoxyphényl-, 2,3-diméthyl-1H-indole-5-yle, 3,4-dihydro-2H-quinoléine-1-yle, et 6-morpholine-4-yl-pyridine-3-yle.

7. Composé suivant la revendication 1, dans lequel un ou plusieurs des substituants R² sont choisis dans les groupes consistant en les suivants :
(a) des groupes alkyl- en C₁ à C₆, alcynyl- en C₁ à C₆, alkoxy- en C₁ à C₆, trihalogéno (alkyle en C₁ à C₆) -, consistant de préférence en un groupe trifluorométhyl-, (alkyle en C₁ à C₆) -amino-, di-(alkyle en C₁ à C₆)₂-amino-, amino-, cyano- et halogéno- ; et
(b) des groupes benzyloxy-, phénylsulfonyl-, phénylaminocarbonyl-, (hétéroaryle en C₁ à C₉)-sulfonyl- et (hétéroaryle en C₁ à C₉)-aminocarbonyl-, facultativement substitués avec un ou plusieurs groupes choisis dans le groupe consistant en des substituants alkyl- en C₁ à C₆, alcynyl- en C₂ à C₆, trihalogéno-(alkyle en C₁ à C₆) - consistant de préférence en un groupe trifluorométhyl-, alkoxy- en C₁ à C₆, (alkyle en C₁ à C₆) -amino-, (alkyle en C₁ à C₆) ₂amino- et halogéno.

8. Composé suivant la revendication 1, dans lequel un ou plusieurs des substituants R³ sont choisis dans les groupes consistant en des groupes alkyl- en C₁ à C₆, trihalogéno-(alkyle en C₁ à C₆)-, deutérium et fluor.

9. Composé suivant la revendication 8, dans lequel R³ représente un groupe trifluorométhyle.

10. Composé suivant la revendication 1, choisi dans le groupe consistant en les composés suivants :
(a) 1-[(2-anilino)-4-pyrimidinyl]-6-méthyl-1,2,3,4-tétrahydroquinoléine ;
(b) 1-[2-[(4-bromophényl)-amino]-4-pyrimidinyl]-6-méthyl-1,2,3,4-tétrahydroquinoléine ;
(c) 1-[2-[(4-méthoxyphényl)amino]-4-pyrimidinyl]-6-méthyl-1,2,3,4-tétrahydroquinoléine ;
(d) 1-[2-[(1H-indazole-5-yl)]-4-pyrimidyl]-6-méthyl-1,2,3,4-tétrahydroquinoléine ;
(e) 1-[2-[(4-phénoxyphényl)-amino]-4-pyrimidinyl]-6-méthyl-1,2,3,4-tétrahydroquinoléine ;
(f) 1-[2-[(3,4-diméthoxyphényl)-amino]-4-pyrimidinyl] - 6-méthyl-1,2,3,4-tétrahydroquinoléine ;
(g) 1- [2- [(3,4,5-triméthoxyphényl)-amino] -4-pyrimidinyl]-6-méthyl-1,2,3,4-tétrahydroquinoléine ;
(h) 1-[2-[(4,N-phénylaminophényl)amino]-4-pyrimidinyl]-6-méthyl-1,2,3,4-tétrahydroquinoléine ;
(i) [4-(6-méthyl-3,4-dihydro-2H-quinoléine-1-yl)-pyrimidine-2-yl]-(6-morpholine-4-yl-pyridine-3-yl)-amine ;
(j) 5-[4-(6-méthyl-3,4-dihydro-2H-quinoléine-1-yl)-pyrimidine-2-ylamino]-1,3-dihydro-benzo-imidazole-2-one ;
(k) (2,3-diméthyl-1H-indole-5-yl)-[4-(6-méthyl-3,4-dihydro-2H-quinoléine-1-yl)-pyrimidine-2-yl]-amine ;
(l) [4-(6-méthyl-3,4-dihydro-2H-quinoléine-1-yl)-pyrimidine-2-yl]-(2-méthyl-2H-pyrazole-3-yl)-amine;
(m) (6-méthoxy-pyridine-3-yl)-[4-(6-méthyl-3,4-dihydro-2H-quinoléine-1-yl)-pyrimidine-2-yl]-amine ;
(n) (4-fluoro-3-méthyl-phényl)-[4-(6-méthyl-3,4-dihydro-2H-quinoléine-1-yl)-pyrimidine-2-yl]-amine ;
(o) (5-cyclopropyl-2H-pyrazole-3-yl)-[4-(6-méthyl-3,4-dihydro-2H-quinoléine-1-yl)-pyrimidine-2-yl]-amine ;
(p) 4-benzyl-N-3-[4-(6-méthyl-3,4-dihydro-2H-quinoléine-1-yl)-pyrimidine-2-yl]-1H-pyrazole-3,5-diamine ;
(q) [4-(6-méthyl-3,4-dihydro-2H-quinoléine-1-yl)-pyrimidine-2-yl]-(4-méthyl-thiazole-2-yl)-amine ; et
(r) [4-(6-méthyl-3,4-dihydro-2H-quinoléine-1-yl)-pyrimidine-2-yl]-(5-méthyl-1H-pyrazole-3-yl)-amine.

11. Composé suivant la revendication 1, choisi dans le groupe consistant en les composés suivants :
(a) [4-(3,4-dihydro-2H-quinoléine-1-yl)-pyrimidine-2-yl]-(6-pyrrolidine-1-yl-pyridine-3-yl)-amine ;
(b) (1-cyclopentyl-1H-indole-6-yl)-[4-(6-méthyl-3,4-dihydro-2H-quinoléine-1-yl)-pyrimidine-2-yl]-amine ;
(c) [4-(6-méthyl-3,4-dihydro-2H-quinoléine-1-yl)-pyrimidine-2-yl]oxazole-4-yl-amine ;
(d) (3,4-dichloro-phényl)-[4-(6-méthyl-3,4-dihydro-2H-quinoléine-1-yl)-pyrimidine-2-yl]-amine ; et
(e) [4-(3,4-dihydro-2H-quinoléine-1-yl)-pyrimidine-2-yl]-isothiazole-3-yl-amine.

12. Composé suivant la revendication 1, choisi dans le groupe consistant en les composés suivants :
(a) 2-({5-[4-(2,3-dihydro-benzo[1,4]oxazine-4-yl)-pyrimidine-2-ylamino]-pyridine-2-yl}-méthyl-amino)-éthanol ;
(b) N-{5-[4-(3-oxo-3,4-dihydro-2H-quinoxaline-1-yl)-pyrimidine-2-ylamino]-pyridine-2-yl}-acétamide ;
(c) 3-chloro-N-[4-(4-méthyl-3-oxo-3,4-dihydro-2H-quinoxaline-1-yl)-pyrimidine-2-yl]-benzamide ;
(d) [4-(2,3-dihydro-benzo[1,4]thiazine-4-yl)-pyrimidine-2-yl]-oxazole-4-ylamine ;
(e) N-[4-(5-fluoro-2,3-dihydro-indole-1-yl)-pyrimidine-2-yl]-3-méthoxy-benzènesulfonamide ;
(f) [4- (5, 6-dihydro-pyrrolo [2, 3-d] pyrimidine-7-yl) - pyrimidine-2-yl]-(2-trifluorométhyl-phényl)-amine ;
(g) 6-méthoxy-1-[2-(pyridazine-3-ylamino)-pyrimidine-4-yl]-2,3-dihydro-1H-quinoléine-4-one ;
(h) 2-{5-[4-(3,4-dihydro-2H-quinoxaline-1-yl)-pyrimidine-2-ylamino]-indole-1-yl}-éthanol ;
(i) (2H-pyrazole-3-yl) - [4- (7-trifluorométhyl-3,4-dihydro-2H-quinoléine-1-yl)-pyrimidine-2-yl]-amine ;
(j) 1-[4-(3,4-dihydro-2H-[1,5]-naphthyridine-1-yl)- pyrimidine-2-yl]-3-éthyl-urée ;
(k) 1- [4- (2, 3-dihydro-benzo [1, 4] oxazine-4-yl) - pyrimidine-2-yl]-3-(2-éthoxy-éthyl)-urée ;
(l) ester tertiobutylique d'acide [4-(3,3-diméthyl-2,3-dihydro-indole-1-yl)-pyrimidine-2-yl]carbamique ;
(m) 3-cyano-N-[4-(7-méthoxy-2,3,4,5-tétrahydro-benzo[b]azépine-1-yl)-pyrimidine-2-yl]-benzamide ;
(n) isoxazole-4-yl-[4-(2,3,4,5-tétrahydro-benzo[b]-[1,4]diazépine-1-yl)-pyrimidine-2-yl]-amine ;
(o) (3,4-dichlorophényl) - [4- (3,4-dihydro-2H-benzo[b][1,4]thiazépine-5-yl)-pyrimidine-2-yl]-amine ;
(p) (6-aziridine-1-yl-pyridine-3-yl)-[4-(5-méthanesulfonyl-2,3-dihydro-indole-1-yl)-pyrimidine-2-yl]-amine ;
(q) N²-cyclopropyl-N⁵- [4- (6-fluoro-3,4-dihydro-2H-quinoléine-1-yl)-pyrimidine-2-yl]-pyridine-2,5-diamine ; et
(r) [4-(6-fluoro-3,4-dihydro-2H-quinoléine-1-yl)-pyrimidine-2-yl]-amide d'acide benzo[1,3]dioxole-5-carboxylique.

13. Composé suivant la revendication 1, dans lequel X représente un groupe méthylène.

14. Composé suivant la revendication 1, dans lequel la structure : 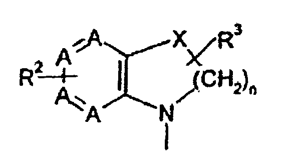 comprend un groupe choisi dans le groupe consistant en : 2,3-dihydro-1H-pyrrolo[2,3-b]pyridine ; 2,3-dihydro-1H-pyrrolo-[2,3-c]pyridine ; 2,3-dihydro-1H-pyrrolo[3,2-c]pyridine ; 2,3-dihydro-1H-pyrrolo[3,2-b]pyridine ; 6,7-dihydro-5H-pyrrolo-[3,3-b]pyrimidine ; 6,7-dihydro-5H-pyrrolo[3,2-d][1,2,3]-triazine ; 6,7-dihydro-5H-pyrrolo[2,3-d]-[1,2,3]triazine ; 1,4,5,7-tétraaza-indane ; 1,4,6,7-tétraaza-indane ; 6,7-dihydro-5H-pyrrolo[2,3-c]pyridazine ; 2,3-dihydro-1H-pyrrolo-[2,3-d]pyridazine ; 6,7-dihydro-5H-pyrrolo[3,2-c]pyridazine ; 6,7-dihydro-5H-pyrrolo[2,3-b]-pyrazine ; 6,7-dihydro-5H-pyrimido [4,5-b] [1,4]oxazine ; 5,6,7,8-tétrahydro-ptéridine ; 1,2,3,4-tétrahydro-pyrido-[2,3-b] pyrazine ; 1,2,3,4-tétrahydro-pyrido[3,4-b]pyrazine ; 1,2,3,4-tétrahydro-pyrido[3,4-b]pyrazine ; 1,2,3,4-tétrahydro-pyrido[2,3-b]pyrazine ; 5,6,7,8-tétrahydro-pyrazino[2,3-c]pyridazine ; 5,6,7,8-tétrahydro-ptéridine ; 1,2,3,4-tétrahydro-pyrazino[2,3-d]pyridazine ; 5,6,7,8-tétrahydro-pyrazino[2,3-c]pyridazine ; 1,2,3,4-tétrahydro-pyrazino[2,3-b]-pyrazine ; 5, 6, 7, 8-tétrahydro-pyrazino[2, 3-e] [1,2,4]triazine ; 5,6,7,8-tétrahydro-pyrazino[2,3-e] [1,2,4]triazine ; 5,6,7,8-tétrahydro-pyrazino[2,3-d] [1,2,3]triazine ; 5,6,7,8-tétrahydro-pyrazino[2,3-d] [1,2,3]triazine ; 2,3-dihydro-1H-4-oxa-1,5-diaza-naphtalène ; 2,3-dihydro-1H-4-oxa-1,6-diaza-naphtalène ; 3,4-dihydro-2H-1-oxa-4,6-diaza-naphtalène ; 3,4-dihydro-2H-1-oxa-4,5-diaza-naphtalène ; 7,8-dihydro-6H-5-oxa-1,2,8-triaza-naphtalène ; 3,4-dihydro-2H-1-oxa-4,6,7-triaza-naphtalène ; 6,7-dihydro-5H-8-oxa-1,2,5-triaza-naphtalène ; 3,4-dihydro-2H-1-oxa-4,5,8-triaza-naphtalène ; 7,8-dihydro-6H-pyrimido-[5,4-b] [1,4]oxazine ; 6,7-dihydro-5H-pyrimido-[4,5-b] [1,4]oxazine ; 6,7-dihydro-5H-8-oxa-1,2,3,5-tétraaza-naphtalène ; 6,7-dihydro-5H-8-oxa-1,2,4,5-tétraaza-naphtalène ; 7,8-dihydro-6H-5-oxa-1,2,3,8-tétraaza-naphtalène ; 6,7-dihydro-5H-8-oxa-1,2,4,5-tétraaza-naphtalène ; 2,3-dihydro-1H-pyrido[2,3-b][2,4]thiazine ; 2,3-dihydro-1H-4-thia-1,6-diaza-naphtalène ; 3,4-dihydro-2H-1-thia-4,6-diaza-naphtalène ; 3,4-dihydro-2H-pyrido[3,2-b]-[1,4]thiazine ; 7,8-dihydro-6H-5-thia-1,2,8-triaza-naphtalène ; 3,4-dihydro-2H-1-thia-4,6,7-triaza-naphtalène ; 6,7-dihydro-5H-8-thia-1,2,5-triaza-naphtalène ; 6,7-dihydro-5H-pyrimido-[4,5-b][1,4]thiazine ; 7,8-dihydro-6H-pyrimido[5,4-b] [1,4]- thiazine ; 3,4-dihydro-2H-1-thia-4,5,8-triaza-naphtalène ; 6,7-dihydro-5H-8-thia-1,2,4,5-tétraaza-naphtalène ; 7,8-dihydro-6H-5-thia-1,2,4,8-tétraaza-naphtalène ; 7,8-dihydro-6H-5-thia-1,2,3,8-tétraaza-naphtalène ; 6,7-dihydro-5H-8-thia-1,2,3,5-tétraaza-naphtalène ; 5,6,7,8-tétrahydro-pyrido-[3,2-d]pyrimidine ; 1,2,3,4-tétrahydro-pyrido[2,3-d]-pyridazine, 5,6,7,8-tétrahydro-pyrido[2,3-b]pyrazine ; 5,6,7,8-tétrahydro-pyrido[3,2-e][1,2,4]-triazine ; 5,6,7,8-tétrahydro-pyrido[2,3-e][1,2,4]triazine ; 5,6,7,8-tétrahydro-pyrido[3,2-d][1,2,3]triazine ; et 5,6,7,8-tétrahydro-pyrido[2,3-d][1,2,3]triazine.

15. Composition pharmaceutique comprenant une quantité efficace d'un composé suivant la revendication 1, et un support pharmaceutique.

16. Composition pharmaceutique suivant la revendication 15, pour le traitement ou la prévention chez un mammifère d'une affection pour laquelle un avantage thérapeutique est obtenu par modulation d'un processus cellulaire à médiation par les lymphocytes T.

17. Composition pharmaceutique suivant la revendication 15, pour le traitement ou la prévention du rejet d'un transplant chez un mammifère.

18. Composition pharmaceutique suivant la revendication 15, pour le traitement ou la prévention d'une maladie auto-immune chez un mammifère.

19. Composition pharmaceutique suivant la revendication 15, pour le traitement ou la prévention d'une maladie inflammatoire chez un mammifère.

20. Composition pharmaceutique suivant la revendication 15, pour le traitement ou la prévention d'une allergie chez un mammifère.

21. Composition pharmaceutique suivant la revendication 15, pour le traitement d'une leucémie à lymphocytes T ou d'un lymphome à lymphocytes T chez un mammifère.

22. Composition pharmaceutique suivant la revendication 15, pour le traitement d'une maladie dont le traitement peut être effectué en inhibant l'activation des lymphocytes T ou les résultats de ladite activation, chez un mammifère.

23. Composition pharmaceutique suivant la revendication 15, comprenant en outre un agent supplémentaire qui module le système immunitaire d'un mammifère ou qui est un agent anti-inflammatoire.

24. Composition pharmaceutique suivant la revendication 15, utile dans le traitement, chez un mammifère, d'un trouble ou d'une affection choisie dans le groupe consistant en l'asthme, le rhume des foins, l'urticaire, l'eczéma infantile, la dermatite atopique et d'autres maladies allergiques comportant des réactions d'hypersensibilité à médiation par anticorps (de type intermédiaire), le rejet d'un transplant, le psoriasis, la colite ulcérative, la maladie de Crohn, le lupus, la sclérose en plaques, la polyarthrite rhumatoïde, le diabète de type I, des troubles thyroïdiens auto-immuns, des états malins à lymphocytes T comprenant la leucémie à lymphocytes T, et le lymphome à lymphocytes T, ainsi que la maladie d'Alzheimer.

25. Composition pharmaceutique suivant la revendication 15, utile pour inhiber la tyrosine-kinase Lck chez un mammifère.

26. Composition pharmaceutique suivant la revendication 15, dans laquelle le composé est fourni sous forme d'un de ses sels, produits de solvatation ou hydrates pharmaceutiquement acceptables.

27. Composé, sel, produit de solvatation ou hydrate suivant l'une quelconque des revendications 1 à 14, destiné à être utilisé en médecine.

28. Utilisation d'un composé, sel, produit de solvatation ou hydrate suivant l'une quelconque des revendications 1 à 14, dans la production d'un médicament destiné au traitement d'une affection, chez un mammifère, dans laquelle un avantage thérapeutique est obtenu par modulation d'un processus cellulaire à médiation par les lymphocytes T, du rejet des transplants chez un mammifère, d'une maladie auto-immune chez un mammifère, d'une maladie inflammatoire chez un mammifère, de l'allergie chez un mammifère, d'une leucémie à lymphocytes T ou d'un lymphome à lymphocytes T chez un mammifère, d'une maladie chez un mammifère dont le traitement peut être effectué par inhibition de l'activation des lymphocytes T, ou des résultats de cette activation, de l'asthme, du rhume des foins, de l'urticaire, de l'eczéma infantile, de la dermatite atopique et d'autres maladies allergiques comportant des réactions d'hypersensibilité à médiation par anticorps (de type intermédiaire), du rejet d'un transplant, du psoriasis, de la colite ulcérative, de la maladie de Crohn, du lupus, de la sclérose en plaques, de la polyarthrite rhumatoïde, du diabète de type I, de troubles thyroïdiens auto-immuns, d'états malins à lymphocytes T comprenant la leucémie à lymphocytes T et le lymphome à lymphocytes T, ainsi que de la maladie d'Alzheimer.
